# EUROPEAN PATENT SPECIFICATION

(11) **EP 3 976 790 B1**
(45) Date of publication and mention of the grant of the patent: **08.04.2026**
(21) Application number: 20815356.9
(22) Date of filing: 28.05.2020
(51) Int. Cl.: C12N 15/113, A61K 48/00, C12N 5/10

(54) **METHODS AND COMPOSITIONS FOR GENERATING DOMINANT ALLELES USING GENOME EDITING**
VERFAHREN UND ZUSAMMENSETZUNGEN ZUR ERZEUGUNG DOMINANTER ALLELE UNTER VERWENDUNG VON GENOMEDITIERUNG
PROCÉDÉS ET COMPOSITIONS POUR GÉNÉRER DES ALLÈLES DOMINANTS À L'AIDE D'ÉDITION DE GÉNOME

(30) Priority: 29.05.2019 US 201962854142 P; 14.08.2019 US 201962886726 P; 14.08.2019 US 201962886732 P
(43) Date of publication of application: 06.04.2022
(60) Divisional application: 26161819.3
(73) Proprietor: Monsanto Technology LLC, St. Louis, Missouri 63167 (US)
(72) Inventor: CARGILL, Edward James, St. Louis, Missouri 63167 (US); EUDY, Douglas Michael, St. Louis, Missouri 63167 (US); KOURANOV, Andrei Y., St. Louis, Missouri 63167 (US); LAWRENCE, Richard Joseph, St. Louis, Missouri 63167 (US); SLEWINSKI, Thomas L., St. Louis, Missouri 63167 (US); SHULTZ, Randy, St. Louis, Missouri 63167 (US); TO, PokChun Jennifer, St. Louis, Missouri 63167 (US); YANG, Samuel Sukhwan, St. Louis, Missouri 63167 (US); ZHANG, Yuanji, St. Louis, Missouri 63167 (US)
(74) Representative: BIP Patents
(86) International application number: PCT/US2020/035001
(87) International publication number: WO 2020/243368

(56) References cited:
- WO-A1-2015/117041
- WO-A1-2017/143061
- WO-A1-2018/097257
- WO-A1-2018/119225
- WO-A1-2019/036599
- WO-A1-2020/117553
- WO-A2-2019/089884
- KASIM VIVI ET AL: "Control of siRNA expression using the Cre-loxP recombination system", NUCLEIC ACIDS RESEARCH, OXFORD UNIVERSITY PRESS, GB, vol. 32, no. 7, 1 January 2004 (2004-01-01), pages e66, XP002333060, ISSN: 0305-1048, DOI: 10.1093/NAR/GNH061
- HAHN FLORIAN ET AL: "Generation of Targeted Knockout Mutants in Arabidopsis thaliana Using CRISPR/Cas9", BIO-PROTOCOL, vol. 7, no. 13, 1 January 2017 (2017-01-01), Sunnyvale, CA, USA, XP093047451, ISSN: 2331-8325, DOI: 10.21769/BioProtoc.2384
- QI YIPING ET AL: "Targeted Deletion and Inversion of Tandemly Arrayed Genes in Arabidopsis thaliana Using Zinc Finger Nucleases", vol. 3, no. 10, 1 October 2013 (2013-10-01), pages 1707 - 1715, XP055902241, Retrieved from the Internet <URL:http://academic.oup.com/g3journal/article-pdf/3/10/1707/37142887/g3journal1707.pdf> DOI: 10.1534/g3.113.006270
- KRAFT ET AL.: "Deletions, Inversions, Duplications: Engineering of Structural Variants using CRISPR/Cas in Mice", CELL REPORTS, vol. 10, 10 February 2015 (2015-02-10), pages 833 - 839, XP055365089, DOI: 10.1016/j.celrep.2015.01.016
- RAIJA SOININEN, HUOTARI MERJA, HOSTIKKA SIRKKA LIISA, PROCKOPS DARWIN J, TRYGGVASON KARL: "The Structural Genes for a1 and a2 Chains of Human Type IV Collagen Are Divergently Encoded on Opposite DNA Strands and Have an Overlapping Promoter Region", JOURNAL BIOLOGICAL CHEMISTRY, vol. 263, no. 33, 25 November 1998 (1998-11-25), pages 17217 - 17220, XP055763141

## Description

### CROSS-REFERENCE TO RELATED APPLICATIONS

This application claims the benefit of U.S. Provisional Application No. 62/854,142, filed May 29, 2019, U.S. Provisional Application No. 62/886,726, filed August 14, 2019, and U.S. Provisional Application No. 62/886,732, filed August 14, 2019.

### FIELD

The present disclosure relates to methods and compositions for generating dominant alleles via targeted editing of genomes.

### INCORPORATION OF SEQUENCE LISTING

A sequence listing contained in the file named "P34497WO00_SL.TXT" which is 172,842 bytes (measured in MS-Windows^{®}) and created on May 28, 2020, is filed electronically herewith.

### BACKGROUND

Dominant alleles are alleles that mask the contribution of a second allele at the same locus. A dominant allele can be a dominant negative allele or a dominant positive allele. Dominant negative alleles, or antimorphs, are alleles that act in opposition to normal allelic function. For example, a dominant negative allele often abrogates the normal function of an allele in a heterozygous or homozygous state. Dominant positive alleles can increase normal gene function *(e.g.,* a hypermorph) and/or provide broadened or new functions for a gene *(e.g.,* a neomorph).

Naturally occurring and random mutagenesis techniques (*e.g.,* ethyl methyl sulfonate and T-DNA insertions) have been used to generate mutations in a variety of cell types. However, dominant mutations occur at low frequencies and are difficult to obtain in a given gene of interest. Therefore, methods and compositions to selectively edit a genome to create a dominant negative allele or a dominant positive allele would be beneficial.

WO2018/119225 A1 discloses plants exhibiting a semi-dwarf phenotype with reduced plant height compared to control wildtype plants. Some of the disclosed semi-dwarf plants comprise at least one non-natural brachytic mutation in which the activity of a BR2 gene is reduced. Also disclosed are methods for producing a semi-dwarf com plant using a CRISPR based genome editing system.

KASIM VIVI ET AL (2004) NUCLEIC ACIDS RESEARCH, vol. 32, no. 7, page e66 discloses the control of siRNA expression using the Cre-loxP recombination system. HAHN FLORIAN ET AL (January 2017) BIO-PROTOCOL, vol. 7, no. 13, January 2017 discloses generation of Targeted Knockout Mutants in Arabidopsis thaliana Using CRISPR/Cas9.

WO2020/117553 A1 relates to methods and compositions for gene silencing by genome editing. In some embodiments, nucleases are provided selected from the group consisting of meganucleases (MNs), zinc-finger nucleases (ZFNs), transcription-activator like effector nucleases (TALENs), Cas9 nuclease, Cpfl nuclease, dCas9-FokI, dCpf1-FokI, chimeric Cas9/Cpf1-cytidine deaminase, chimeric Cas9/Cpf1-adenine deaminase, chimeric FEN1-FokI, and Mega-TALs, a nickase Cas9 (nCas9), chimeric dCas9 non-FokI nuclease and dCpf1 non-FokI nuclease. Also provided are methods and compositions for rearranging a chromosome by genome editing.

KRAFT et al. (2015) Cell Reports, vol. 10, pages 833-839, discloses deletions, inversions, and duplications in the engineering of structural variants using CRISPR/Cas in mice.

WO2015/117041 A1 discloses methods for generating dominate traits in eukaryotic systems using one or more gene modification-mediated methods. Aspects of the technology are further directed to methods for silencing *SbCSE* and/or *SbCAD2* gene expression or activity in a sorghum plant. Silencing the *SbCSE* and/or *SbCAD2* gene leads to reduced lignin content in sorghum.

### SUMMARY

.

**In** one aspect, this disclosure provides a method comprising: (a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; (b) inducing a first double-stranded break and a second double-stranded break flanking a targeted region; (c) identifying one or more cells comprising a deletion of the targeted region of the chromosome; and (d) selecting one or more cells comprising the deletion of the targeted region of the chromosome.

.

.

In one aspect, this disclosure provides a method of reducing expression of a gene in a cell comprising: (a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; (b) inducing a first double-stranded break and a second double-stranded break flanking a targeted region using a targeted editing technique, where the targeted region comprises the second coding region and the intervening region; and (c) identifying one or more cells comprising a deletion of the targeted region, where the second promoter generates at least one antisense RNA of the first coding region, and where expression of the first coding region is reduced as compared to a control cell that does not comprise the deletion of the targeted region.

.

.

A method comprising: (a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; (b) providing to one or more cells at least one RNA-guided nuclease or one or more vectors encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a first target site and a second target site flanking a targeted region of a chromosome, where the targeted region comprises the second encoding region and the intervening region, where the RNA-guided nuclease creates double-stranded breaks in the chromosome at the first target site and the second target site; (c) identifying one or more cells comprising a deletion of the targeted region; and (d) selecting one or more cells comprising the deletion of the targeted region.

### BRIEF DESCRIPTION OF THE DRAWINGS

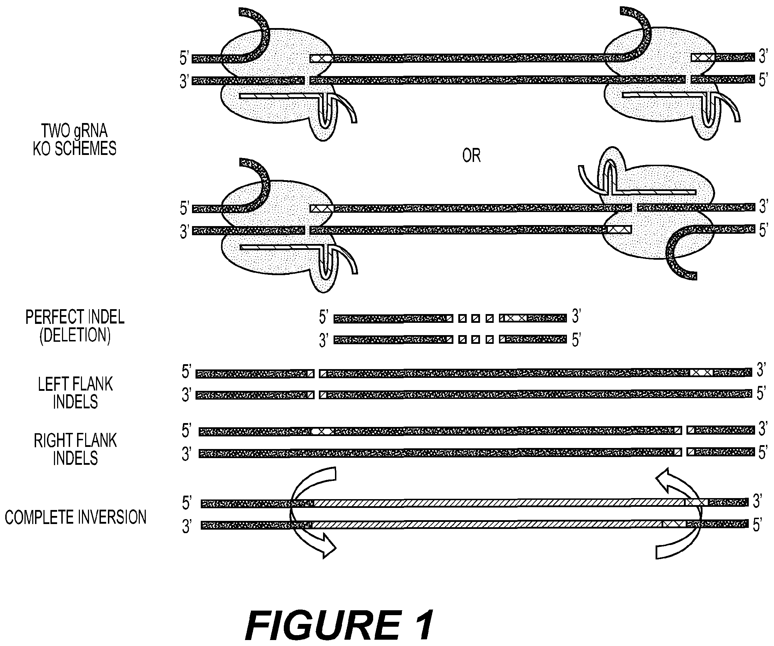

**Figure 1** comprises Panel A and Panel B. Panel A shows that two sRNAs coupled to two RNA-guided nucleases can hybridize target DNA on the same or different strands to isolate a region of DNA from the rest of the DNA strand(s) by generating two double-stranded breaks (DSBs). Panel B shows different outcomes that are possible after two RNA-guided nucleases generate two DSBs in DNA. Native cellular machinery can repair the two DSBs by deleting the entire region between the DSBs, by deleting a small portion of the region from the 5' or 3' end, or by inverting the region between the DSBs.

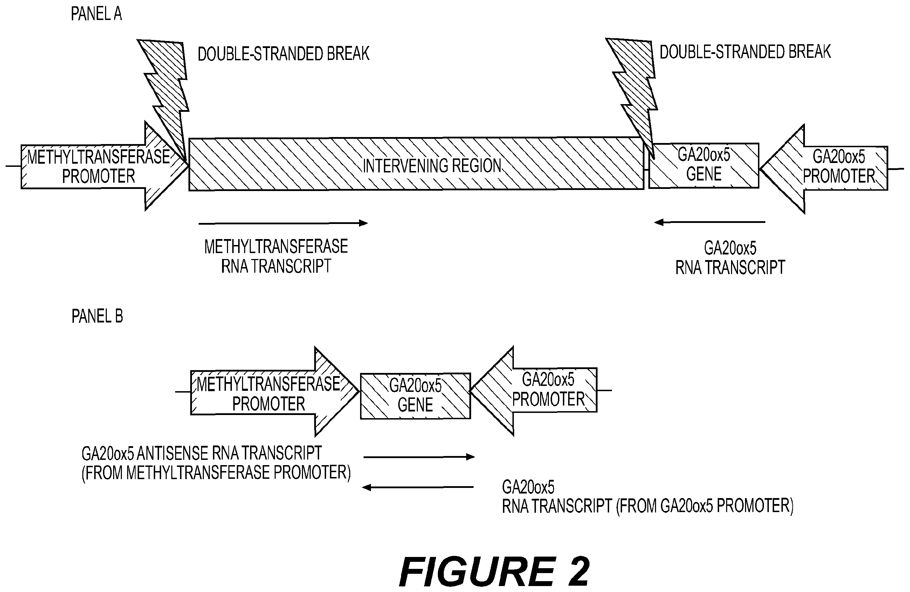

**Figure 2** comprises Panel A and Panel B. Panel A shows a representation of the maize genomic region near the GA20 oxidase_5 gene. As shown in Panel A, a methyltransferase/ SAMT gene is adjacent to the GA20 oxidase_5 gene, but it is in an opposite orientation. The intervening region between the SAMT promoter and the GA20 oxidase_5 coding gene is deleted after generating a double-stranded break on each end. Panel B shows the structure of the region following the deletion of the intervening region. By removing the intervening region the SAMT promoter can drive the expression of an antisense GA20 oxidase_5 RNA transcript, which can form a double-stranded RNA with sense GA20 oxidase_5 RNA transcripts generated by the native GA20 oxidase_5 promoter.

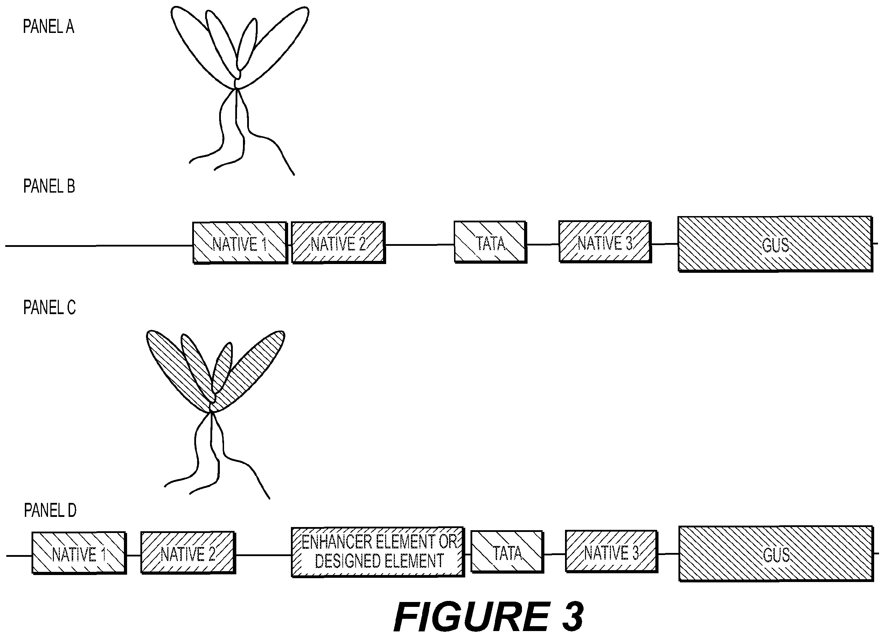

**Figure 3** comprises Panels A, B, C, and D. Panel A shows GUS staining of an *Arabidopsis thaliana* plant, which demonstrates that the Native 3 promoter expresses GUS only in root tissue. Panel B shows the genomic structure around the Native 3 promoter and GUS transgene. Panel C shows expanded GUS staining after insertion of a native genomic expression element such as an enhancer element or designed expression element upstream of the Native 3 TATA box. Panel D shows the genomic structure around the Native 3 promoter after targeted insertion of the native enhancer or designed element.

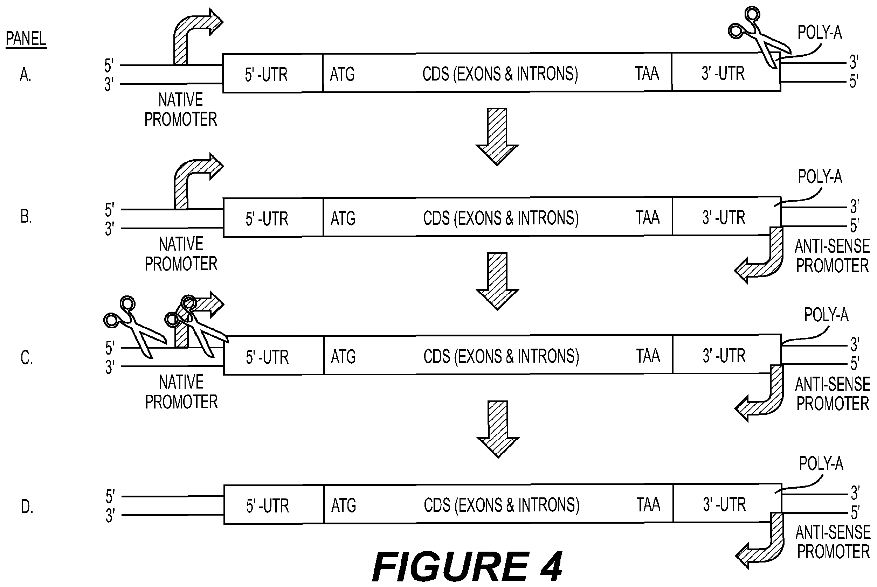

**Figure 4** comprises Panels A, B, C, and D. Panel A shows the structure of a gene of interest and indicates that a double-stranded break can be generated immediately upstream of the poly-adenylation site in the 3'-UTR of the gene of interest. Panel B shows the insertion of an anti-sense promoter at the double-stranded break site. The promoter can be the native promoter of the gene or any promoter of interest. Panel C shows the optional generation of two double-stranded breaks surrounding the native promoter of the gene of interest, leading to its deletion (Panel D).

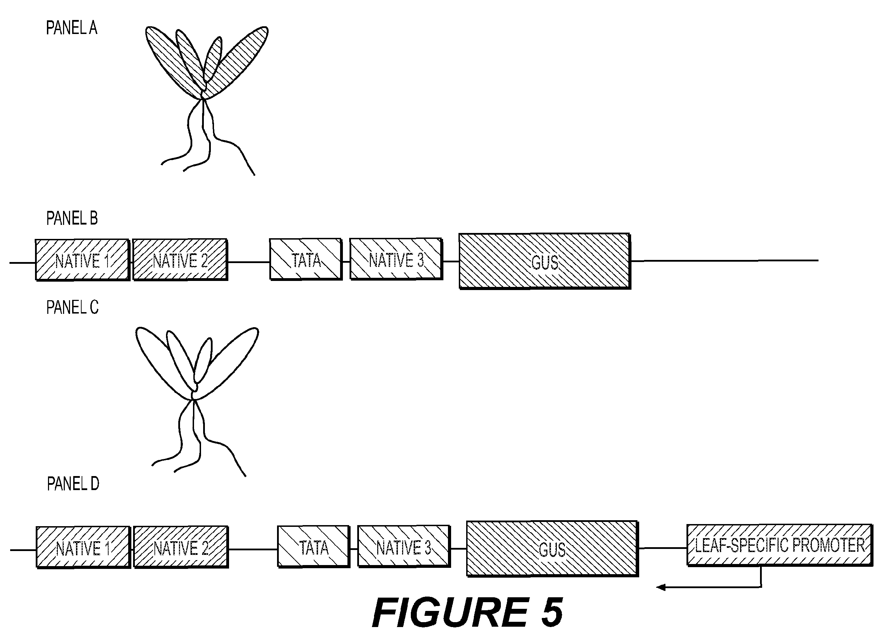

**Figure 5** comprises Panels A, B, C, and D. Panel A shows GUS staining of an *Arabidopsis thaliana* plant comprising a GUS transgene under the control of the Native 3 promoter. GUS is expressed throughout the plant. Panel B shows the genomic region around the Native 3 promoter and the GUS transgene. Panel C shows the reduction of GUS staining when a leaf-specific promoter is inserted downstream of the GUS transgene such that it transcribes antisense GUS RNA. Panel D shows the genomic region around the Native 3 promoter and GUS transgene following the insertion of the antisense leaf-specific promoter.

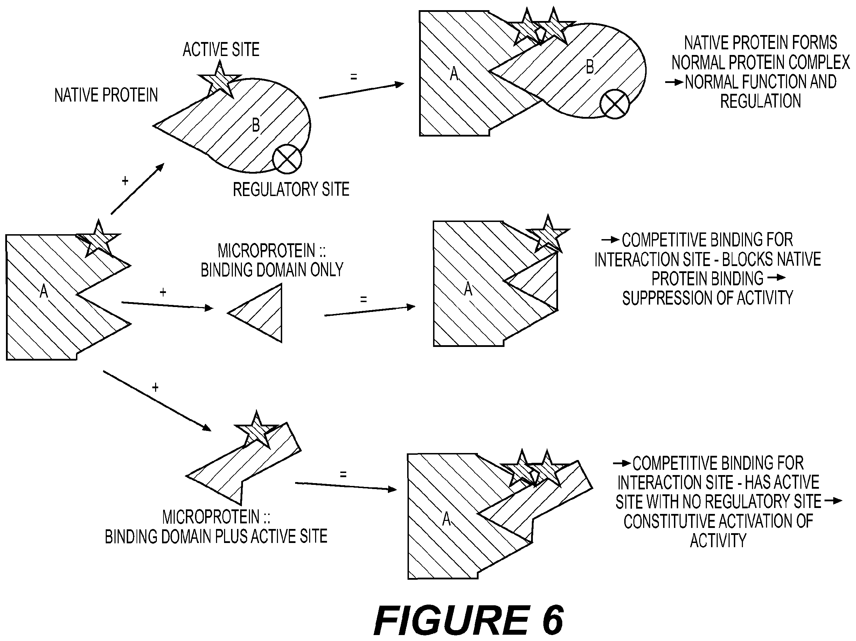

**Figure 6** shows potential outcomes of protein truncation. The upper flow shows normal protein interactions that brings all of the encoded components together in the correct conformation to allow function of a protein complex. The center flow shows a truncation of one of the protein components that removes a functional unit of the protein but retains the interaction domain that can still bind the interacting protein and block the interaction site from a complete fully functional version of the truncated protein. In the center case - this would act as a dominant negative allele. The lower flow shows the strategic deletion of part of a protein that encodes a regulatory element for activity and leaves the functional domain and the interaction domain intact - thus leading to a protein complex that is stuck in either a constitutively active or suppressed state of activity.

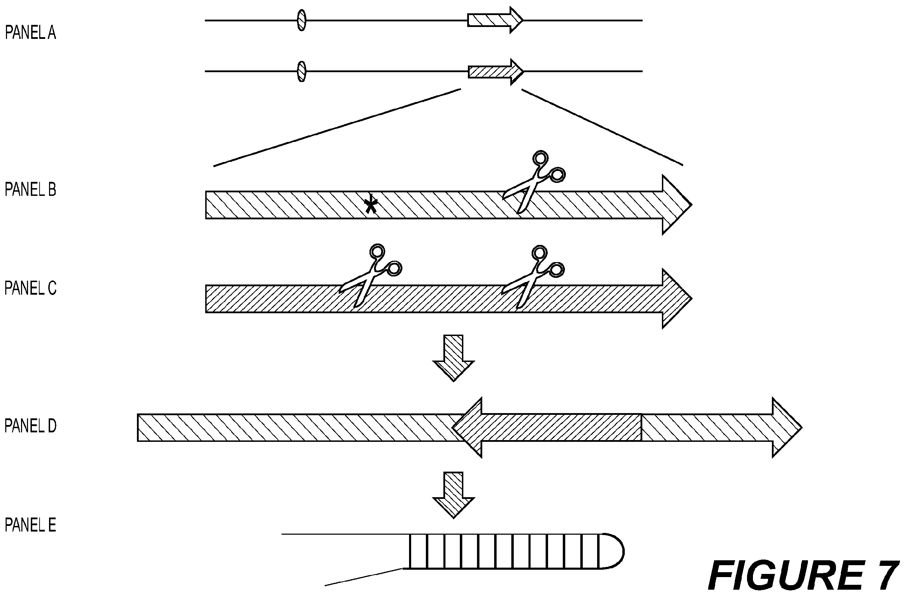

**Figure 7** comprises Panels A, B, C, D, and E. Panel A shows a heterozygous genomic locus. Panel B shows that a nuclease (represented by scissors) generates one DSB in the first allele. Panel C shows that a nuclease generates two DSBs in the second allele. In one possible outcome (Panel D) the region between the two DSBs on the second allele (Panel C) is inverted and inserted into the single DSB in the first allele. Panel E shows a hairpin RNA transcript produced form transcribing the allele structure in Panel D.

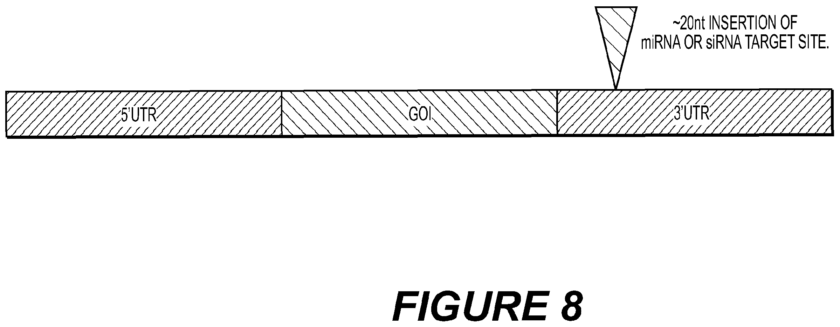

**Figure 8** provides a schematic of a non-coding RNA target site insertion in a gene of interest. Such an insertion can lead to the production of transient secondary siRNAs to down-regulate the gene of interest (GOI).

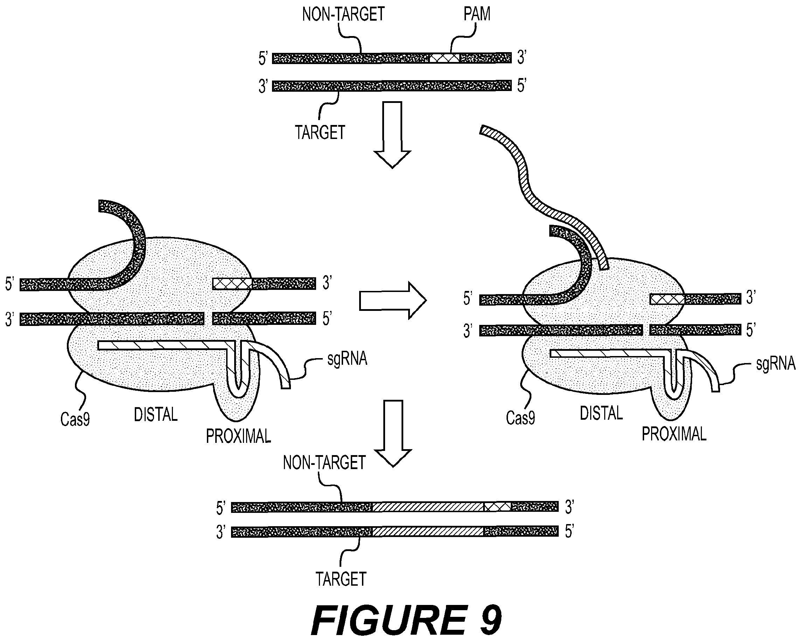

**Figure 9** provides a schematic of a Cas9-mediated double-stranded break (DSB) and a tether guide oligo (tgOligo) bound to a target DNA site. The Cas9-PAM interaction occurs on the non-target strand; sgRNA-DNA annealing occurs on the target strand. The blunt ends at the Cas9 cut site are held in place by Cas9 at the 5' end of the non-target strand (PAM location), and at both cut ends (3' and 5') of the target strand. The 3' cut end of the non-target strand is free and 'flaps' around. The 3' free 'flap' end of the non-target strand can be up to 35 nucleotides which can be sufficient for specific complementarity binding. A tgOligo (*e.g.,* a ssDNA template) can be included for integration of desired nucleotide modification. The drawing scheme used here is followed in the subsequent figures.

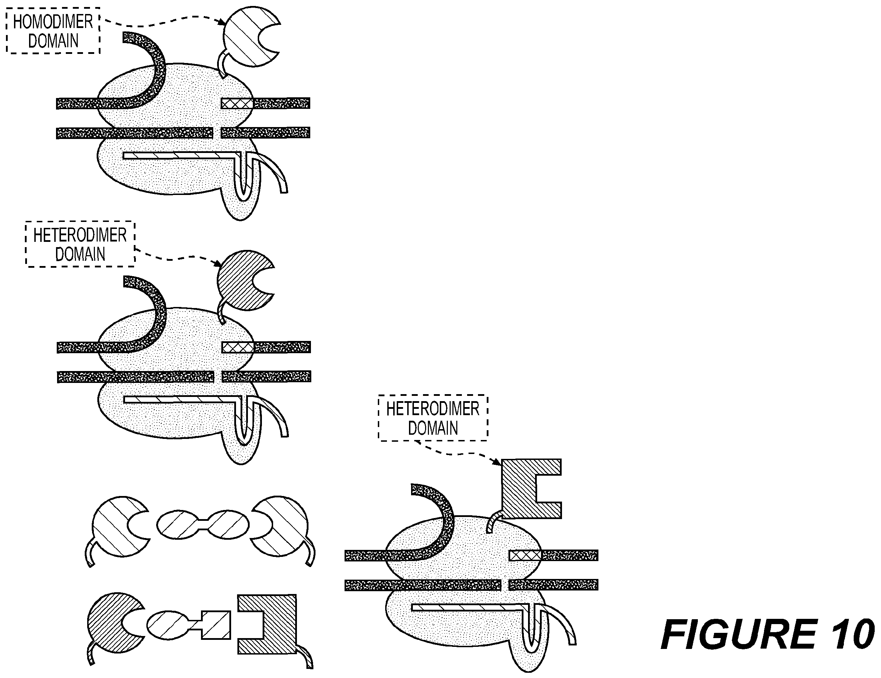

**Figure 10** depicts Cas9 conjugated with a homodimer domain (top) and heterodimer domains (middle and bottom right) to facilitate dimerization. Ligands for the homodimer and heterodimer domains are shown (bottom left). The drawing scheme used here is followed in the subsequent figures; e.g., the ligands, the homodimer or heterodimer domains, ssDNA binding domains. Each component of the Cas9/sgRNA complex and target DNA are shown as illustrated in Figure 9. The drawing scheme used here for different dimerization domains is followed in the subsequent figures.

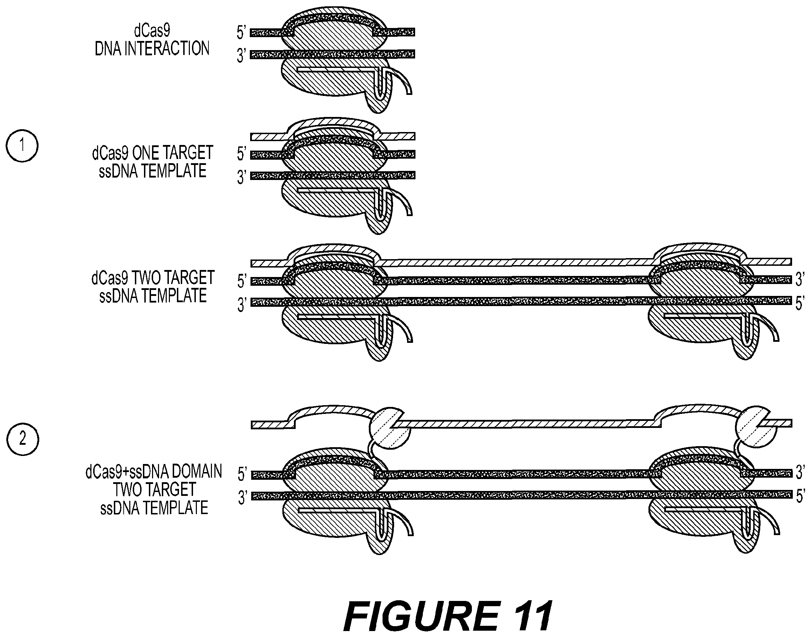

**Figure 11** depicts use of catalytically deactivated Cas9 (dCas9) to increase genome editing efficiency. Panel 1 illustrates that dCas9 binds to DNA at a target site specified by the gRNA and creates a loop structure accessible for template-based editing. Panel 2 illustrates a modified scheme for further facilitating template-based editing via a dCas9 conjugated with a ssDNA-binding domain. The editing efficiency with this modified scheme is expected to be higher compared to those in Panel 1, because a ssDNA template is bound to dCas9 complex and would be brought into proximity of the gRNA target.

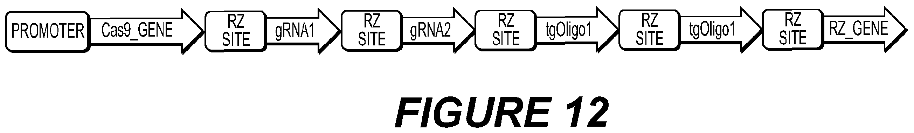

**Figure 12** provides an example construct containing Cas9, gRNAs, and tgOligos. RZ stands for Ribozyme, an enzyme that cleaves a 15bp recognition site in RNA (RZ site).

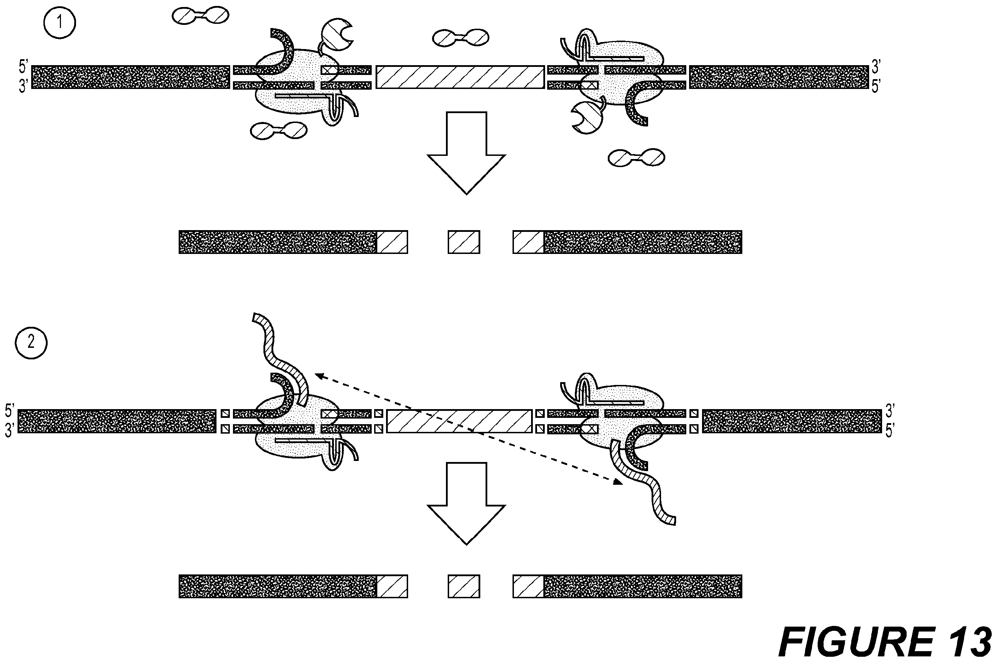

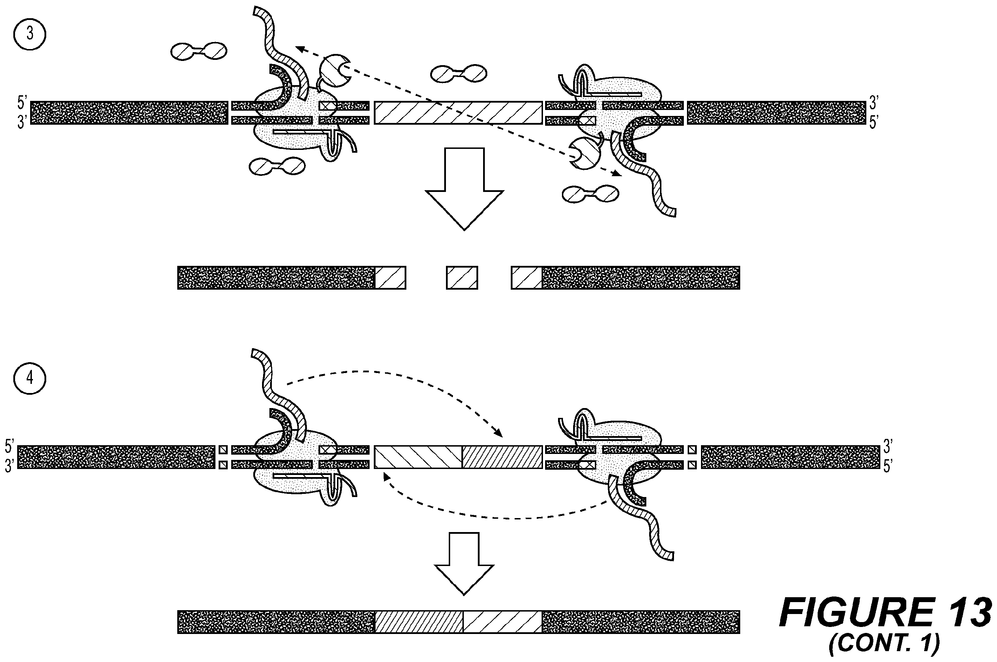

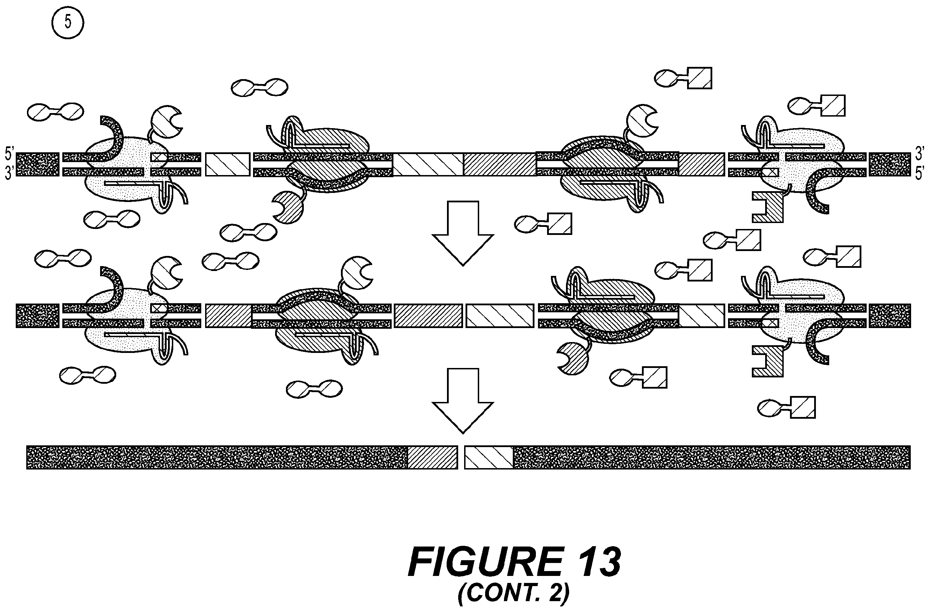

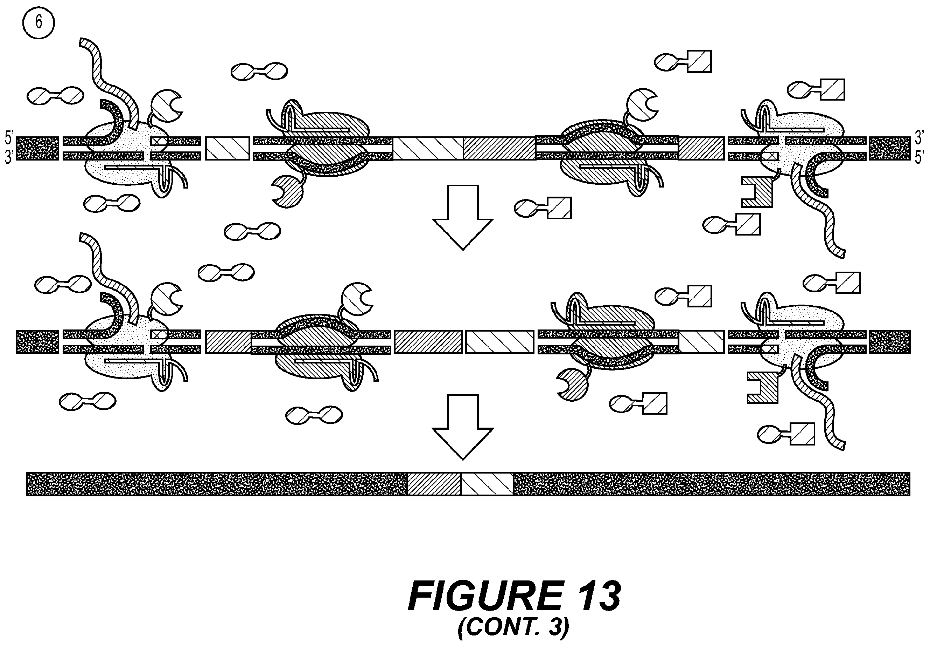

**Figure 13** provides anllustration of various approaches for improving genome editing efficiency. Using dimerization domains (*See* Figure 10), tgOligos (*See* Figure 9), or a combination of both can enhance recovery of complete knockout (deletion) of the genomic region flanked by the two gRNA target sites. Panel 1 shows a dimerization-enhanced knock out (KO) event. Panel 2 shows a tgOligo-enhanced KO event. Panel 3 shows an enhanced KO event via a combination of dimerization and tgOligos. Panel 4 shows a tgOligo-enhanced inversion event. Panel 5 shows a dimerization-enhanced inversion event. Panel 6 shows an inversion event assisted by a combination of Cas9 dimerization/deactivation and tgOligos. Only shown is the configuration where two gRNAs recognize different strands of a target dsDNA. The same concept is equally applicable to the other configuration where two gRNAs recognize the same strand of a target dsDNA.

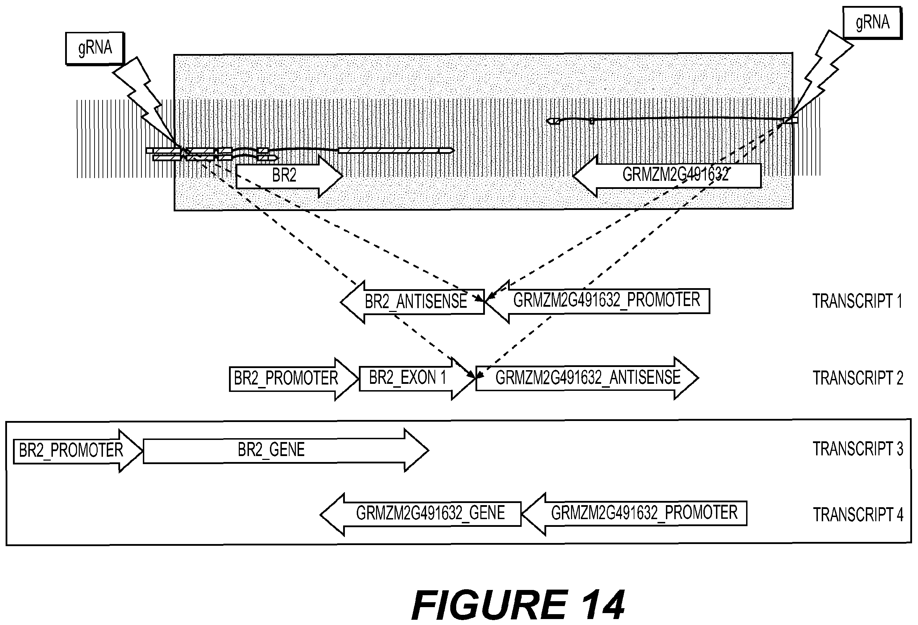

**Figure 14** provides an illustration of editing the com BR2 gene to generate a dominant knockout allele via genome inversion. Two exemplary gRNAs are used. A first gRNA (shown on the left) targets the end of the first exon of BR2; a second gRNA (shown on the right) recognizes the start codon region of the adjacent GRMZM2G491632 gene. Inversion of the genomic segment flanked by these two gRNAs can lead to a BR2 antisense partial transcript (*See* Transcript 1). This BR2 antisense transcript is produced via the GRMZM2G491632 promoter activity. Adjusting the relative position of the two gRNAs can achieve a BR2 antisense complete transcript (*e.g*., moving the first gRNA on the left to target the start codon region of the BR2 gene) or a BR2 antisense transcript under the control of the native BR2 promoter (e.g., moving the second gRNA on the right to target the stop codon region of the BR2 gene).

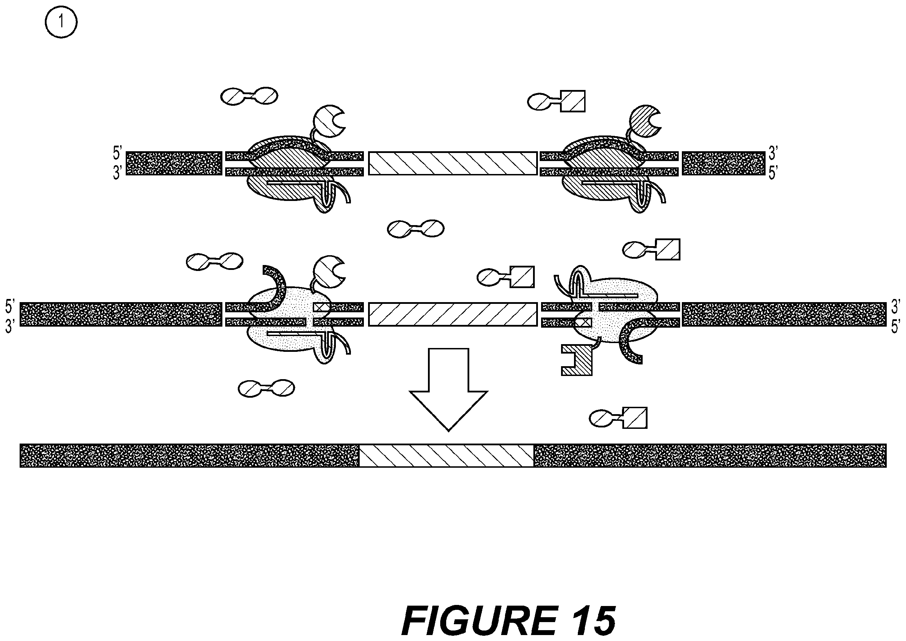

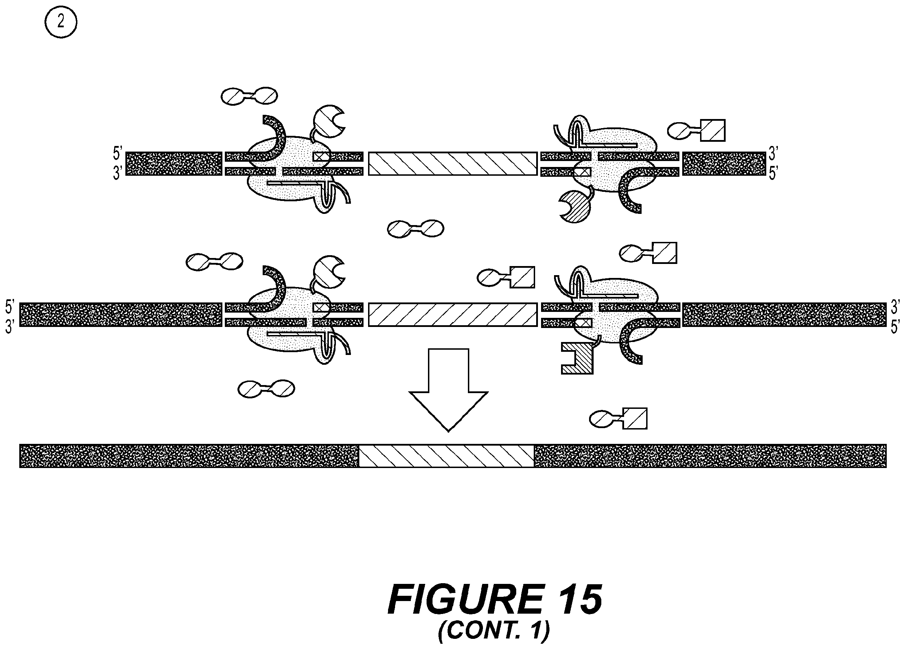

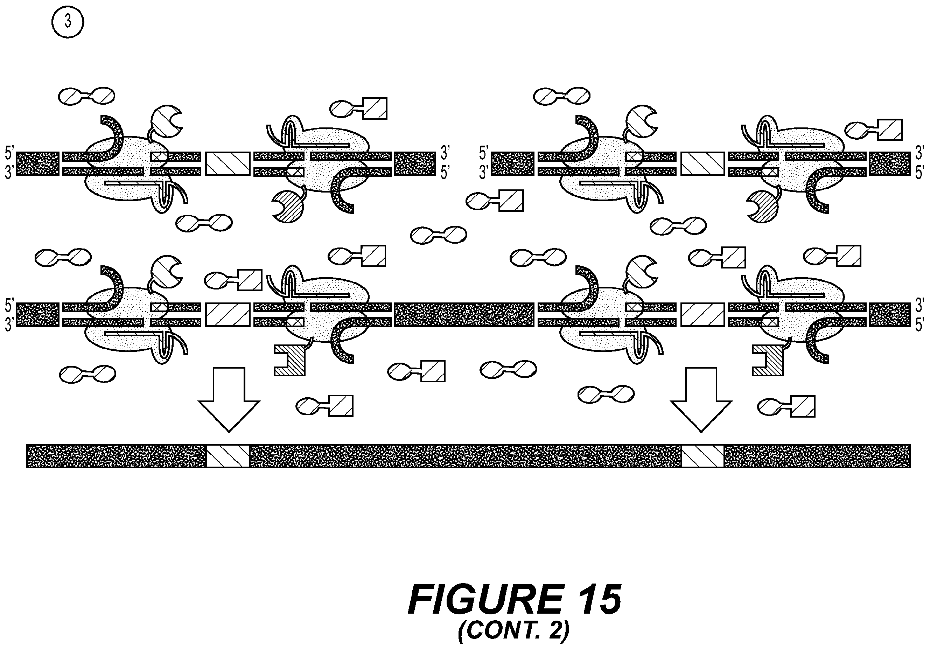

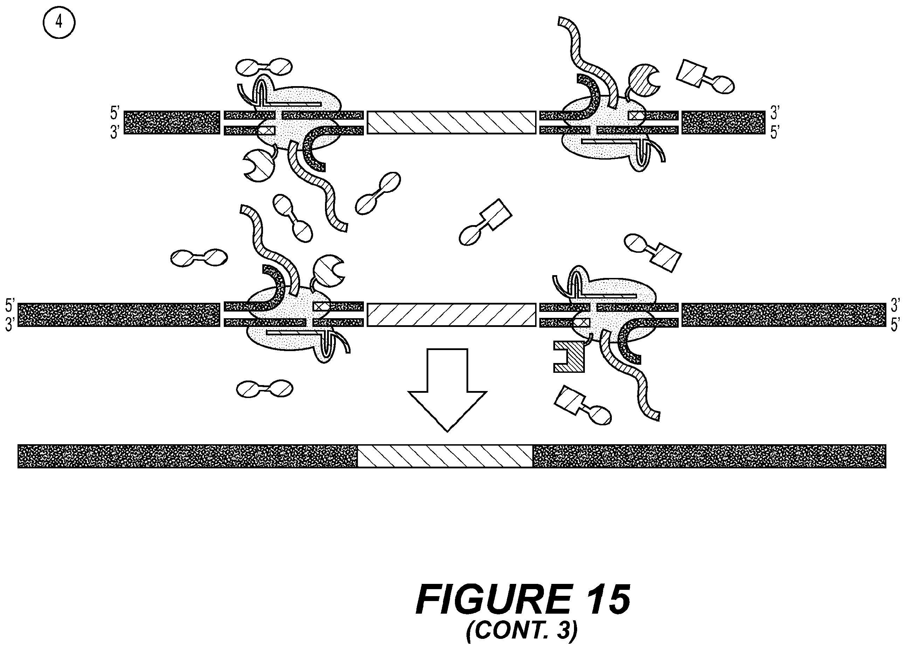

**Figure 15** provides an illustration of dimerization-enhanced template-based editing or site directed integration (SDI) at a single location (Panels 1 and 2) or multiple locations (Panel 3), and dimerization/tgOligo-enhanced template-based editing or SDI (Panel 4).

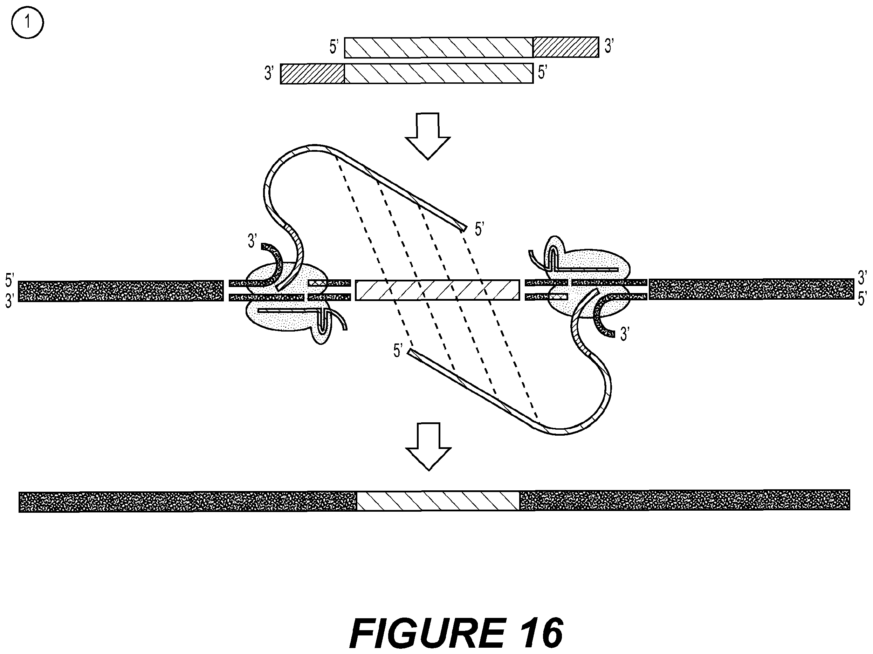

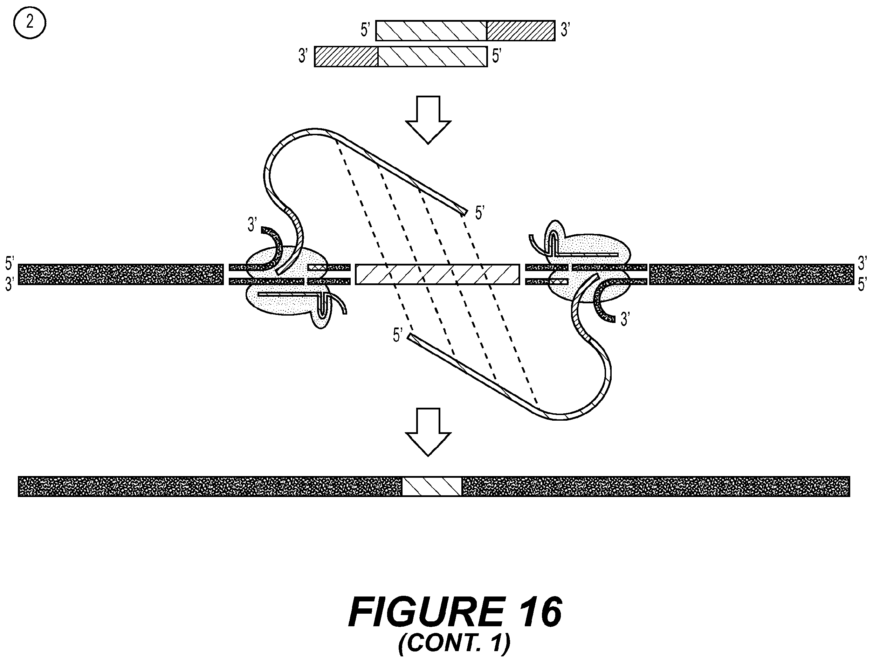

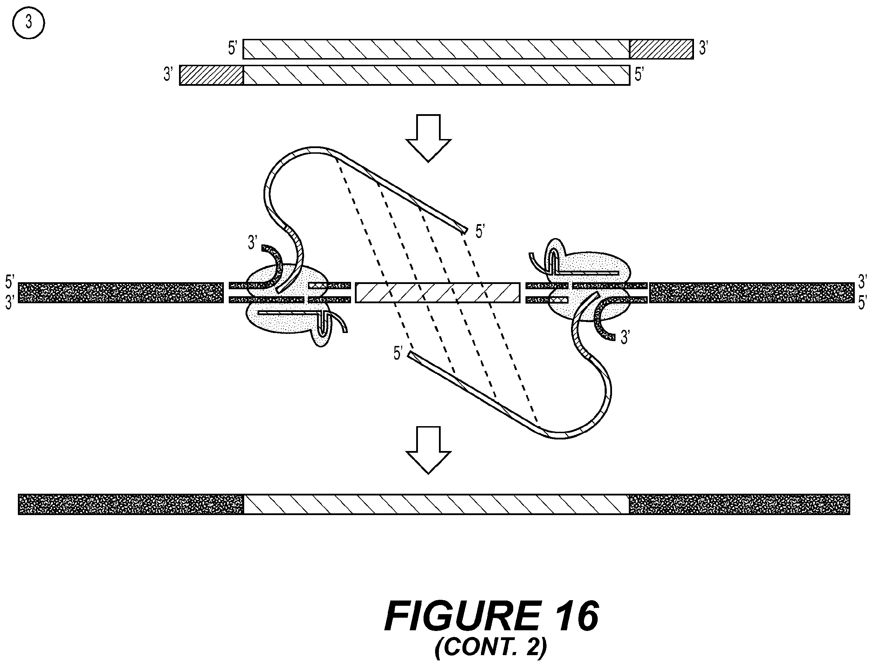

**Figure 16** provides an illustration of template editing (Panel 1), site directed integration (Panel 2), and/or recombination with tgOligos (Panel 3).

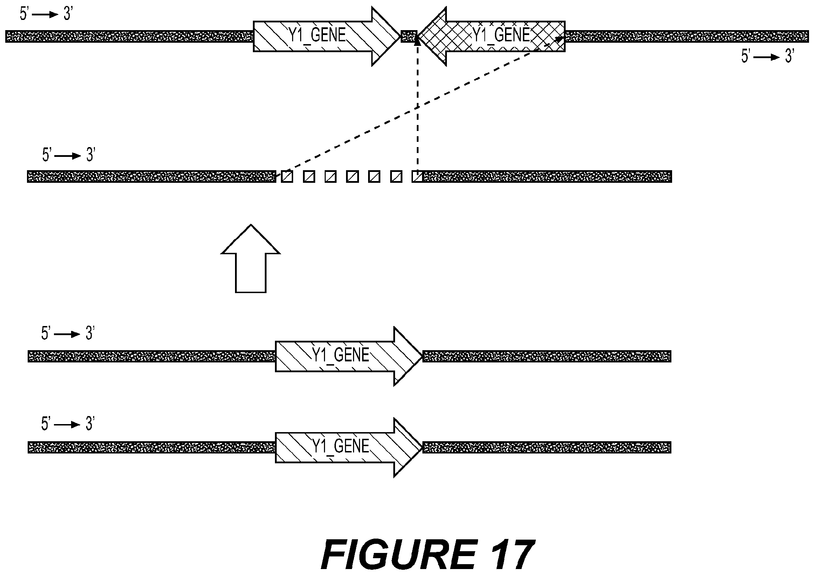

**Figure 17** provides an illustration of the stacking of an inverted Y1 gene head-to-tail to produce an antisense transcript to silence the gene expression. This approach can create a dominant mutant Y1 allele for a normally recessive trait. This dominant allele remains controlled by the native Y1 promoter.

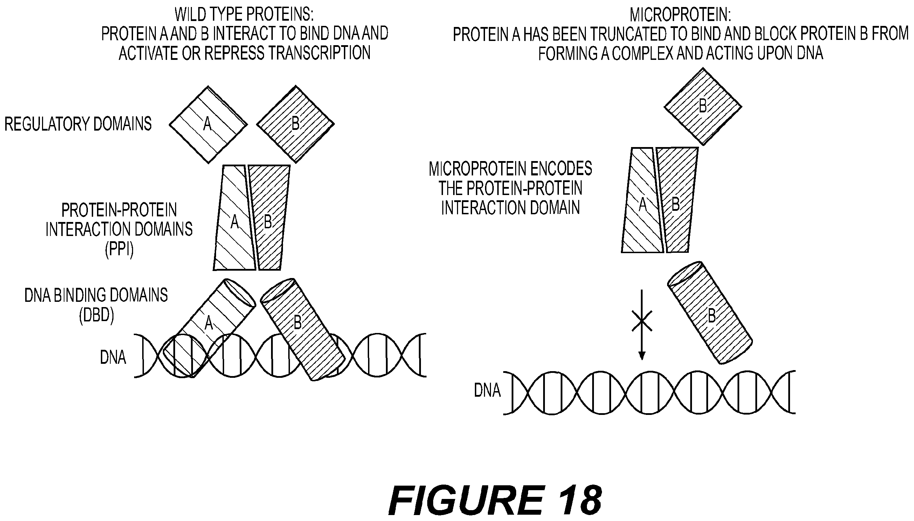

**Figure 18** provides an illustration of a microprotein. Targets of microproteins are often transcriptional regulators that bind to DNA as active homodimers. Microproteins interfere with their targets by forming non-functional heterodimeric complexes that cannot bind to DNA. DBD, DNA-binding domain; PPI, protein-protein interaction domain.

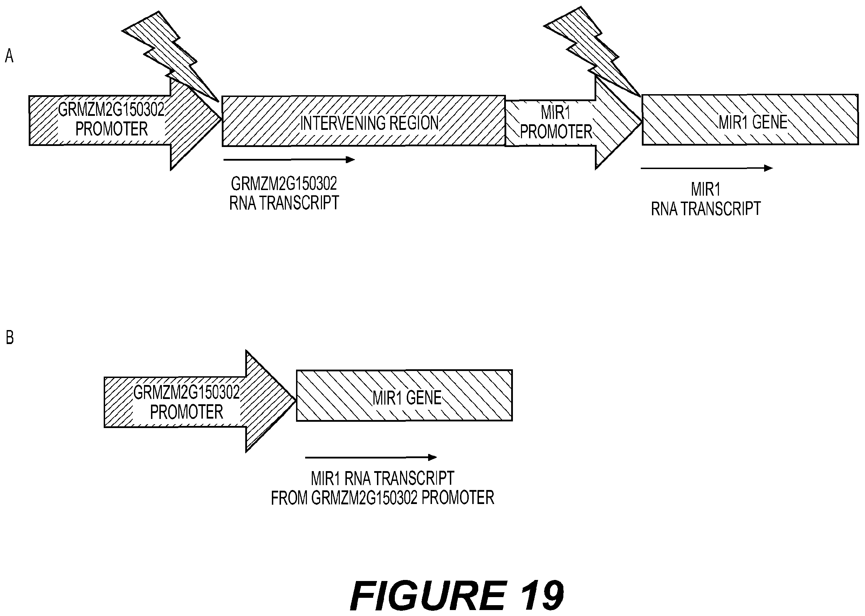

**Figure 19** comprises Panel A and Panel B. Panel A shows a representation of the maize genomic region near the MIR1 gene. As shown in Panel A, the GRMZM2G150302 gene is adjacent and upstream to the GA20 oxidase_5 gene. The intervening region between the GRMZM2G150302 promoter and the MIR1 coding gene is deleted after generating a double-stranded break on each end. Panel B shows the structure of the region following the deletion of the intervening region. By removing the intervening region the GRMZM2G150302 promoter can drive the expression of the MIR1gene.

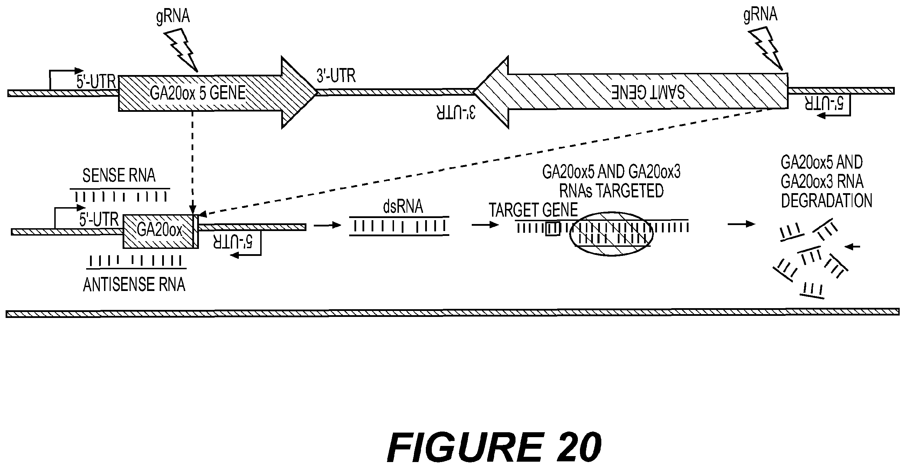

**Figure 20** provides illustrative examples for creating an antisense RNA molecule that targets the Zm.GA20ox5 gene and the Zm.GA20ox3 gene by deleting a genomic region between the Zm.GA20ox5 and its neighboring gene Zm.SAMT oriented in the opposite direction, through genome editing.

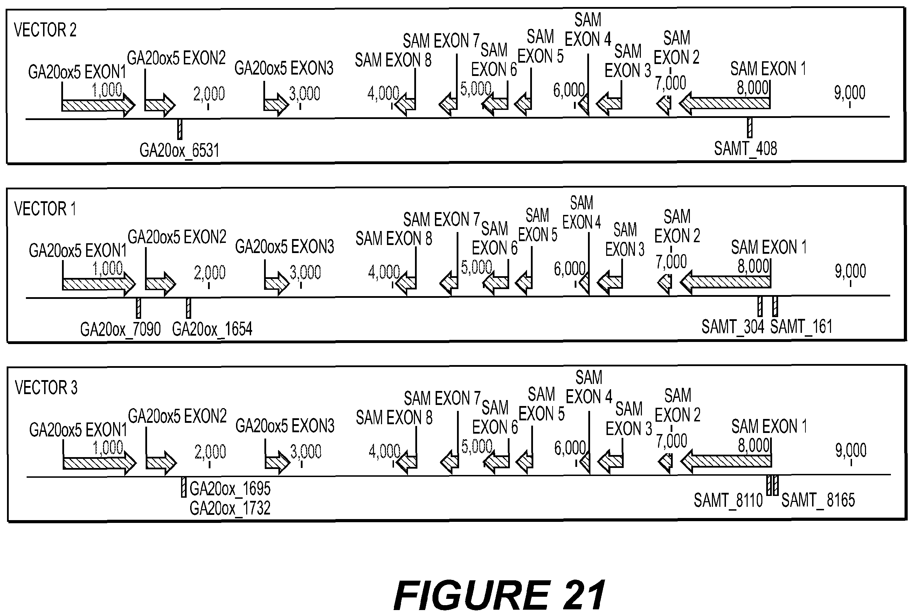

**Figure 21** illustrates the genomic position of various guide RNA target sites in three exemplified vectors for creating a genomic deletion between the Zm.GA20ox5 gene and its neighboring Zm.SAMT gene.

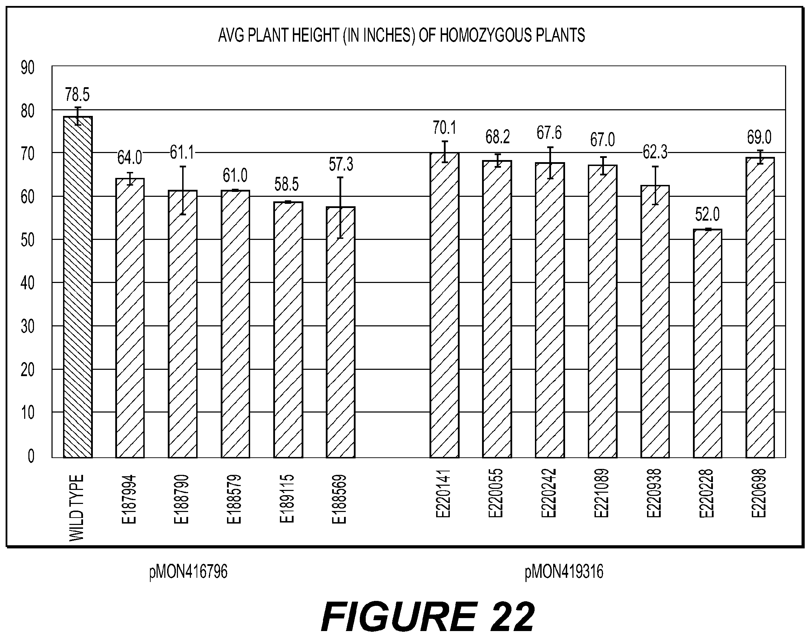

**Figure 22** depicts the average height of wild type plants and homozygous edited plants in inches (Y-axis).

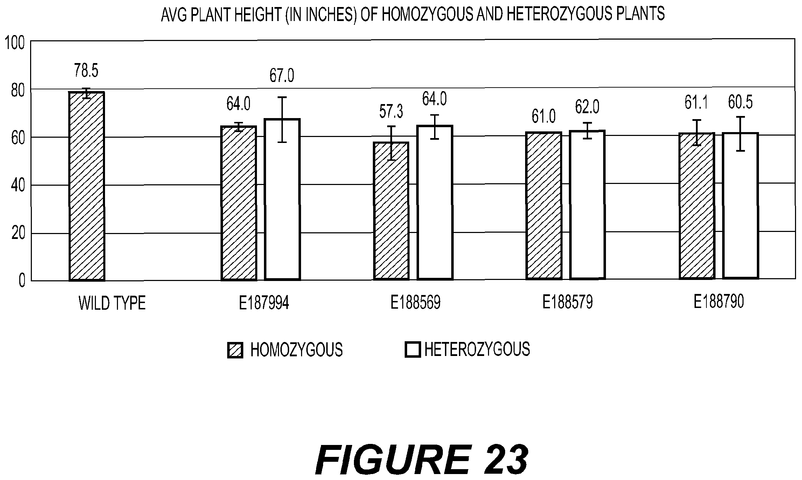

**Figure 23** depicts the average height of wild type plants and homozygous or heterozygous edited plants in inches (Y-axis).

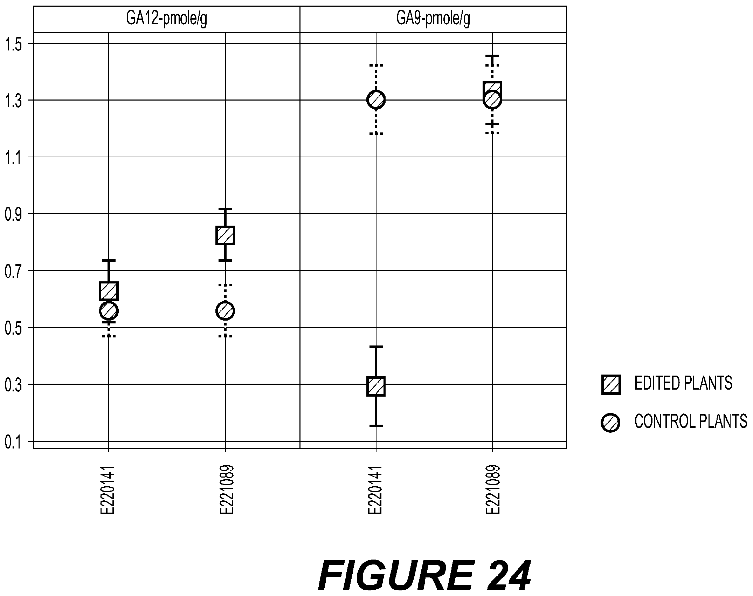

**Figure 24** depicts the concentration of GA12 and GA9 in pmol/g (Y-axis) in edited and control plants.

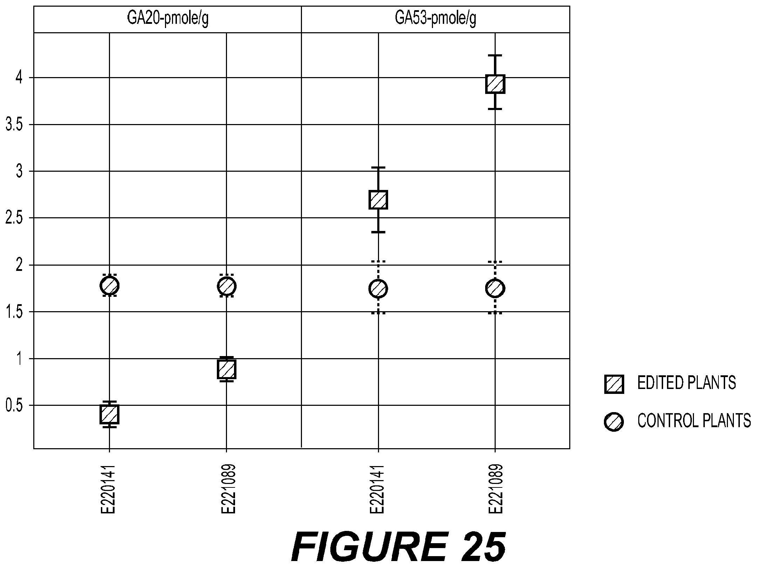

**Figure 25** depicts the concentration of GA20 and GA53 in pmol/g (Y-axis) in edited and control plants.

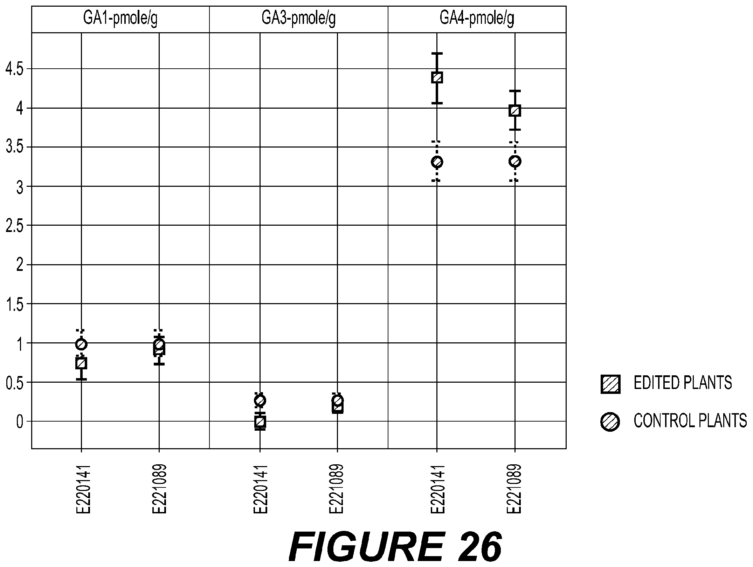

**Figure 26** depicts the concentration of the active gibberellic acids GA1, GA3, and GA4 in pmol/g (Y-axis) in edited and control plants.

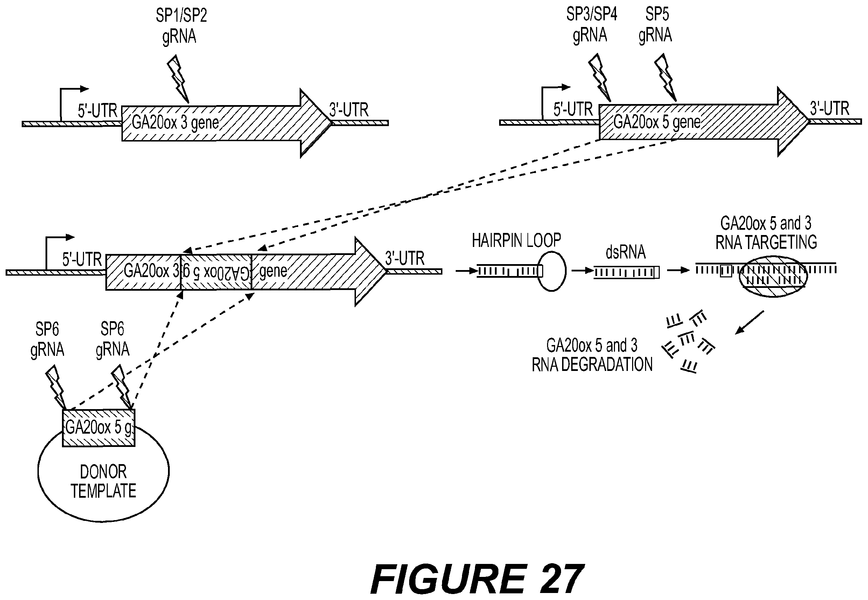

**Figure 27** provides illustrative examples for the production of a genomic modification of the Zm.GA20ox3 locus to encode a RNA transcript with an inverted sequence that can hybridize to a corresponding sequence of the RNA transcript to produce a stem-loop structure, to cause the suppression of one or both copies or alleles at the endogenous Zm.GA20ox3 and Zm.GA20ox5 loci.

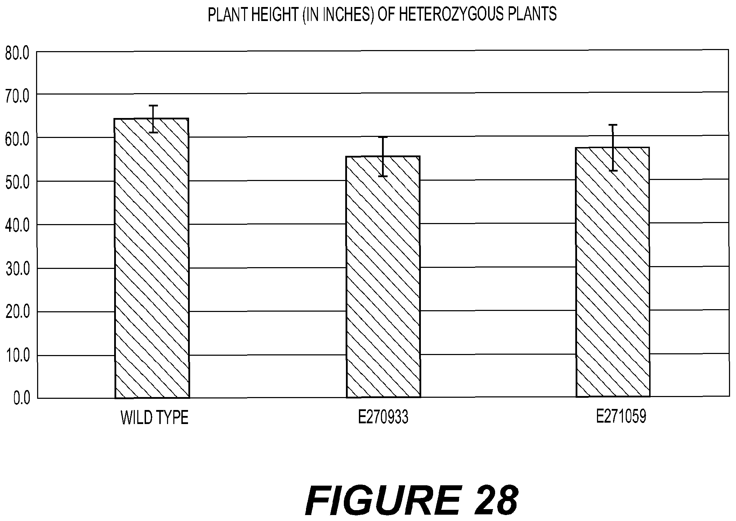

**Figure 28** depicts the average heights of wild type and heterozygous edited corn plants.

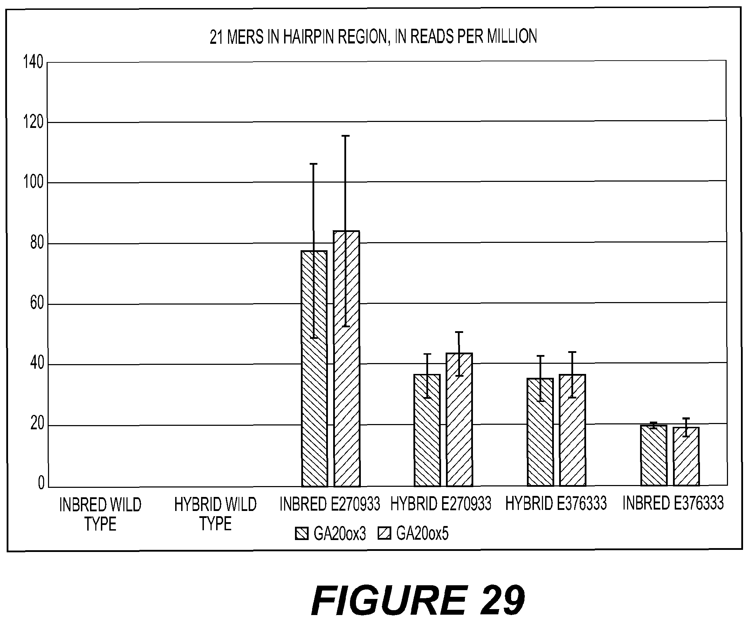

**Figure 29** depicts the number of 21-mer small RNAs (Y-axis) per million reads that mapped to the regions in the stem of the edited stem-loop comprising the inversion sequence from GA20ox5 and a corresponding sequence of the edited GA20ox3 gene that were detected in samples from plants containing edited GA20ox3 alleles.

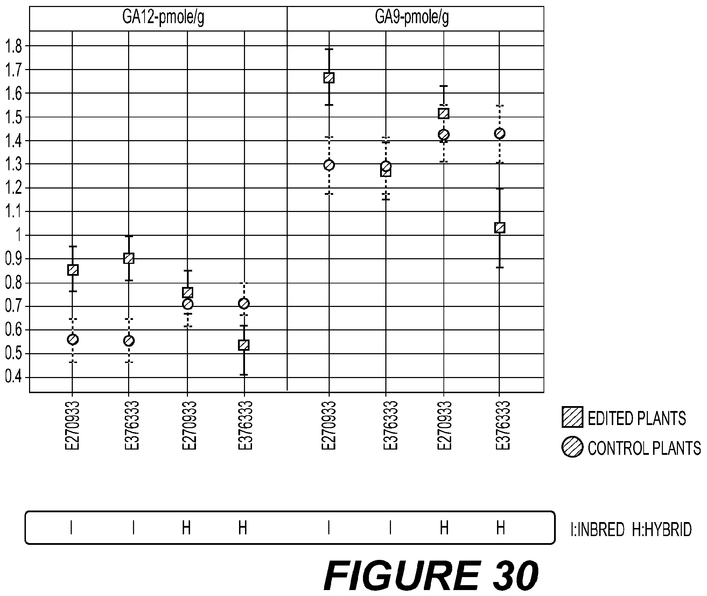

**Figure 30** depicts the concentrations of GA12 and GA9 in pmole/g (Y-axis) in edited and control corn plants.

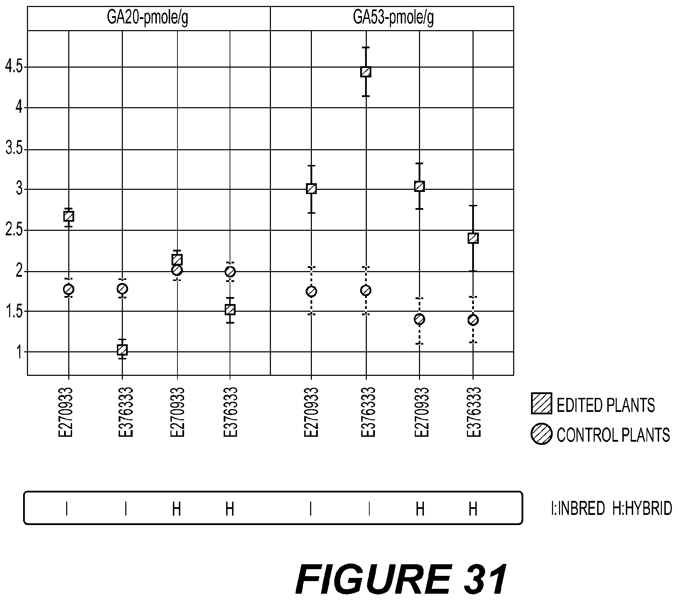

**Figure 31** depicts the concentrations of GA20 and GA53 in pmole/g (Y-axis) in edited and control corn plants.

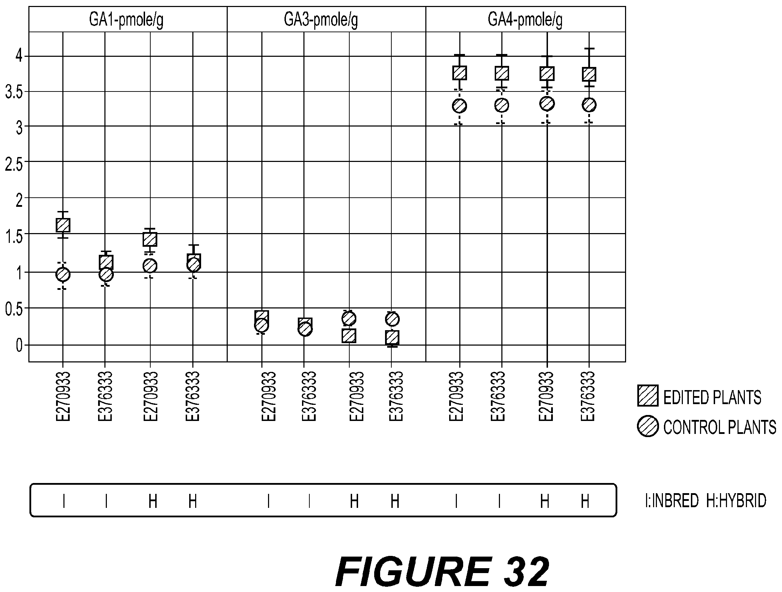

**Figure 32** depicts the concentrations of GA1 and GA3 and GA4 in pmole/g (Y-axis) in edited and control corn plants.

### DETAILED DESCRIPTION

Unless defined otherwise, technical and scientific terms used herein have the same meaning as commonly understood by one of ordinary skill in the art.. For purposes of the present disclosure, the following terms are defined below.

The present specification provides methods and compositions for generating dominant alleles using targeted editing techniques in a wide range of organisms including plants, animals, fungi, and protozoa. Dominant alleles are alleles that mask the contribution of a second allele at the same locus. A dominant allele can be a "dominant negative allele" or a "dominant positive allele." Dominant negative alleles, or antimorphs, are alleles that act in opposition to normal allelic function. A dominant negative allele typically does not function normally and either directly inhibits the activity of a wild-type protein (e.g., through dimerization) or inhibits the activity of a second protein that is required for the normal function of the wild-type protein (e.g., an activator or a downstream component of a pathway). For example, a dominant negative allele abrogates or reduces the normal function of an allele in a heterozygous or homozygous state. Dominant positive alleles can increase or expand normal gene function (*e.g.,* a hypermorph) or provide new functions for a gene (*e.g.,* a neomorph). A semi-dominant allele occurs when penetrance of a linked phenotype in individuals heterozygous for the allele is less than that which is observed in individuals homozygous for the allele.

The practice of the present disclosure employs, unless otherwise indicated, conventional techniques of biochemistry, chemistry, molecular biology, microbiology, cell biology, genomics and biotechnology, which are within the skill of the art. See Green and Sambrook, Molecular Cloning: A Laboratory Manual, 4th Edition (2012); Current Protocols In Molecular Biology (F. M. Ausubel, et al. eds., (1987)); the series Methods In Enzymology (Academic Press, Inc.): PCR 2: A Practical Approach (M. J. MacPherson, B. D. Hames and G. R. Taylor eds. (1995)); Harlow and Lane, eds. (1988) Antibodies, A Laboratory Manual*;* Animal Cell Culture (R. I. Freshney, ed. (1987)); Recombinant Protein Purification: Principles And Methods, 18-1142-75, GE Healthcare Life Sciences; C. N. Stewart, A. Touraev, V. Citovsky, T. Tzfira eds. (2011) Plant Transformation Technologies (Wiley-Blackwell); and R. H. Smith (2013) Plant Tissue Culture. Techniques And Experiments (Academic Press, Inc.).

As used herein, the singular form "a," "an," and "the" include plural references unless the context clearly dictates otherwise. For example, the term "a compound" or "at least one compound" can include a plurality of compounds, including mixtures thereof.

The term "and/or" when used in a list of two or more items, means that any one of the listed items can be employed by itself or in combination with any one or more of the listed items. For example, the expression "A and/or B" is intended to mean either or both of A and B *- i.e.,* A alone, B alone, or A and B in combination. The expression "A, B and/or C" is intended to mean A alone, B alone, C alone, A and B in combination, A and C in combination, B and C in combination, or A, B, and C in combination.

Nucleic acid molecules provided herein include deoxyribonucleic acids (DNA) and ribonucleic acids (RNA) and functional analogues thereof, such as complementary DNA (cDNA). Nucleic acid molecules provided herein can be single stranded or double stranded. Nucleic acid molecules comprise the nucleotide bases adenine (A), guanine (G), thymine (T), cytosine (C). Uracil (U) replaces thymine in RNA molecules. The symbol "N" can be used to represent any nucleotide base *(e.g.,* A, G, C, T, or U). As used herein, "encoding" refers to a polynucleotide encoding for the amino acids of a polypeptide. A series of three nucleotide bases encodes one amino acid. As used herein, "expressed," "expression," or "expressing" refers to transcription of RNA from a DNA molecule. As used herein, terms "polypeptide", "peptide" and "protein" are used interchangeably to refer to a polymer of amino acid residues. The term also applies to amino acid polymers in which one or more amino acids are chemical analogues or modified derivatives of a corresponding naturally-occurring amino acids. A "messenger RNA" or "mRNA" refers to an RNA transcript that is transcribed from a polynucleotide, where the RNA transcript is capable of being translated into a protein. Typically, DNA encodes an mRNA, which encodes a protein. When DNA is transcribed by an RNA polymerase to ultimately generate a protein, a sense mRNA strand is typically produced by the RNA polymerase from the antisense DNA strand. A sense strand of DNA or RNA runs from 5' to 3', while the antisense strand runs from 3' to 5'. Sense and antisense strands of the same polynucleotide are complementary to each other.

In one aspect, a nucleic acid molecule provided herein comprises a protein coding nucleic acid molecule that is codon optimized for a eukaryotic cell. In another aspect, a protein-coding nucleic acid molecule is codon optimized for a plant cell. In another aspect, a protein-coding nucleic acid molecule is codon optimized for a monocot species. In a further aspect, a protein-coding nucleic acid molecule is codon optimized for a corn or soybean cell.

The terms "percent identity" or "percent identical" as used herein in reference to two or more nucleotide or protein sequences is calculated by (i) comparing two optimally aligned sequences (nucleotide or protein) over a window of comparison, (ii) determining the number of positions at which the identical nucleic acid base (for nucleotide sequences) or amino acid residue (for proteins) occurs in both sequences to yield the number of matched positions, (iii) dividing the number of matched positions by the total number of positions in the window of comparison, and then (iv) multiplying this quotient by 100% to yield the percent identity. If the "percent identity" is being calculated in relation to a reference sequence without a particular comparison window being specified, then the percent identity is determined by dividing the number of matched positions over the region of alignment by the total length of the reference sequence. Accordingly, for purposes of the present application, when two sequences (query and subject) are optimally aligned (with allowance for gaps in their alignment), the "percent identity" for the query sequence is equal to the number of identical positions between the two sequences divided by the total number of positions in the query sequence over its length (or a comparison window), which is then multiplied by 100%. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity can be adjusted upwards to correct for the conservative nature of the substitution. Sequences that differ by such conservative substitutions are said to have "sequence similarity" or "similarity."

For optimal alignment of sequences to calculate their percent identity, various pair-wise or multiple sequence alignment algorithms and programs are known in the art, such as ClustalW or Basic Local Alignment Search Tool^{®} (BLAST), etc., that can be used to compare the sequence identity or similarity between two or more nucleotide or protein sequences. Although other alignment and comparison methods are known in the art, the alignment and percent identity between two sequences (including the percent identity ranges described above) can be as determined by the ClustalW algorithm, see, e.g., Chenna R. et al., "Multiple sequence alignment with the Clustal series of programs," Nucleic Acids Research 31: 3497-3500 (2003); Thompson JD et al., "Clustal W: Improving the sensitivity of progressive multiple sequence alignment through sequence weighting, position-specific gap penalties and weight matrix choice," Nucleic Acids Research 22: 4673-4680 (1994); Larkin MA et al., "Clustal W and Clustal X version 2.0," Bioinformatics 23: 2947-48 (2007); and Altschul, S.F., Gish, W., Miller, W., Myers, E.W. & Lipman, D.J. (1990) "Basic local alignment search tool." J. Mol. Biol. 215:403-410 (1990).

The terms "percent complementarity" or "percent complementary" as used herein in reference to two nucleotide sequences is similar to the concept of percent identity but refers to the percentage of nucleotides of a query sequence that optimally base-pair or hybridize to nucleotides a subject sequence when the query and subject sequences are linearly arranged and optimally base paired without secondary folding structures, such as loops, stems or hairpins. Such a percent complementarity can be between two DNA strands, two RNA strands, or a DNA strand and a RNA strand. The "percent complementarity" can be calculated by (i) optimally base-pairing or hybridizing the two nucleotide sequences in a linear and fully extended arrangement (i.e., without folding or secondary structures) over a window of comparison, (ii) determining the number of positions that base-pair between the two sequences over the window of comparison to yield the number of complementary positions, (iii) dividing the number of complementary positions by the total number of positions in the window of comparison, and (iv) multiplying this quotient by 100% to yield the percent complementarity of the two sequences. Optimal base pairing of two sequences can be determined based on the known pairings of nucleotide bases, such as G-C, A-T, and A-U, through hydrogen binding. If the "percent complementarity" is being calculated in relation to a reference sequence without specifying a particular comparison window, then the percent identity is determined by dividing the number of complementary positions between the two linear sequences by the total length of the reference sequence. Thus, for purposes of the present application, when two sequences (query and subject) are optimally base-paired (with allowance for mismatches or non-base-paired nucleotides), the "percent complementarity" for the query sequence is equal to the number of base-paired positions between the two sequences divided by the total number of positions in the query sequence over its length, which is then multiplied by 100%.

The term "operably linked" refers to a functional linkage between a promoter or other regulatory element and an associated transcribable DNA sequence or coding sequence of a gene (or transgene), such that the promoter, etc., operates to initiate, assist, affect, cause, and/or promote the transcription and expression of the associated transcribable DNA sequence or coding sequence, at least in certain tissue(s), developmental stage(s) and/or condition(s). In addition to promoters, regulatory elements include, without being limiting, an enhancer, a leader, a transcription start site (TSS), a linker, 5' and 3' untranslated regions (UTRs), an intron, a polyadenylation signal, and a termination region or sequence, etc., that are suitable, necessary or preferred for regulating or allowing expression of the gene or transcribable DNA sequence in a cell. Such additional regulatory element(s) can be optional and used to enhance or optimize expression of the gene or transcribable DNA sequence. For purposes of the present application, an "enhancer" can be distinguished from a "promoter" in that an enhancer typically lacks a transcription start site, TATA box, or equivalent sequence and is thus insufficient alone to drive transcription. As used herein, a "leader" can be defined generally as the DNA sequence of the 5'-UTR of a gene (or transgene) between the transcription start site (TSS) and 5' end of the transcribable DNA sequence or protein coding sequence start site of the gene.

As commonly understood in the art, the term "promoter" refers to a DNA sequence that contains an RNA polymerase binding site, transcription start site, and/or TATA box and assists or promotes the transcription and expression of an associated transcribable polynucleotide sequence and/or gene (or transgene). A promoter can be synthetically produced, varied or derived from a known or naturally occurring promoter sequence or other promoter sequence. A promoter can also include a chimeric promoter comprising a combination of two or more heterologous sequences. A promoter of the present application can thus include variants of promoter sequences that are similar in composition, but not identical to, other promoter sequence(s) known or provided herein. A promoter can be classified according to a variety of criteria relating to the pattern of expression of an associated coding or transcribable sequence or gene (including a transgene) operably linked to the promoter, such as constitutive, developmental, tissue-specific, inducible, etc. Promoters that drive expression in all or most tissues of the plant are referred to as "constitutive" promoters. Promoters that drive expression during certain periods or stages of development are referred to as "developmental" promoters. Promoters that drive enhanced expression in certain tissues of the plant relative to other plant tissues are referred to as "tissue-enhanced" or "tissue-preferred" promoters. Thus, a "tissue-preferred" promoter causes relatively higher or preferential expression in a specific tissue(s) of the plant, but with lower levels of expression in other tissue(s) of the plant. Promoters that express within a specific tissue(s) of the plant, with little or no expression in other plant tissues, are referred to as "tissue-specific" promoters. An "inducible" promoter is a promoter that initiates transcription in response to an environmental stimulus such as cold, drought or light, or other stimuli, such as wounding or chemical application. A promoter can also be classified in terms of its origin, such as being heterologous, homologous, chimeric, synthetic, etc. A "heterologous" promoter is a promoter sequence having a different origin relative to its associated transcribable sequence, coding sequence, or gene (or transgene), and/or not naturally occurring in the plant species to be transformed.

Examples describing a promoter that can be used herein include without limitation U.S. Pat. No. 6,437,217 (maize RS81 promoter), U.S. Pat. No. 5,641,876 (rice actin promoter), U.S. Pat. No. 6,426,446 (maize RS324 promoter), U.S. Pat. No. 6,429,362 (maize PR-1 promoter), U.S. Pat. No. 6,232,526 (maize A3 promoter), U.S. Pat. No. 6,177,611 (constitutive maize promoters), U.S. Pat. Nos. 5,322,938, 5,352,605, 5,359,142 and 5,530,196 (35S promoter), U.S. Pat. No. 6,433,252 (maize L3 oleosin promoter), U.S. Pat. No. 6,429,357 (rice actin 2 promoter as well as a rice actin 2 intron), U.S. Pat. No. 5,837,848 (root specific promoter), U.S. Pat. No. 6,294,714 (light inducible promoters), U.S. Pat. No. 6,140,078 (salt inducible promoters), U.S. Pat. No. 6,252,138 (pathogen inducible promoters), U.S. Pat. No. 6,175,060 (phosphorus deficiency inducible promoters), U.S. Pat. No. 6,635,806 (gamma-coixin promoter), and U.S. patent application Ser. No. 09/757,089 (maize chloroplast aldolase promoter). Additional promoters that can find use are a nopaline synthase (NOS) promoter (Ebert et al., 1987), the octopine synthase (OCS) promoter (which is carried on tumor-inducing plasmids of *Agrobacterium tumefaciens*)*,* the caulimovirus promoters such as the cauliflower mosaic virus (CaMV) 19S promoter (Lawton et al., Plant Molecular Biology (1987) 9: 315-324), the CaMV 35S promoter (Odell et al., Nature (1985) 313: 810-812), the figwort mosaic virus 35S-promoter (U.S. Pat. Nos. 6,051,753; 5,378,619), the sucrose synthase promoter (Yang and Russell, Proceedings of the National Academy of Sciences, USA (1990) 87: 4144-4148), the R gene complex promoter (Chandler et al., Plant Cell (1989) 1: 1175-1183), and the chlorophyll a/b binding protein gene promoter, PC1SV (U.S. Pat. No. 5,850,019), and AGRtu.nos (GenBank Accession V00087; Depicker et al., Journal of Molecular and Applied Genetics (1982) 1: 561-573; Bevan et al., 1983) promoters.

Promoter hybrids can also be used and constructed to enhance transcriptional activity (*see* U.S. Pat. No. 5,106,739), or to combine desired transcriptional activity, inducibility and tissue specificity or developmental specificity. Promoters that function in plants include but are not limited to promoters that are inducible, viral, synthetic, constitutive, temporally regulated, spatially regulated, and spatio-temporally regulated. Other promoters that are tissue-enhanced, tissue-specific, or developmentally regulated are also known in the art and envisioned to have utility in the practice of this disclosure.

As used herein, the term "heterologous" in reference to a promoter is a promoter sequence having a different origin relative to its associated transcribable DNA sequence, coding sequence or gene (or transgene), and/or not naturally occurring in the plant species to be transformed. The term "heterologous" can refer more broadly to a combination of two or more DNA molecules or sequences, such as a promoter and an associated transcribable DNA sequence, coding sequence or gene, when such a combination is man-made and not normally found in nature.

In addition, the term "recombinant" in reference to a polynucleotide (DNA or RNA) molecule, protein, construct, vector, etc., refers to a polynucleotide or protein molecule or sequence that is man-made and not normally found in nature, and/or is present in a context in which it is not normally found in nature, including a polynucleotide (DNA or RNA) molecule, protein, construct, etc., comprising a combination of polynucleotide or protein sequences that would not naturally occur contiguously or in close proximity together without human intervention, and/or a polynucleotide molecule, protein, construct, etc., comprising at least two polynucleotide or protein sequences that are heterologous with respect to each other. A recombinant polynucleotide or protein molecule, construct, etc., can comprise polynucleotide or protein sequence(s) that is/are (i) separated from other polynucleotide or protein sequence(s) that exist in proximity to each other in nature, and/or (ii) adjacent to (or contiguous with) other polynucleotide or protein sequence(s) that are not naturally in proximity with each other. Such a recombinant polynucleotide molecule, protein, construct, etc., can also refer to a polynucleotide or protein molecule or sequence that has been genetically engineered and/or constructed outside of a cell. For example, a recombinant DNA molecule can comprise any suitable plasmid, vector, etc., and can include a linear or circular DNA molecule. Such plasmids, vectors, etc., can contain various maintenance elements including a prokaryotic origin of replication and selectable marker, as well as one or more transgenes or expression cassettes perhaps in addition to a plant selectable marker gene, etc.

As used herein, "adjacent" refers to a nucleic acid sequence that is in close proximity, or next to another nucleic acid sequence. In one aspect, adjacent nucleic acid sequences are physically linked. In another aspect, adjacent nucleic acid sequences or genes are immediately next to each other such that there are no intervening nucleotides between the end of a first nucleic acid sequence and the start of a second nucleic acid sequence. In an aspect, a first gene and a second gene are adjacent to each other if they are separated by less than 50,000, less than 25,000, less than 10,000, less than 9000, less than 8000, less than 7000, less than 6000, less than 5000, less than 4000, less than 3000, less than 2500, less than 2000, less than 1750, less than 1500, less than 1250, less than 1000, less than 900, less than 800, less than 700, less than 600, less than 500, less than 400, less than 300, less than 200, less than 100, less than 75, less than 50, less than 25, less than 20, less than 10, less than 5, less than 4, less than 3, less than 2, or less than 1 nucleotides.

In one aspect, methods and compositions provided herein comprise a vector. As used herein, the terms "vector" or "plasmid" are used interchangeably and refer to a circular, double-stranded DNA molecule that is physically separate from chromosomal DNA. In one aspect, a plasmid or vector used herein is capable of replication *in vivo.* A "transformation vector," as used herein, is a plasmid that is capable of transforming a plant cell. In an aspect, a plasmid provided herein is a bacterial plasmid. In another aspect, a plasmid provided herein is *an Agrobacterium* Ti plasmid or derived from *an Agrobacterium* Ti plasmid.

In one aspect, a plasmid or vector provided herein is a recombinant vector. As used herein, the term "recombinant vector" refers to a vector formed by laboratory methods of genetic recombination, such as molecular cloning. In another aspect, a plasmid provided herein is a synthetic plasmid. As used herein, a "synthetic plasmid" is an artificially created plasmid that is capable of the same functions (*e.g.,* replication) as a natural plasmid (*e.g.,* Ti plasmid). Without being limited, one skilled in the art can create a synthetic plasmid *de novo* via synthesizing a plasmid by individual nucleotides, or by splicing together nucleic acid molecules from different pre-existing plasmids.

As used herein, "modified", in the context of plants, seeds, plant components, plant cells, and plant genomes, refers to a state containing changes or variations from their natural or native state. For instance, a "native transcript" of a gene refers to an RNA transcript that is generated from an unmodified gene. Typically, a native transcript is a sense transcript. Modified plants or seeds contain molecular changes in their genetic materials, including either genetic or epigenetic modifications. Typically, modified plants or seeds, or a parental or progenitor line thereof, have been subjected to mutagenesis, genome editing (e.g., without being limiting, via methods using site-specific nucleases), genetic transformation (e.g., without being limiting, via methods of *Agrobacterium* transformation or microprojectile bombardment), or a combination thereof. In one aspect, a modified plant described herein comprises no non-plant genetic material or sequences. In yet another aspect, a modified plant described herein comprises no interspecies genetic material or sequences. In one aspect, this disclosure describes methods and compositions related to modified plants, seeds, plant components, plant cells, and products made from modified plants, seeds, plant parts, and plant cells. In one aspect, a modified seed described herein gives rise to a modified plant provided herein. In one aspect, a modified plant, seed, plant component, plant cell, or plant genome described herein comprises a recombinant DNA construct or vector provided herein. In another aspect, a product described herein comprises modified a plant, plant component, plant cell, or plant chromosome or genome provided herein. The present disclosure describes modified plants with desirable or enhanced properties, e.g., without being limiting, disease, insect, or pest tolerance (for example, virus tolerance, bacteria tolerance, fungus tolerance, nematode tolerance, arthropod tolerance, gastropod tolerance); herbicide tolerance; environmental stress resistance; quality improvements such as yield, nutritional enhancements, environmental or stress tolerances; any desirable changes in plant physiology, growth, development, morphology or plant product(s) including starch production, modified oils production, high oil production, modified fatty acid content, high protein production, fruit ripening, enhanced animal and human nutrition, biopolymer production, pharmaceutical peptides and secretable peptides production; improved processing traits; improved digestibility; low raffinose; industrial enzyme production; improved flavor; nitrogen fixation; hybrid seed production; and fiber production.

As used herein, "genome editing" or editing refers to targeted mutagenesis, insertion, deletion, inversion, substitution, or translocation of a nucleotide sequence of interest in a genome using a targeted editing technique. A nucleotide sequence of interest can be of any length, *e.g.,* at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 75, at least 100, at least 250, at least 500, at least 1000, at least 2500, at least 5000, at least 10,000, or at least 25,000 nucleotides. As used herein, a "targeted editing technique" refers to any method, protocol, or technique that allows the precise and/or targeted editing of a specific location in a genome (e.g., the editing is not random). Without being limiting, use of a site-specific nuclease is one example of a targeted editing technique. Another non-limiting example of a targeted editing technique is the use of one or more tether guide Oligos (tgOligos). As used herein, a "targeted edit" refers to a targeted mutagenesis, insertion, deletion, inversion, or substitution caused by a targeted editing technique. A nucleotide sequence of interest can be an endogenous genomic sequence or a transgenic sequence.

**In** one aspect, a "targeted editing technique" refers to any method, protocol, or technique that allows the precise and/or targeted editing of a specific location in a genome *(e.g.,* the editing is not random). Without being limiting, use of a site-specific nuclease is one example of a targeted editing technique.

**In** one aspect, a targeted editing technique is used to edit an endogenous locus or an endogenous gene. In another aspect, a targeted editing technique is used to edit a transgene. As used herein, an "endogenous gene" or a "native copy" of a gene refers to a gene that originates from within a given organism, cell, tissue, genome, or chromosome. An "endogenous gene" or a "native copy" of a gene is a gene that was not previously modified by human action.

As used herein, a "locus" refers to a specific position on a chromosome or other nucleic acid molecule. Without being limiting, a locus can comprise a polynucleotide that encodes a protein or an RNA. A locus can also comprise a non-coding RNA. A locus can comprise a gene. A locus can comprise a promoter, a 5'-untranslated region (UTR), an exon, an intron, a 3'-UTR, or any combination thereof. A locus can comprise a coding region.

As used herein, "physically linked" refers to two or more loci that are positioned on the same nucleic acid molecule.

As used herein, a "coding region," a "gene region," or a "gene" refers to a polynucleotide that can produce a functional unit (e.g., without being limiting, for example, a protein, or a non-coding RNA molecule). A "coding region," "gene," or "gene region" can comprise a promoter, an enhancer sequence, a leader sequence, a transcriptional start site, a transcriptional stop site, a polyadenylation site, one or more exons, one or more introns, a 5'-UTR, a 3'-UTR, or any combination thereof. A "coding region sequence," a "gene sequence," or a "gene region sequence" can comprise a polynucleotide sequence encoding a promoter, an enhancer sequence, a leader sequence, a transcriptional start site, a transcriptional stop site, a polyadenylation site, one or more exons, one or more introns, a 5'-UTR, a 3'-UTR, or any combination thereof. In one aspect, a "gene" encodes a non-coding RNA molecule or a precursor thereof. In another aspect, a "gene" encodes a protein.

Non-limiting examples of a non-coding RNA molecule include a microRNA (miRNA), a miRNA precursor (pre-miRNA), a small interfering RNA (siRNA), a small RNA (18-26 nt in length) and precursor encoding same, a heterochromatic siRNA (hc-siRNA), a Piwi-interacting RNA (piRNA), a hairpin double strand RNA (hairpin dsRNA), a *trans-*acting siRNA (ta-siRNA), a naturally occurring antisense siRNA (nat-siRNA), a CRISPR RNA (crRNA), a tracer RNA (tracrRNA), a guide RNA (gRNA), and a single-guide RNA (sgRNA). In one aspect, a non-coding RNA provided herein is selected from the group consisting of a microRNA, a small interfering RNA, a secondary small interfering RNA, a transfer RNA, a ribosomal RNA, a *trans-acting* small interfering RNA, a naturally occurring antisense small interfering RNA, a heterochromatic small interfering RNA, and precursors thereof. In another aspect, a non-coding RNA provided herein is selected from the group consisting of a miRNA, a pre-miRNA, a siRNA, a hc-siRNA, a piRNA, a hairpin dsRNA, a ta-siRNA, a nat-siRNA, a crRNA, a tracrRNA, a gRNA, and a sgRNA. Non-coding RNAs are often 100% complementary, or at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99%, at least 99.5% complementary, to a non-coding RNA target site. A non-coding RNA target site can be present in a DNA molecule or an RNA molecule. Non-coding RNA target sites can be hybridized (or bound) by a non-coding RNA to effect various outcomes. For instance, some non-coding RNAs (without being limiting, *e.g*., miRNA, siRNA, ta-siRNA) assist in the cleavage of mRNA transcripts comprising a complementary non-coding RNA target site. Alternatively, a non-coding RNA (without being limiting, *e.g*., miRNA, siRNA, ta-siRNA) can assist with the inhibition of protein translation of an mRNA transcript by binding to a non-coding RNA target site in the mRNA. Some non-coding RNAs (without being limiting, e.g., hc-siRNA) assist with inducing epigenetic changes to DNA. In one aspect, a non-coding RNA target site is a miRNA target site or an siRNA target site. In another aspect, a gene provided herein comprises at least one, at least two, at least three, at least four, at least five, at least six, at least seven, at least eight, at least nine, or at least ten non-coding RNA target sites. In another aspect, a gene provided herein comprises at least one, at least two, at least three, at least four, at least five, at least six, at least seven, at least eight, at least nine, or at least ten heterologous non-coding RNA target sites. In another aspect, a dominant negative allele provided herein comprises at least one, at least two, at least three, at least four, at least five, at least six, at least seven, at least eight, at least nine, or at least ten non-coding RNA target sites. In another aspect, a dominant negative allele provided herein comprises at least one, at least two, at least three, at least four, at least five, at least six, at least seven, at least eight, at least nine, or at least ten heterologous non-coding RNA target sites. In an aspect, an endogenous gene provided herein is modified to comprise at least one, at least two, at least three, at least four, at least five, at least six, at least seven, at least eight, at least nine, or at least ten heterologous non-coding RNA target sites.

As used herein, an "allele" refers to a variant of a given locus or gene in a genome. If the same allele is present on both chromosomes of a chromosome pair in a cell the cell is considered homozygous at the given locus. If each member of the chromosome pair comprises a different allele for the given locus the cell is heterozygous for the locus. A minimum of one allele is possible for a given locus, although typically multiple alleles are possible for any given locus in a genome.

As used herein, the terms "suppress," "inhibit," "inhibition," "inhibiting", and "downregulation" are defined as any method known in the art or described herein that decreases the expression or function of a gene product (e.g., an mRNA, a protein, a non-coding RNA). "Inhibition" can be in the context of a comparison between two cells, for example, a modified cell versus a control cell. Inhibition of expression or function of a gene product can also be in the context of a comparison between plant cells, organelles, organs, tissues, or plant components within the same plant or between different plants, and includes comparisons between developmental or temporal stages within the same plant or plant component or between plants or plant components. "Inhibition" includes any relative decrement of function or production of a gene product of interest, up to and including complete elimination of function or production of that gene product. The term "inhibition" encompasses any method or composition that down-regulates translation and/or transcription of the target gene product or functional activity of the target gene product. "Inhibition" need not comprise complete elimination of expression of a gene product. In an aspect, a gene product in a modified cell provided herein comprises expression that is at least 1%, at least 2%, at least 3%, at least 4%, at least 5%, at least 10%, at least 20%, at least 25%, at least 30%, at least 40%, at least 50%, at least 60%, at least 70%, at least 80%, at least 90%, at least 95%, or 100% lower than the expression of the gene product in a control cell. In another aspect, a gene product in a modified cell provided herein comprises expression that is between 1% and 100%, between 1% and 95%, between 1% and 90%, between 1% and 80%, between 1% and 70%, between 1% and 60%, between 1% and 50%, between 1% and 40%, between 1% and 30%, between 1% and 25%, between 1% and 20%, between 1% and 15%, between 1% and 10%, between 1% and 5%, between 5% and 25%, between 5% and 50%, between 5% and 75%, between 5% and 100%, between 10% and 25%, between 10% and 50%, between 10% and 75%, between 10% and 100%, between 25% and 50%, between 25% and 75%, between 25% and 100%, or between 50% and 100% lower than the expression of the gene product in a control cell.

As used herein, a "target site" refers to a location of a polynucleotide sequence that is bound to and cleaved by a site-specific nuclease introducing a double stranded break into the nucleic acid backbone. In another aspect a target site comprises at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 29, or at least 30 consecutive nucleotides. In another aspect, a target site provided herein is at least 10, at least 20, at least 30, at least 40, at least 50, at least 75, at least 100, at least 125, at least 150, at least 200, at least 250, at least 300, at least 400, or at least 500 nucleotides. In one aspect a site-specific nuclease binds to a target site. In another aspect a site-specific nuclease binds to a target site via a guiding non-coding RNA *(i.e.,* such as, without being limiting, a CRISPR RNA or single-guide RNA (both described in detail below)). In one aspect, a non-coding RNA provided herein is complementary to a target site. It will be appreciated that perfect complementarity is not required for a non-coding RNA to bind to a target site; at least 1, at least 2, at least 3, at least 4, or at least 5, at least 6, at least 7 or at least 8 mismatches between a target site and a non-coding RNA can be tolerated. As used herein, a "target region" or a "targeted region" refers to a polynucleotide sequence that desired to be modified. In one aspect, a "target region," "targeted region", or a "target gene" is flanked by two or more, three or more, four or more, five or more, six or more, seven or more, eight or more, nine or more, or ten or more target sites. A "target gene" refers to a polynucleotide sequence encoding a gene that is desired to be modified. In one aspect, a polynucleotide sequence comprising a target gene further comprises one or more target sites. In another aspect, a target region comprises one or more, two or more, three or more, four or more, five or more, six or more, seven or more, eight or more, nine or more, or ten or more target genes. Without being limiting, in one aspect a target region can be subject to deletion or inversion. As used herein, "flanked" when used to describe a target region, refers to two or more target sites physically surrounding the target region, with one target site on each side of the target region.

A target site can be positioned in a polynucleotide sequence encoding a leader, an enhancer, a transcriptional start site, a promoter, a 5'-UTR, an exon, an intron, a 3'-UTR, a polyadenylation site, or a termination sequence. It will be appreciated that a target site can be also be positioned upstream or downstream of a sequence encoding a leader, an enhancer, a transcriptional start site, a promoter, a 5'-UTR, an exon, an intron, a 3'-UTR, a polyadenylation site, or a termination sequence. In one aspect, a target site is positioned within 10, within 20, within 30, within 40, within 50, within 75, within 100, within 125, within 150, within 200, within 250, within 300, within 400, within 500, within 600, within 700, within 800, within 900, within 1000, within 1250, within 1500, within 2000, within 2500, within 5000, within 10,000, or within 25,000 nucleotides of a polynucleotide encoding a leader, an enhancer, a transcriptional start site, a promoter, a 5'-UTR, an exon, an intron, a 3'-UTR, a polyadenylation site, a gene, or a termination sequence.

As used herein, "upstream" refers to a nucleic acid sequence that is positioned before the 5' end of a linked nucleic acid sequence. As used herein, "downstream" refers to a nucleic acid sequence is positioned after the 3' end of a linked nucleic acid sequence. As used herein, "5'" refers to the start of a coding DNA sequence or the beginning of an RNA molecule. As used herein, "3'" refers to the end of a coding DNA sequence or the end of an RNA molecule. It will be appreciated that an "inversion" refers to reversing the orientation of a given polynucleotide sequence. For example, if the sample sequence 5'-ATGATC-3' is inverted it will read 5'-CTAGTA-3' in reverse orientation. Additionally, the sample sequence 5'-ATGATC-3' is considered to be in "opposite orientation" to the sample sequence 5'-CTAGTA-3'.

The present application describes methods of screening and selecting cells for targeted edits and methods of selecting cells comprising targeted edits. Nucleic acids can be isolated using various techniques. For example, nucleic acids can be isolated using any method including, without limitation, recombinant nucleic acid technology, and/or the polymerase chain reaction (PCR). General PCR techniques are described, for example in PCR Primer: A Laboratory Manual, Dieffenbach & Dveksler, Eds., Cold Spring Harbor Laboratory Press, 1995. Recombinant nucleic acid techniques include, for example, restriction enzyme digestion and ligation, which can be used to isolate a nucleic acid. Isolated nucleic acids also can be chemically synthesized, either as a single nucleic acid molecule or as a series of oligonucleotides. Polypeptides can be purified from natural sources (e.g., a biological sample) by known methods such as DEAE ion exchange, gel filtration, and hydroxyapatite chromatography. A polypeptide also can be purified, for example, by expressing a nucleic acid in an expression vector. In addition, a purified polypeptide can be obtained by chemical synthesis. The extent of purity of a polypeptide can be measured using any appropriate method, e.g., column chromatography, polyacrylamide gel electrophoresis, or HPLC analysis.

This disclosure describes methods of detecting recombinant nucleic acids and polypeptides in modified and unmodified plant cells. Without being limiting, nucleic acids also can be detected using hybridization. Hybridization between nucleic acids is discussed in detail in Sambrook et al. (1989, Molecular Cloning: A Laboratory Manual, 2nd Ed., Cold Spring Harbor Laboratory Press, Cold Spring Harbor, NY).

Polypeptides can be detected using antibodies. Techniques for detecting polypeptides using antibodies include enzyme linked immunosorbent assays (ELISAs), Western blots, immunoprecipitations and immunofluorescence. An antibody provided herein can be a polyclonal antibody or a monoclonal antibody. An antibody having specific binding affinity for a polypeptide provided herein can be generated using methods well known in the art. An antibody provided herein can be attached to a solid support such as a microtiter plate using methods known in the art.

Detection (e.g., of an amplification product, of a hybridization complex, of a polypeptide) can be accomplished using detectable labels. The term "label" is intended to encompass the use of direct labels as well as indirect labels. Detectable labels include enzymes, prosthetic groups, fluorescent materials, luminescent materials, bioluminescent materials, and radioactive materials.

The screening and selection of modified, engineered, or transgenic plants or plant cells can be through any methodologies known to those having ordinary skill in the art. Examples of screening and selection methodologies include, but are not limited to, Southern analysis, PCR amplification for detection of a polynucleotide, Northern blots, RNase protection, primer-extension, RT-PCR amplification for detecting RNA transcripts, Sanger sequencing, Next Generation sequencing technologies (e.g., Illumina, PacBio, Ion Torrent, 454) enzymatic assays for detecting enzyme or ribozyme activity of polypeptides and polynucleotides, and protein gel electrophoresis, Western blots, immunoprecipitation, and enzyme-linked immunoassays to detect polypeptides. Other techniques such as *in situ* hybridization, enzyme staining, and immunostaining also can be used to detect the presence or expression of polypeptides and/or polynucleotides. Methods for performing all of the referenced techniques are known.

Genome editing or targeted editing can be effected via the use of one or more site-specific nucleases. Site-specific nucleases can induce a double-stranded break (DSB) at a target site of a genome sequence that is then repaired by the natural processes of either homologous recombination (HR) or non-homologous end-joining (NHEJ). Sequence modifications, such as insertions, deletions, can occur at the DSB locations via NHEJ repair. If two DSBs flanking one target region are created, the breaks can be repaired via NHEJ by reversing the orientation of the targeted DNA (also referred to as an "inversion"). HR can be used to integrate a donor nucleic acid sequence into a target site. Without being limited by any theory, in order to integrate a donor nucleic acid sequence (or donor molecule) into a DSB, the donor molecule comprises a polynucleotide of interest flanked by a first and second homologous region, where the first and second homologous regions are homologous to each side of the DSB at the target site. Homologous recombination machinery in the cell then repairs the DSB by integrating the donor molecule into the target site. A double-stranded break described herein may be repaired by NHEJ. A double-stranded break described herein may be repaired by HR.

Although a double-stranded break only occurs between two nucleotides on each strand, a double-stranded break site can comprise at least 2, at least 3, at least 4, at least 5, at least 6, at least 7, at least 8, at least 9, at least 10, at least 15, at least 20, at least 25, at least 30, at least 40, at least 50, at least 60, at least 70, at least 75, at least 80, at least 90, or at least 100 nucleotides. As used herein, a "double-stranded break site" refers to a polynucleotide sequence that is recognized and bound by a site-specific nuclease or guide RNA.

A vector or construct described herein comprises polynucleotides encoding at least 1, at least 2, at least 3, at least 4, at least 5, at least 6, at least 7, at least 8, at least 9, or at least 10 site-specific nuclease. In another aspect, a cell provided herein already comprises a site-specific nuclease. In an aspect, a polynucleotide encoding a site-specific nuclease provided herein is stably transformed into a cell. A polynucleotide encoding a site-specific nuclease described herein may be transiently transformed into a cell. A polynucleotide encoding a site-specific nuclease may be under the control of a regulatable promoter, a constitutive promoter, a tissue specific promoter, or any promoter useful for expression of the site-specific nuclease.

A vector may comprise *in cis* a cassette encoding a site-specific nuclease and a donor molecule such that when contacted with the genome of a cell, the site-specific nuclease enables site-specific integration of the donor molecule. A first vector may comprise a cassette encoding a site-specific nuclease and a second vector may comprise a donor molecule such that when contacted with the genome of a cell, the site-specific nuclease provided *in trans* enables site-specific integration of the donor molecule.

Site-specific nucleases described herein can be used as part of a targeted editing technique. Non-limiting examples of site-specific nucleases used in methods and/or compositions described herein include meganucleases, zinc finger nucleases (ZFNs), transcription activator-like effector nucleases (TALENs), RNA-guided nucleases (e.g., Cas9 and Cpf1), a recombinase (without being limiting, for example, a serine recombinase attached to a DNA recognition motif, a tyrosine recombinase attached to a DNA recognition motif), a transposase (without being limiting, for example, a DNA transposase attached to a DNA binding domain), or any combination thereof. A method described herein may comprise the use of one or more, two or more, three or more, four or more, or five or more site-specific nucleases to induce one, two, three, four, five, or more than five DSBs at one, two, three, four, five, or more than five target sites.

A site-specific nuclease protein is provided to a cell. In another aspect, a nucleic acid sequence (e.g., a vector) encoding a site-specific nuclease protein is provided to a cell. In another aspect, a site-specific nuclease protein and a guide RNA are provided to a cell separately. A site-specific nuclease protein and a guide RNA may be provided to a cell as a complex. A site-specific nuclease protein and a guide RNA may be assembled into a complex *in vitro, in vivo,* or *ex vivo.*

A genome editing system described herein (e.g., a meganuclease, a ZFN, a TALEN, a CRISPR/Cas9 system, a CRISPR/Cpf1 system, a recombinase, a transposase), or a combination of genome editing systems described herein, may be used in a method to introduce one or more insertions, deletions, substitutions, or inversions to a locus in a cell to generate a dominant negative allele or a dominant positive allele.

Site-specific nucleases, such as meganucleases, ZFNs, TALENs, Argonaute proteins (non-limiting examples of Argonaute proteins include *Thermus thermophilus* Argonaute (TtAgo), *Pyrococcus furiosus* Argonaute (PfAgo), *Natronobacterium gregoryi* Argonaute (NgAgo), homologs thereof, or modified versions thereof), Cas9 nucleases (non-limiting examples of RNA-guided nucleases include Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmrl, Cmr3, Cmr4, Cmr5, Cmr6, Csbl, Csb2, Csb3, Csx17, Csx14, Csx10, Csx16, CsaX, Csx3, Csx1, Csx15, Csfl, Csf2, Csf3, Csf4, Cpfl, CasX, CasY, homologs thereof, or modified versions thereof), induce a double-strand DNA break at the target site of a genomic sequence that is then repaired by the natural processes of HR or NHEJ. Sequence modifications then occur at the cleaved sites, which can include inversions, deletions, or insertions that result in gene disruption in the case of NHEJ, or integration of nucleic acid sequences by HR.

A site-specific nuclease described herein can be selected from the group consisting of a zinc-finger nuclease, a meganuclease, an RNA-guided nuclease, a TALE-nuclease, a recombinase, a transposase, or any combination thereof. A site-specific nuclease described herein may be selected from the group consisting of a Cas9 or a Cpfl. A site-specific nuclease described herein may be selected from the group consisting of a Cas1, a Cas1B, a Cas2, a Cas3, a Cas4, a Cas5, a Cas6, a Cas7, a Cas8, a Cas9, a Cas10, a Csy1, a Csy2, a Csy3, a Csel, a Cse2, a Csc1, a Csc2, a Csa5, a Csn2, a Csm2, a Csm3, a Csm4, a Csm5, a Csm6, a Cmrl, a Cmr3, a Cmr4, a Cmr5, a Cmr6, a Csbl, a Csb2, a Csb3, a Csx17, a Csx14, a Csx10, a Csx16, a CsaX, a Csx3, a Csx1, a Csx15, a Csfl, a Csf2, a Csf3, a Csf4, a Cpfl, a CasX, a CasY, a homolog thereof, or a modified version thereof. An RNA-guided nuclease descrobed herein may be selected from the group consisting of a Cas9 or a Cpfl. An RNA guided nuclease described herein may be selected from the group consisting of a Cas1, a Cas1B, a Cas2, a Cas3, a Cas4, a Cas5, a Cas6, a Cas7, a Cas8, a Cas9, a Cas10, a Csy1, a Csy2, a Csy3, a Csel, a Cse2, a Csc1, a Csc2, a Csa5, a Csn2, a Csm2, a Csm3, a Csm4, a Csm5, a Csm6, a Cmrl, a Cmr3, a Cmr4, a Cmr5, a Cmr6, a Csbl, a Csb2, a Csb3, a Csx17, a Csx14, a Csx10, a Csx16, a CsaX, a Csx3, a Csx1, a Csx15, a Csfl, a Csf2, a Csf3, a Csf4, a Cpfl, a CasX, a CasY, a homolog thereof, or a modified version thereof. An RNA-guided nuclease may be a Cas9 nuclease or a homolog or modified version thereof. An RNA-guided nuclease may be a Cas9 protein, or a modified version thereof, from *Streptococcus pyogenes, Streptococcus thermophilius, Staphylococcus aureus, Neisseria meningitides,* or *Treponema denticola.* An RNA-guided nuclease is Cpfl or a homolog or modified version thereof.

A method and/or a composition described herein may comprise at least one, at least two, at least three, at least four, at least five, at least six, at least seven, at least eight, at least nine, or at least ten site-specific nucleases. A method and/or a composition described herein may comprise at least one, at least two, at least three, at least four, at least five, at least six, at least seven, at least eight, at least nine, or at least ten polynucleotides encoding at least one, at least two, at least three, at least four, at least five, at least six, at least seven, at least eight, at least nine, or at least ten site-specific nucleases.

In one aspect, a targeted editing technique described herein comprises the use of a recombinase. A tyrosine recombinase attached to a DNA recognition motif described herein may be selected from the group consisting of a Cre recombinase, a *Gin* recombinase a Flp recombinase, and a Tnp1 recombinase. A Cre recombinase or a *Gin* recombinase described herein may be tethered to a zinc-finger DNA binding domain. The Flp-FRT site-directed recombination system comes from the 2µ plasmid from the baker's yeast *Saccharomyces cerevisiae.* In this system, Flp recombinase (flippase) recombines sequences between flippase recognition target (*FRT*) sites. *FRT* sites comprise 34 nucleotides. Flp binds to the "arms" of the *FRT* sites (one arm is in reverse orientation) and cleaves the *FRT* site at either end of an intervening nucleic acid sequence. After cleavage, Flp recombines nucleic acid sequences between two *FRT* sites. Cre-lox is a site-directed recombination system derived from the bacteriophage P1 that is similar to the *Flp-FRT* recombination system. Cre-lox can be used to invert a nucleic acid sequence, delete a nucleic acid sequence, or translocate a nucleic acid sequence. In this system, Cre recombinase recombines a pair of lox nucleic acid sequences. Lox sites comprise 34 nucleotides, with the first and last 13 nucleotides (arms) being palindromic. During recombination, Cre recombinase protein binds to two lox sites on different nucleic acids and cleaves at the lox sites. The cleaved nucleic acids are spliced together (reciprocally translocated) and recombination is complete. In another aspect, a lox site provided herein is a loxP, lox 2272, loxN, lox 511, lox 5171, lox71, lox66, M2, M3, M7, or M11 site.

A serine recombinase attached to a DNA recognition motif described herein may be selected from the group consisting of a PhiC31 integrase, an R4 integrase, and a TP-901 integrase. A DNA transposase attached to a DNA binding domain described herein may be selected from the group consisting of a TALE-piggyBac and TALE-Mutator.

In one aspect, a targeted editing technique described herein comprises the use of a zinc-finger nuclease (ZFN). ZFNs are synthetic proteins consisting of an engineered zinc finger DNA-binding domain fused to the cleavage domain of the *FokI* restriction nuclease. ZFNs can be designed to cleave almost any long stretch of double-stranded DNA for modification of the zinc finger DNA-binding domain. ZFNs form dimers from monomers composed of a non-specific DNA cleavage domain of *FokI* nuclease fused to a zinc finger array engineered to bind a target DNA sequence.

The DNA-binding domain of a ZFN is typically composed of 3-4 zinc-finger arrays. The amino acids at positions -1, +2, +3, and +6 relative to the start of the zinc finger ∞-helix, which contribute to site-specific binding to the target DNA, can be changed and customized to fit specific target sequences. The other amino acids form the consensus backbone to generate ZFNs with different sequence specificities. Rules for selecting target sequences for ZFNs are known in the art.

The *FokI* nuclease domain requires dimerization to cleave DNA and therefore two ZFNs with their C-terminal regions are needed to bind opposite DNA strands of the cleavage site (separated by 5-7 nt). The ZFN monomer can cute the target site if the two-ZF-binding sites are palindromic. The term ZFN, as used herein, is broad and includes a monomeric ZFN that can cleave double stranded DNA without assistance from another ZFN. The term ZFN is also used to refer to one or both members of a pair of ZFNs that are engineered to work together to cleave DNA at the same site.

Without being limited by any scientific theory, because the DNA-binding specificities of zinc finger domains can in principle be re-engineered using one of various methods, customized ZFNs can theoretically be constructed to target nearly any gene sequence. Publicly available methods for engineering zinc finger domains include Context-dependent Assembly (CoDA), Oligomerized Pool Engineering (OPEN), and Modular Assembly.

In one aspect, a method and/or composition provided herein comprises one or more, two or more, three or more, four or more, or five or more ZFNs. A ZFN described herein is capable of generating a targeted DSB. Vectors comprising polynucleotides encoding one or more, two or more, three or more, four or more, or five or more ZFNs may be provided to a cell by transformation methods known in the art *(e.g.,* without being limiting, viral transfection, particle bombardment, PEG-mediated protoplast transfection *or Agrobacterium-mediated* transformation).

In one aspect, a targeted editing technique described herein comprises the use of a meganuclease. Meganucleases, which are commonly identified in microbes, are unique enzymes with high activity and long recognition sequences (> 14 nt) resulting in site-specific digestion of target DNA. Engineered versions of naturally occurring meganucleases typically have extended DNA recognition sequences (for example, 14 to 40 nt). The engineering of meganucleases can be more challenging than that of ZFNs and TALENs because the DNA recognition and cleavage functions of meganucleases are intertwined in a single domain. Specialized methods of mutagenesis and high-throughput screening have been used to create novel meganuclease variants that recognize unique sequences and possess improved nuclease activity.

In one aspect, a method and/or composition provided herein comprises one or more, two or more, three or more, four or more, or five or more meganucleases. A meganuclease described herein is capable of generating a targeted DSB. Vectors comprising polynucleotides encoding one or more, two or more, three or more, four or more, or five or more meganucleases may be provided to a cell by transformation methods known in the art (e.g., without being limiting, viral transfection, particle bombardment, PEG-mediated protoplast transfection *or Agrobacterium-mediated* transformation).

In one aspect, a targeted editing technique described herein comprises the use of a transcription activator-like effector nuclease (TALEN). TALENs are artificial restriction enzymes generated by fusing the transcription activator-like effector (TALE) DNA binding domain to a *FokI* nuclease domain. When each member of a TALEN pair binds to the DNA sites flanking a target site, the *FokI* monomers dimerize and cause a double-stranded DNA break at the target site. Besides the wild-type *FokI* cleavage domain, variants of the *FokI* cleavage domain with mutations have been designed to improve cleavage specificity and cleavage activity. The *FokI* domain functions as a dimer, requiring two constructs with unique DNA binding domains for sites in the target genome with proper orientation and spacing. Both the number of amino acid residues between the TALEN DNA binding domain and the *FokI* cleavage domain and the number of bases between the two individual TALEN binding sites are parameters for achieving high levels of activity.

TALENs are artificial restriction enzymes generated by fusing the transcription activator-like effector (TALE) DNA binding domain to a nuclease domain. In one aspect, the nuclease is selected from a group consisting of *PvuII, MutH, TevI* and *FokI, AlwI, MlyI, SbjI, SdaI, StsI, CleDORF, Clo051, Pept071.* When each member of a TALEN pair binds to the DNA sites flanking a target site, the *FokI* monomers dimerize and cause a double-stranded DNA break at the target site.

The term TALEN, as used herein, is broad and includes a monomeric TALEN that can cleave double stranded DNA without assistance from another TALEN. The term TALEN is also used to refer to one or both members of a pair of TALENs that work together to cleave DNA at the same site.

Transcription activator-like effectors (TALEs) can be engineered to bind practically any DNA sequence. TALE proteins are DNA-binding domains derived from various plant bacterial pathogens of the genus *Xanthomonas.* The X pathogens secrete TALEs into the host plant cell during infection. The TALE moves to the nucleus, where it recognizes and binds to a specific DNA sequence in the promoter region of a specific DNA sequence in the promoter region of a specific gene in the host genome. TALE has a central DNA-binding domain composed of 13-28 repeat monomers of 33-34 amino acids. The amino acids of each monomer are highly conserved, except for hypervariable amino acid residues at positions 12 and 13. The two variable amino acids are called repeat-variable diresidues (RVDs). The amino acid pairs NI, NG, HD, and NN of RVDs preferentially recognize adenine, thymine, cytosine, and guanine/adenine, respectively, and modulation of RVDs can recognize consecutive DNA bases. This simple relationship between amino acid sequence and DNA recognition has allowed for the engineering of specific DNA binding domains by selecting a combination of repeat segments containing the appropriate RVDs.

Besides the wild-type *FokI* cleavage domain, variants of the *FokI* cleavage domain with mutations have been designed to improve cleavage specificity and cleavage activity. The *FokI* domain functions as a dimer, requiring two constructs with unique DNA binding domains for sites in the target genome with proper orientation and spacing. Both the number of amino acid residues between the TALEN DNA binding domain and the *FokI* cleavage domain and the number of bases between the two individual TALEN binding sites are parameters for achieving high levels of activity. *PvuII, MutH,* and *TevI* cleavage domains are useful alternatives to *FokI* and *FokI* variants for use with TALEs. *PvuII* functions as a highly specific cleavage domain when coupled to a TALE *(see* Yank et al. 2013. PLoS One. 8: e82539). *MutH* is capable of introducing strand-specific nicks in DNA *(see* Gabsalilow et al. 2013. Nucleic Acids Research. 41: e83). *TevI* introduces double-stranded breaks in DNA at targeted sites(see Beurdeley et al., 2013. Nature Communications. 4: 1762).

The relationship between amino acid sequence and DNA recognition of the TALE binding domain allows for designable proteins. Software programs such as DNA Works can be used to design TALE constructs. Other methods of designing TALE constructs are known to those of skill in the art. *See* Doyle et al., Nucleic Acids Research (2012) 40: W117-122.; Cermak et al., Nucleic Acids Research (2011). 39:e82; and tale-nt.cac.comell.edu/about.

In one aspect, a method and/or composition provided herein comprises one or more, two or more, three or more, four or more, or five or more TALENs. A TALEN described herein is capable of generating a targeted DSB. In one aspect, vectors comprising polynucleotides encoding one or more, two or more, three or more, four or more, or five or more TALENs are provided to a cell by transformation methods known in the art *(e.g.,* without being limiting, viral transfection, particle bombardment, PEG-mediated protoplast transfection *or Agrobacterium-mediated* transformation).

In one aspect, a targeted editing technique described herein comprises the use of a RNA-guided nuclease. A CRISPR/Cas9 system or a CRISPR/Cpf1 system are alternatives to the *FokI*-based methods ZFN and TALEN. The CRISPR systems are based on RNA-guided engineered nucleases that use complementary base pairing to recognize DNA sequences at target sites.

In an aspect, a vector provided herein can comprise any combination of a nucleic acid sequence encoding a RNA-guided nuclease (non-limiting examples of RNA-guided nucleases include Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmrl, Cmr3, Cmr4, Cmr5, Cmr6, Csbl, Csb2, Csb3, Csx17, Csx14, Csx10, Csx16, CsaX, Csx3, Csx1, Csx15, Csfl, Csf2, Csf3, Csf4, Cpfl, CasX, CasY, homologs thereof, or modified versions thereof); and, optionally, a guide RNA necessary for targeting the respective nucleases. As used herein, the term "guide RNA" or gRNA generally refers to an RNA molecule (or a group of RNA molecules collectively) that can bind to an RNA-guided endonuclease and aid in targeting the nuclease to a specific location within a target polynucleotide (e.g., a DNA).

While not being limited by any particular scientific theory, CRISPR/Cas nucleases are part of the adaptive immune system of bacteria and archaea, protecting them against invading nucleic acids such as viruses by cleaving target DNA in a sequence-dependent manner. The immunity is acquired by the integration of short fragments of the invading DNA, known as spacers, between ~20 nucleotide long CRISPR repeats at the proximal end of a CRISPR locus (a CRISPR array). A well described Cas protein is the Cas9 nuclease (also known as Csn1), which is part of the Class 2, type II CRISPR/Cas system in *Streptococcus pyogenes. See* Makarova et al. Nature Reviews Microbiology (2015) doi: 10.1038/nrmicro3569. Cas9 comprises an RuvC-like nuclease domain at its amino terminus and an HNH-like nuclease domain positioned in the middle of the protein. Cas9 proteins also contain a PAM-interacting (PI) domain, a recognition lobe (REC), and a BH domain. The Cpfl nuclease, another type II system, acts in a similar manner to Cas9, but Cpfl does not require a tracrRNA. *See* Cong et al. Science (2013) 339: 819-823; Zetsche et al., Cell (2015) doi: 10.1016/j.cell.2015.09.038; U. S. Patent Publication No. 2014/0068797; U. S. Patent Publication No. 2014/0273235; U. S. Patent Publication No. 2015/0067922; U. S. Patent No. 8,697,359; U. S. Patent No. 8,771,945; U. S. Patent No. 8,795,965; U. S. Patent No. 8,865,406; U. S. Patent No. 8,871,445; U. S. Patent No. 8,889,356; U. S. Patent No. 8,889,418; U. S. Patent No. 8,895,308; and U. S. Patent No. 8,906,616.

When Cas9 or Cpfl cleaves targeted DNA, endogenous double stranded break (DSB) repair mechanisms are activated. DSBs can be repaired via non-homologous end joining, which can incorporate insertions or deletions (indels) into the targeted locus. If two DSBs flanking one target region are created, the breaks can be repaired by reversing the orientation of the targeted DNA. Alternatively, if a donor polynucleotide with homology to the target DNA sequence is provided, the DSB can be repaired via homology-directed repair. This repair mechanism allows for the precise integration of a donor polynucleotide into the targeted DNA sequence.

While not being limited by any particular scientific theory, in Class 2, type II CRISPR/Cas systems, CRISPR arrays, including spacers, are transcribed during encounters with recognized invasive DNA and are processed into small interfering CRISPR RNAs (crRNAs), which are approximately 40 nucleotides in length. The crRNAs hybridize with trans-activating crRNAs (tracrRNAs) to activate and guide the Cas9 nuclease to a target site. Nucleic acid molecules provided herein can combine a crRNA and a tracrRNA into one nucleic acid molecule in what is herein referred to as a "single-chain guide RNA (sgRNA)." A prerequisite for cleavage of the target site by Cas9 is the presence of a conserved protospacer-adj acent motif (PAM) downstream of the target DNA, which usually has the sequence 5-NGG-3 but less frequently NAG. Specificity is provided by the so-called "seed sequence" approximately 12 bases upstream of the PAM, which must match between the RNA and target DNA. Cpfl acts in a similar manner to Cas9, but Cpfl does not require a tracrRNA. Therefore, in an aspect utilizing Cpfl a sgRNA can be replaced by a crRNA. The PAM motif of Cpf1 is upstream of the target site. Additionally, for Cpfl orthologs LbCpf1 and AsCpf1, the PAM sequence is 5-TTTV-3 where V can be A, C, or G. In an aspect, when two or more sgRNAs are provided herein, the first sgRNA and the second sgRNA are complementary to different strands of a double-stranded DNA molecule. In another aspect, when two or more sgRNAs are provided herein, the first sgRNA and the second sgRNA are complementary to the same strand of a double-stranded DNA molecule. As used herein, a "protospacer adjacent motif"(PAM) refers to a 2-6 base pair DNA sequence immediately upstream or downstream of a target sequence of a CRISPR complex.In another aspect, a first and a second gRNA target different PAM sequences. In another aspect, a first and a second gRNA targetthe same PAM sequences.

In one aspect, a method and/or composition provided herein comprises one or more, two or more, three or more, four or more, or five or more Cas9 nucleases. In one aspect, a method and/or composition provided herein comprises one or more polynucleotides encoding one or more, two or more, three or more, four or more, or five or more Cas9 nucleases. In another aspect, a Cas9 nuclease provided herein is capable of generating a targeted DSB. In one aspect, a method and/or composition provided herein comprises one or more, two or more, three or more, four or more, or five or more Cpfl nucleases. In one aspect, a method and/or composition provided herein comprises one or more polynucleotides encoding one or more, two or more, three or more, four or more, or five or more Cpfl nucleases. In another aspect, a Cpfl nuclease provided herein is capable of generating a targeted DSB.

When a Cas9 nuclease hybridizes to a target site via an sgRNA, Cas9 produces two blunt-end cuts in the double-stranded DNA. The "target strand" of the double-stranded DNA is complementary to the sgRNA, while the "non-target strand" comprises the PAM motif adjacent to, and on the 3' end of, the cut site on the non-target strand. Cas9 holds the target stand and the PAM motif, but the 3' cut end of the non-target strand is free and is referred to as the "3' flap." In one aspect, the 3' flap comprises at least 10, at least 15, at least 20, at least 25, at least 26, at least 27, at least 28, at least 29, at least 30, at least 31, at least 32, at least 33, at least 34, at least 35, at least 36, at least 37, at least 38, at least 39, or at least 40 nucleotides.

In one aspect, vectors comprising polynucleotides encoding a site-specific nuclease, and optionally one or more, two or more, three or more, or four or more sgRNAs are provided to a plant cell by transformation methods known in the art (e.g., without being limiting, particle bombardment, PEG-mediated protoplast transfection or *Agrobacterium-*mediated transformation). In one aspect, vectors comprising polynucleotides encoding a Cas9 nuclease, and optionally one or more, two or more, three or more, or four or more sgRNAs are provided to a plant cell by transformation methods known in the art (e.g., without being limiting, particle bombardment, PEG-mediated protoplast transfection or *Agrobacterium-*mediated transformation).In another aspect, vectors comprising polynucleotides encoding a Cpfl and, optionally one or more, two or more, three or more, or four or more crRNAs are provided to a cell by transformation methods known in the art (e.g., without being limiting, viral transfection, particle bombardment, PEG-mediated protoplast transfection or *Agrobacterium-mediated* transformation).

In one aspect, an RNA-guided nuclease described herein is selected from the group consisting of Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmrl, Cmr3, Cmr4, Cmr5, Cmr6, Csbl, Csb2, Csb3, Csx17, Csx14, Csx10, Csx16, CsaX, Csx3, Csx1, Csx15, Csfl, Csf2, Csf3, Csf4, Cpfl, CasX, CasY, homologs thereof, or modified versions thereof, an Argonaute (non-limiting examples of Argonaute proteins include *Thermus thermophilus* Argonaute (TtAgo), *Pyrococcus furiosus* Argonaute (PfAgo), *Natronobacterium gregoryi* Argonaute (NgAgo), homologs thereof, modified versions thereof), a DNA guide for an Argonaute protein, and any combination thereof. In another aspect, an RNA-guided nuclease provided herein is selected from the group consisting of Cas9 and Cpf1. an RNA-guided nuclease provided herein comprises Cas9. In one aspect, an RNA-guided nuclease described herein is selected from the group consisting of Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, homologs thereof, or modified versions thereof. In one aspect a site-specific nuclease is selected from the group consisting of Cas1, Cas1B, Cas2, Cas3, Cas4, Cas5, Cas6, Cas7, Cas8, Cas9, Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, TtAgo, PfAgo, and NgAgo. In another aspect, an RNA-guided nuclease is selected from the group consisting of Cas1, Cas1B, Cas2, Cas3, Cas4, Cas5, Cas6, Cas7, Cas8, Cas9, Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpfl, CasX, CasY, TtAgo, PfAgo, and NgAgo.

Nucleases, such as Cas9, can also be engineered to form a catalytically deactivated from, such catalytically deactivated Cas9 (dCas9). dCas9 binds to DNA at a target site specified by a gRNA and creates a loop structure accessible for template-based editing (Figure 11, Panel 1). dCas9 can be further modified to form a fusion with a ssDNA binding domain for further facilitating template-based editing (Figure 11, Panel 2). The editing efficiency with this modified dCas9-ssDNA binding scheme is expected to be higher compared to a dCas9-alone approach, because a ssDNA template is bound to dCas9 complex and would be brought into proximity of the gRNA target. As used herein, a "deactivated Cas nuclease" (dCas) refers to an enzymatically inactive form of a Cas nuclease protein which can bind DNA, but cannot cleave DNA. In one aspect, a nuclease described herein is a dCas. In another aspect, a site-specific nuclease described herein is a dCas.

In one aspect, methods and compositions described herein can be used to edit a locus in a eukaryotic cell. In one aspect, a A eukaryotic cell described herein is part of a multicellular eukaryotic organism. In another aspect, a eukaryotic cell described herein is a unicellular organism. In another aspect, a eukaryotic cell described herein is selected from the group consisting of an animal cell, a plant cell, a fungus cell, and a protozoan cell. In one aspect, an animal cell described herein is selected from the group consisting of an insect cell, an arachnid cell, an arthropod cell, a crustacean cell, a rotifer cell, a cnidarian cell, a Platyhelminthes cell, a mollusk cell, a gastropod cell, a nematode cell, an annelid cell, a vertebrate cell, a mammal cell, an avian cell, a fish cell, a reptile cell, and an amphibian cell. In another aspect a plant cell described herein is a monocot cell or a dicot cell. In still another aspect a plant cell described herein is an algae cell. In yet another aspect, a plant cell described herein is selected from the group consisting of a corn cell, a wheat cell, a sorghum cell, a canola cell, a soybean cell, an alfalfa cell, a cotton cell, and a rice cell. In still another aspect, a plant cell described herein is selected from the group consisting of an Acacia cell, an alfalfa cell, an aneth cell, an apple cell, an apricot cell, an artichoke cell, an arugula cell, an asparagus cell, an avocado cell, a banana cell, a barley cell, a bean cell, a beet cell, a blackberry cell, a blueberry cell, a broccoli cell, a Brussels sprout cell, a cabbage cell, a canola cell, a cantaloupe cell, a carrot cell, a cassava cell, a cauliflower cell, a celery cell, a Chinese cabbage cell, a cherry cell, a cilantro cell, a citrus cell, a clementine cell, a coffee cell, a corn cell, a cotton cell, a cucumber cell, a Douglas fir cell, an eggplant cell, an endive cell, an escarole cell, an eucalyptus cell, a fennel cell, a fig cell, a forest tree cell, a gourd cell, a grape cell, a grapefruit cell, a honey dew cell, a jicama cell, kiwifruit cell, a lettuce cell, a leek cell, a lemon cell, a lime cell, a Loblolly pine cell, a mango cell, a maple tree cell, a melon cell, a mushroom cell, a nectarine cell, a nut cell, an oat cell, an okra cell, an onion cell, an orange cell, an ornamental plant cell, a papaya cell, a parsley cell, a pea cell, a peach cell, a peanut cell, a pear cell, a pepper cell, a persimmon cell, a pine cell, a pineapple cell, a plantain cell, a plum cell, a pomegranate cell, a poplar cell, a potato cell, a pumpkin cell, a quince cell, a radiata pine cell, a radicchio cell, a radish cell, a rapeseed cell, a raspberry cell, a rice cell, a rye cell, a sorghum cell, a Southern pine cell, a soybean cell, a spinach cell, a squash cell, a strawberry cell, a sugar beet cell, a sugarcane cell, a sunflower cell, a sweet corn cell, a sweet potato cell, a sweetgum cell, a tangerine cell, a tea cell, a tobacco cell, a tomato cell, a turf cell, a vine cell, watermelon cell, a wheat cell, a yam cell, and a zucchini cell. In another aspect, a plant cell described herein is selected from the group consisting of a corn cell, a soybean cell, a canola cell, a cotton cell, a wheat cell, and a sugarcane cell.

In still another aspect an engineered plant described herein is an algae. In yet another aspect, an engineered plant or seed described herein is selected from the group consisting of a corn plant, a wheat plant, a sorghum plant, a canola plant, a soybean plant, an alfalfa plant, a cotton plant, and a rice plant. In still another aspect, an engineered plant or seed described herein is selected from the group consisting of an Acacia plant, an alfalfa plant, an aneth plant, an apple plant, an apricot plant, an artichoke plant, an arugula plant, an asparagus plant, an avocado plant, a banana plant, a barley plant, a bean plant, a beet plant, a blackberry plant, a blueberry plant, a broccoli plant, a Brussels sprout plant, a cabbage plant, a canola plant, a cantaloupe plant, a carrot plant, a cassava plant, a cauliflower plant, a celery plant, a Chinese cabbage plant, a cherry plant, a cilantro plant, a citrus plant, a clementine plant, a coffee plant, a corn plant, a cotton plant, a cucumber plant, a Douglas fir plant, an eggplant plant, an endive plant, an escarole plant, an eucalyptus plant, a fennel plant, a fig plant, a forest tree plant, a gourd plant, a grape plant, a grapefruit plant, a honey dew plant, a jicama plant, kiwifruit plant, a lettuce plant, a leek plant, a lemon plant, a lime plant, a Loblolly pine plant, a mango plant, a maple tree plant, a melon plant, a mushroom plant, a nectarine plant, a nut plant, an oat plant, an okra plant, an onion plant, an orange plant, an ornamental plant, a papaya plant, a parsley plant, a pea plant, a peach plant, a peanut plant, a pear plant, a pepper plant, a persimmon plant, a pine plant, a pineapple plant, a plantain plant, a plum plant, a pomegranate plant, a poplar plant, a potato plant, a pumpkin plant, a quince plant, a radiata pine plant, a radicchio plant, a radish plant, a rapeseed plant, a raspberry plant, a rice plant, a rye plant, a sorghum plant, a Southern pine plant, a soybean plant, a spinach plant a squash plant, a strawberry plant, a sugar beet plant, a sugarcane plant, a sunflower plant, a sweet corn plant, a sweet potato plant, a sweetgum plant, a tangerine plant, a tea plant, a tobacco plant, a tomato plant, a turf plant, a vine plant, watermelon plant, a wheat plant, a yam plant, and a zucchini plant. In another aspect, a plant described herein is selected from the group consisting of a corn plant, a soybean plant, a canola plant, a cotton plant, a wheat plant, and a sugarcane plant.

In still another aspect a modified plant described herein is an algae. In yet another aspect, a modified plant described herein is selected from the group consisting of a corn plant, a wheat plant, a sorghum plant, a canola plant, a soybean plant, an alfalfa plant, a cotton plant, and a rice plant. In still another aspect, a modified plant described herein is selected from the group consisting of an Acacia plant, an alfalfa plant, an aneth plant, an apple plant, an apricot plant, an artichoke plant, an arugula plant, an asparagus plant, an avocado plant, a banana plant, a barley plant, a bean plant, a beet plant, a blackberry plant, a blueberry plant, a broccoli plant, a Brussels sprout plant, a cabbage plant, a canola plant, a cantaloupe plant, a carrot plant, a cassava plant, a cauliflower plant, a celery plant, a Chinese cabbage plant, a cherry plant, a cilantro plant, a citrus plant, a clementine plant, a coffee plant, a corn plant, a cotton plant, a cucumber plant, a Douglas fir plant, an eggplant plant, an endive plant, an escarole plant, an eucalyptus plant, a fennel plant, a fig plant, a forest tree plant, a gourd plant, a grape plant, a grapefruit plant, a honey dew plant, a jicama plant, kiwifruit plant, a lettuce plant, a leek plant, a lemon plant, a lime plant, a Loblolly pine plant, a mango plant, a maple tree plant, a melon plant, a mushroom plant, a nectarine plant, a nut plant, an oat plant, an okra plant, an onion plant, an orange plant, an ornamental plant, a papaya plant, a parsley plant, a pea plant, a peach plant, a peanut plant, a pear plant, a pepper plant, a persimmon plant, a pine plant, a pineapple plant, a plantain plant, a plum plant, a pomegranate plant, a poplar plant, a potato plant, a pumpkin plant, a quince plant, a radiata pine plant, a radicchio plant, a radish plant, a rapeseed plant, a raspberry plant, a rice plant, a rye plant, a sorghum plant, a Southern pine plant, a soybean plant, a spinach plant, a squash plant, a strawberry plant, a sugar beet plant, a sugarcane plant, a sunflower plant, a sweet corn plant, a sweet potato plant, a sweetgum plant, a tangerine plant, a tea plant, a tobacco plant, a tomato plant, a turf plant, a vine plant, watermelon plant, a wheat plant, a yam plant, and a zucchini plant.

In yet another aspect, a modified seed described herein is selected from the group consisting of a corn seed, a wheat seed, a sorghum seed, a canola seed, a soybean seed, an alfalfa seed, a cotton seed, and a rice seed. In still another aspect, a modified seed described herein is selected from the group consisting of an Acacia seed, an alfalfa seed, an aneth seed, an apple seed, an apricot seed, an artichoke seed, an arugula seed, an asparagus seed, an avocado seed, a banana seed, a barley seed, a bean seed, a beet seed, a blackberry seed, a blueberry seed, a broccoli seed, a Brussels sprout seed, a cabbage seed, a canola seed, a cantaloupe seed, a carrot seed, a cassava seed, a cauliflower seed, a celery seed, a Chinese cabbage seed, a cherry seed, a cilantro seed, a citrus seed, a clementine seed, a coffee seed, a corn seed, a cotton seed, a cucumber seed, a Douglas fir seed, an eggplant seed, an endive seed, an escarole seed, an eucalyptus seed, a fennel seed, a fig seed, a forest tree seed, a gourd seed, a grape seed, a grapefruit seed, a honey dew seed, a jicama seed, kiwifruit seed, a lettuce seed, a leek seed, a lemon seed, a lime seed, a Loblolly pine seed, a mango seed, a maple tree seed, a melon seed, a mushroom seed, a nectarine seed, a nut seed, an oat seed, an okra seed, an onion seed, an orange seed, an ornamental plant seed, a papaya seed, a parsley seed, a pea seed, a peach seed, a peanut seed, a pear seed, a pepper seed, a persimmon seed, a pine seed, a pineapple seed, a plantain seed, a plum seed, a pomegranate seed, a poplar seed, a potato seed, a pumpkin seed, a quince seed, a radiata pine seed, a radicchio seed, a radish seed, a rapeseed seed, a raspberry seed, a rice seed, a rye seed, a sorghum seed, a Southern pine seed, a soybean seed, a spinach seed, a squash seed, a strawberry seed, a sugar beet seed, a sugarcane seed, a sunflower seed, a sweet corn seed, a sweet potato seed, a sweetgum seed, a tangerine seed, a tea seed, a tobacco seed, a tomato seed, a turf seed, a vine seed, watermelon seed, a wheat seed, a yam seed, and a zucchini seed.

.

According to one aspect, a modified plant, plant cell, cell, seed, or chromosome described herein may comprise at least one, at least two, at least three, at least four, at least five, at least six, at least seven, at least eight, at least nine, or at least ten deletions generated by a targeted editing technique. According to one aspect, a modified plant, plant cell, cell, seed, or chromosome described herein comprises at least one, at least two, at least three, at least four, at least five, at least six, at least seven, at least eight, at least nine, or at least ten insertions generated by a targeted editing technique. According to one aspect, a modified plant, plant cell, cell, seed, or chromosome described herein comprises at least one, at least two, at least three, at least four, at least five, at least six, at least seven, at least eight, at least nine, or at least ten inversions generated by a targeted editing technique. According to one aspect, a modified plant, plant cell, cell, seed, or chromosome described herein comprises at least one, at least two, at least three, at least four, at least five, at least six, at least seven, at least eight, at least nine, or at least ten deletions generated by a targeted editing technique, at least one, at least two, at least three, at least four, at least five, at least six, at least seven, at least eight, at least nine, or at least ten insertions generated by a targeted editing technique, at least one, at least two, at least three, at least four, at least five, at least six, at least seven, at least eight, at least nine, or at least ten inversions generated by a targeted editing technique, or any combination thereof. In still another aspect, a modified plant, plant cell, cell, seed, or chromosome described herein comprises at least one, at least two, at least three, at least four, at least five, at least six, at least seven, at least eight, at least nine, or at least ten dominant negative alleles generated by a targeted editing technique. In still another aspect, a modified plant, plant cell, cell, seed, or chromosome described herein comprises at least one, at least two, at least three, at least four, at least five, at least six, at least seven, at least eight, at least nine, or at least ten dominant positive alleles generated by a targeted editing technique. In still another aspect, a modified plant, plant cell, cell, seed, or chromosome described herein comprises at least one, at least two, at least three, at least four, at least five, at least six, at least seven, at least eight, at least nine, or at least ten dominant negative alleles generated by a targeted editing technique, at least one, at least two, at least three, at least four, at least five, at least six, at least seven, at least eight, at least nine, or at least ten dominant positive alleles generated by a targeted editing technique, or any combination thereof.

According to another aspect of the present application, a modified plant(s), plant cell(s), seed(s), chromosome(s) and plant part(s) are described comprising a genome editing event in the genome of at least one plant cell thereof, the genome editing event comprising an insertion, a deletion, a substitution, or an inversion of a targeted locus.

The methods and compositions described herein are capable of editing any locus in a genome. Also described herein are chromosomes edited by using the methods and compositions describd herein. In an aspect, a genome described herein is a nuclear genome, a mitochondrial genome, or a plastid genome. In another aspect, a plastid genome provided herein comprises a chloroplast genome. In one aspect, a method described herein generates a double-stranded break on a chromosome. In an aspect, a chromosome described herein is a nuclear chromosome, a mitochondrial chromosome, or a chloroplast chromosome. In another aspect a chromosome described herein is a supernumerary chromosome or an artificial chromosome. Supernumerary, or B chromosomes, are extra chromosomes found in addition to the normal diploid complement of chromosomes in a cell. Supernumerary chromosomes are dispensable and not required for normal development of a cell or organism. In an aspect, a supernumerary chromosome described herein is a maize supernumerary chromosome or a rye supernumerary chromosome.

A method of targeted editing disclosed herein can involve transient transfection or stable transformation of a cell of interest (e.g., a plant cell).

Numerous methods for transforming chromosomes or plastids in a plant cell with a recombinant DNA molecule or construct are known in the art, which can be used according to methods of the present application to produce a transgenic plant cell and plant. Any suitable method or technique for transformation of a plant cell known in the art can be used according to present methods. Effective methods for transformation of plants include bacterially mediated transformation, such as *Agrobacterium-mediated* or *Rhizobium-mediated* transformation and microprojectile bombardment-mediated transformation. A variety of methods are known in the art for transforming explants with a transformation vector via bacterially mediated transformation or microprojectile bombardment and then subsequently culturing, etc., those explants to regenerate or develop transgenic plants. Other methods for plant transformation, such as microinjection, electroporation, vacuum infiltration, pressure, sonication, silicon carbide fiber agitation, PEG-mediated transformation, etc., are also known in the art. Transgenic plants produced by these transformation methods can be chimeric or non-chimeric for the transformation event depending on the methods and explants used.

Methods of transforming plant cells are well known by persons of ordinary skill in the art. For instance, specific instructions for transforming plant cells by microprojectile bombardment with particles coated with recombinant DNA are found in U.S. Patent Nos. 5,550,318; 5,538,880 6,160,208; 6,399,861; and 6,153,812 and *Agrobacterium-mediated* transformation is described in U.S. Patent Nos. 5,159,135; 5,824,877; 5,591,616; 6,384,301; 5,750,871; 5,463,174; and 5,188,958. Additional methods for transforming plants can be found in, for example, Compendium of Transgenic Crop Plants (2009) Blackwell Publishing. Any appropriate method known to those skilled in the art can be used to transform a plant cell with any of the nucleic acid molecules provided herein.

Recipient cell or explant targets for transformation include, but are not limited to, a seed cell, a fruit cell, a leaf cell, a cotyledon cell, a hypocotyl cell, a meristem cell, an embryo cell, an endosperm cell, a root cell, a shoot cell, a stem cell, a pod cell, a flower cell, an inflorescence cell, a stalk cell, a pedicel cell, a style cell, a stigma cell, a receptacle cell, a petal cell, a sepal cell, a pollen cell, an anther cell, a filament cell, an ovary cell, an ovule cell, a pericarp cell, a phloem cell, a bud cell, or a vascular tissue cell. In another aspect, this disclosure provides a plant chloroplast. In a further aspect, this disclosure provides an epidermal cell, a stomata cell, a trichome cell, a root hair cell, a storage root cell, or a tuber cell. In another aspect, this disclosure provides a protoplast. In another aspect, this disclosure provides a plant callus cell. Any cell from which a fertile plant can be regenerated is contemplated as a useful recipient cell for practice of this disclosure. Callus can be initiated from various tissue sources, including, but not limited to, immature embryos or parts of embryos, seedling apical meristems, microspores, and the like. Those cells which are capable of proliferating as callus can serve as recipient cells for transformation. Practical transformation methods and materials for making transgenic plants of this disclosure (e.g., various media and recipient target cells, transformation of immature embryos, and subsequent regeneration of fertile transgenic plants) are disclosed, for example, in U. S. Patents 6,194,636 and 6,232,526 and U. S. Patent Application Publication 2004/0216189. Transformed explants, cells or tissues can be subjected to additional culturing steps, such as callus induction, selection, regeneration, etc., as known in the art. Transformed cells, tissues or explants containing a recombinant DNA insertion can be grown, developed or regenerated into transgenic plants in culture, plugs or soil according to methods known in the art. In one aspect, this disclosure provides plant cells that are not reproductive material and do not mediate the natural reproduction of the plant. In another aspect, this disclosure also provides plant cells that are reproductive material and mediate the natural reproduction of the plant. In another aspect, this disclosure provides plant cells that cannot maintain themselves via photosynthesis. In another aspect, this disclosure provides somatic plant cells. Somatic cells, contrary to germline cells, do not mediate plant reproduction. In one aspect, this disclosure provides a non-reproductive plant cell.

Modified plants can be further crossed to themselves or other plants to produce modified seeds and progeny. A modified plant can also be prepared by crossing a first plant comprising a recombinant DNA sequence insertion with a second plant lacking the insertion. For example, a recombinant DNA sequence can be introduced into a first plant line that is amenable to transformation, which can then be crossed with a second plant line to introgress the recombinant DNA sequence into the second plant line. A modified plant can also be prepared by crossing a modified plant with an unmodified plant. Progeny of these crosses can be further back crossed into the more desirable line multiple times, such as through 6 to 8 generations or back crosses, to produce a progeny plant with substantially the same genotype as the original parental line but for the introduction of the recombinant DNA construct or modified sequence.

A modified plant, cell, or explant described herein can be of an elite variety or an elite line. An elite variety or an elite line refers to any variety that has resulted from breeding and selection for superior agronomic performance. A modified plant, cell, or explant described herein can be a hybrid plant, cell, or explant. As used herein, a "hybrid" is created by crossing two plants from different varieties, lines, or species, such that the progeny comprises genetic material from each parent. Skilled artisans recognize that higher order hybrids can be generated as well. For example, a first hybrid can be made by crossing Variety C with Variety D to create a C x D hybrid, and a second hybrid can be made by crossing Variety E with Variety F to create an E x F hybrid. The first and second hybrids can be further crossed to create the higher order hybrid (C x D) x (E x F) comprising genetic information from all four parent varieties. A modified plant described herein is fertile. A modified plant described herein is a male or female sterile modified plant, which cannot reproduce without human intervention. In one aspect, a modified plant described herein reproduces via asexual or vegetative reproduction. In still another aspect, a modified plant provided herein reproduces via sexual reproduction.

A recombinant DNA molecule or construct as herein described can comprise or be included within a DNA transformation vector for use in transformation of a target plant cell, tissue or explant. Such a transformation vector of the present application can generally comprise sequences or elements necessary or beneficial for effective transformation in addition to at least one selectable marker gene, at least one expression cassette and/or transcribable DNA sequence encoding one or more site-specific nucleases, and, optionally, one or more sgRNAs or crRNAs. For *Agrobacterium-mediated* transformation, the transformation vector can comprise an engineered transfer DNA (or T-DNA) segment or region having two border sequences, a left border (LB) and a right border (RB), flanking at least a transcribable DNA sequence or transgene, such that insertion of the T-DNA into the plant genome will create a transformation event for the transcribable DNA sequence, transgene or expression cassette. In other words, the transgene, a transcribable DNA sequence, transgene or expression cassette encoding the site-specific nuclease(s), and/or sgRNA(s) or crRNA(s) would be located between the left and right borders of the T-DNA, perhaps along with an additional transgene(s) or expression cassette(s), such as a plant selectable marker transgene and/or other gene(s) of agronomic interest that can confer a trait or phenotype of agronomic interest to a plant. The transcribable DNA sequence, transgene or expression cassette encoding at least one site-specific nuclease, any necessary sgRNAs or crRNAs, and the plant selectable marker transgene (or other gene of agronomic interest) can be present in separate T-DNA segments on the same or different recombinant DNA molecule(s), such as for co-transformation. A transformation vector or construct can further comprise prokaryotic maintenance elements, which for *Agrobacterium-mediated* transformation can be located in the vector backbone outside of the T-DNA region(s).

A plant selectable marker transgene in a transformation vector or construct of the present application can be used to assist in the selection of transformed cells or tissue due to the presence of a selection agent, such as an antibiotic or herbicide, where the plant selectable marker transgene provides tolerance or resistance to the selection agent. Thus, the selection agent can bias or favor the survival, development, growth, proliferation, etc., of transformed cells expressing the plant selectable marker gene, such as to increase the proportion of transformed cells or tissues in the R₀ plant. Commonly used plant selectable marker genes include, for example, those conferring tolerance or resistance to antibiotics, such as kanamycin and paromomycin (*nptII*)*,* hygromycin B *(aph IV),* streptomycin or spectinomycin *(aadA)* and gentamycin *(aac3* and *aacC4*)*,* or those conferring tolerance or resistance to herbicides such as glufosinate *(bar* or *pat),* dicamba (*DMO*) and glyphosate (*aroA* or *Cp4-EPSPS*). Plant screenable marker genes can also be used, which provide an ability to visually screen for transformants, such as luciferase or green fluorescent protein (GFP), or a gene expressing a beta glucuronidase or *uidA* gene (GUS) for which various chromogenic substrates are known. In one aspect, a vector or polynucleotide provided herein comprises at least one marker gene selected from the group consisting of *nptII, aph IV, aadA, aac3, aacC4, bar, pat, DMO, EPSPS, aroA,* GFP, and GUS.

.

As used herein, a "portion" of a nucleic acid sequence or molecule refers to any number of nucleotides less than the full length of the nucleic sequence. For example, a portion of a 100 nucleotide nucleic acid sequence can be any number of nucleotides from 1 to 99 nucleotides. Alternatively, a "portion" of a nucleic sequence refers to anywhere from 0.01% to 99.99% of the full length of a given nucleic acid sequence.

Provided herein are methods of generating dominant alleles of gene regions using targeted editing techniques. In one aspect, a dominant negative allele described herein is capable of suppressing transcription of a locus or a gene in a heterozygous state. In another aspect, a dominant negative allele described herein is capable of suppressing transcription of a locus or a gene in a homozygous state.

Dominant negative alleles of a gene region can reduce or eliminate the function of a gene region product in a heterozygous state.

A dominant negative allele can also be generated by editing a genome to delete a region of DNA between a first gene region and a second, neighboring gene region, where the first gene region and the second gene region are present on the chromosome in opposite orientations (e.g., the first gene region is present in 5' to 3' orientation, while the second gene region is present on the same DNA strand of the chromosome in 3' to 5' orientation). Without being bound by any scientific theory, such a deletion allows the expression of an antisense RNA transcript of the first gene region by the promoter of the second gene region, while the native promoter of the first gene region expresses a sense RNA transcript. The sense and antisense RNA transcripts are complementary to each other and can be processed by RNA silencing mechanisms native to the cell to reduce the expression of the edited and unedited first gene region alleles in a dominant negative manner. Without being bound by any scientific theory, it is also contemplated that the antisense RNA molecule transcribed from the mutant or edited allele of the endogenous gene or locus may affect the expression level(s) of the gene through different mechanisms, such as nonsense mediated decay, nonstop decay, no-go decay, DNA or histone methylation or other epigenetic changes, inhibition or decreased efficiency of transcription and/or translation, ribosomal interference, interference with mRNA processing or splicing, and/or ubiquitin-mediated protein degradation via the proteasome. See, e.g., Nickless, A. et al., "Control of gene expression through the nonsense-mediated RNA decay pathway", Cell Biosci 7:26 (2017); Karamyshev, A. et al., "Lost in Translation: Ribosome-Associated mRNA and Protein Quality Controls", Frontiers in Genetics 9:431 (2018); Inada, T., "Quality controls induced by aberrant translation", Nucleic Acids Res 48:3 (2020); and Szadeczky-Kardoss, I. et al., "The nonstop decay and the RNA silencing systems operate cooperatively in plants", Nucleic Acids Res 46:9 (2018). Each of these different mechanisms may act alternatively or in addition to RNA interference (RNAi), transcriptional gene silencing (PGS) and/or post transcriptional gene silencing (PTGS) mechanisms. See, e.g., Wilson, R.C. et al., "Molecular Mechanisms of RNA Interference", Annu Rev Biophysics 42:217-39 (2013); and Guo, Q. et al., "RNA Silencing in Plants: Mechanism, Technologies and Applications in Horticulture Crops", Current Genomics 17:476-489 (2016). Some of the above mechanisms may reduce expression of the edited allele itself, while others may also reduce the expression of other copy/-ies or allele(s) of the endogenous locus/loci or gene(s). Such dominant or semi-dominant effect(s) on the gene(s) may operate through non-canonical suppression mechanisms that do not involve RNAi and/or formation of targeted small RNAs at a significant or detectable level.

In one aspect, this disclosure provides a method of generating a dominant negative allele of a gene in a cell comprising deleting a portion of a chromosome between a first gene region and a second gene region using a targeted editing technique, where an antisense RNA transcript of the first gene region is generated following the deletion of the portion of the chromosome. In another aspect, a targeted editing technique provided here comprises the use of at least one site-specific nuclease. In an aspect, an antisense RNA transcript provided herein is a partial antisense RNA transcript. In an aspect, a partial antisense RNA transcript is shorter than the corresponding sense RNA transcript. In an aspect, a partial antisense RNA transcript is at least 1, at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 150, at least 200, at least 250, at least 300, at least 400, at least 500, at least 600, at least 700, at least 800, at least 900, at least 1000, at least 1500, at least 2000, or at least 2500 nucleotides shorter than a corresponding sense RNA transcript. In another aspect, a partial antisense RNA transcript is at least 1%, at least 2%, at least 3%, at least 4%, at least 5%, at least 10%, at least 20%, at least 30%, at least 40%, at least 50%, at least 60%, at least 70%, at least 80%, at least 90%, or at least 95% shorter than a corresponding sense RNA transcript. In another aspect, an antisense RNA transcript provided herein is a complete antisense RNA transcript. In an aspect, a complete antisense RNA transcript is the same length as a corresponding sense RNA transcript. In an aspect, an antisense RNA transcript provided herein suppresses expression of a first gene region. In an aspect, an antisense RNA transcript provided herein is capable of suppressing expression of a first gene region.

In another aspect, this disclosure provides a method comprising: a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; b) inducing a first double-stranded break and a second double-stranded break flanking a targeted region; c) identifying one or more cells comprising a deletion of the targeted region of the chromosome; and d) selecting one or more cells comprising the deletion of the targeted region of the chromosome.

As used herein, an "intervening region" or "intervening sequence" refers to a polynucleotide sequence between a physically linked first polynucleotide sequence and second polynucleotide sequence. In one aspect, an intervening region or intervening sequence is between a first gene and a second gene. In an aspect, an intervening region or intervening sequence is between a first gene region and a second gene region. In one aspect, an intervening region or intervening sequence is between a first coding region and a second coding region. In another aspect, an intervening region or intervening sequence is between a first target site and a second target site. In one aspect, an intervening region or intervening sequence is between a first target gene and a second target gene. In one aspect, all or part of an intervening region or intervening sequence is inverted via a targeted editing technique. In another aspect, all or part of an intervening region or intervening sequence is deleted via a targeted editing technique. In one aspect, an intervening region or intervening sequence comprises at least 1, at least 2, at least 3, at least 4, at least 5, at least 10, at least 25, at least 50, at least 100, at least 150, at least 200, at least 250, at least 500, at least 1000, at least 1250, at least 1500, at least 1750, at least 2000, at least 2500, at least 3000, at least 4000, at least 5000, at least 6000, at least 7000, at least 8000, at least 9000, at least 10,000, at least 15,000, at least 20,000, at least 25,000, or at least 50,000 nucleotides. In an aspect, an intervening region or an intervening sequence comprises at least one, at least two, at least three, at least four, at least five, at least six, at least seven, at least eight, at least nine, or at least ten genes. In one aspect, an intervening region or intervening sequence is positioned on a chromosome. In one aspect, an intervening region or intervening sequence is positioned on a vector. In one aspect, an intervening region or intervening sequence comprises a DNA sequence. In one aspect, an intervening region or intervening sequence comprises an RNA sequence. In one aspect, an intervening region or intervening sequences comprises an endogenous nucleic acid sequence. In another aspect, an intervening region or intervening sequences comprises a transgenic nucleic acid sequence. In one aspect, an intervening region or intervening sequences comprises an endogenous nucleic acid sequence and a transgenic nucleic acid sequence.

In one aspect, a first gene region is selected from the group of a GA20 oxidase gene region, a GA3 oxidase gene region, a brachytic2 gene region, and a Y1 gene region. In another aspect, a first gene region is a GA20 oxidase gene region or a GA3 oxidase gene region. In a further aspect, a first gene region is a GA20 oxidase gene region. In an aspect, a first gene region is a GA3 oxidase gene region. In still another aspect, a first gene region is a brachytic2 gene region. In another aspect, a first gene region is a Y1 gene region.

GA oxidases in cereal plants consist of a family of related GA oxidase genes. For example, corn has a family of at least nine GA20 oxidase genes that includes GA20 oxidase_1, GA20 oxidase_2, GA20 oxidase_3, GA20 oxidase_4, GA20 oxidase_5, GA20 oxidase_6, GA20 oxidase_7, GA20 oxidase_8, and GA20 oxidase_9. The DNA and protein sequences by SEQ ID NOs for each of GA20 oxidase_3 and GA20 oxidase_5 are provided in Table 1.

**Table 1. DNA and protein sequences by sequence identifier for GA20 oxidase_3 and GA20 oxidase_5 genes in corn.**

| **GA20 oxidase Gene** | **Genomic DNA** | **cDNA** | **Coding Sequence (CDS)** | **Protein** |
|---|---|---|---|---|
| GA20 oxidase_3 | SEQ ID NO: 27 | SEQ ID NO: 28 | SEQ ID NO: 29 | SEQ ID NO: 30 |
| GA20 oxidase_5 | SEQ ID NO: 31 | SEQ ID NO: 32 | SEQ ID NO: 33 | SEQ ID NO: 34 |

A wild-type genomic DNA sequence of the GA20 oxidase_3 locus from a reference genome is provided in SEQ ID NO: 27, and A wild-type genomic DNA sequence of the GA20 oxidase_5 locus from a reference genome is provided in SEQ ID NO: 31.

For the corn GA20 oxidase_3 gene (also referred to as Zm.GA20ox3), SEQ ID NO: 27 provides 3000 nucleotides upstream (5') of the GA20 oxidase_3 5'-UTR; nucleotides 3001-3096 correspond to the 5'-UTR; nucleotides 3097-3665 correspond to the first exon; nucleotides 3666-3775 correspond to the first intron; nucleotides 3776-4097 correspond to the second exon; nucleotides 4098-5314 correspond to the second intron; nucleotides 5315-5584 correspond to the third exon; and nucleotides 5585-5800 correspond to the 3'-UTR. SEQ ID NO: 27 also provides 3000 nucleotides downstream (3') of the end of the 3'-UTR (nucleotides 5801-8800).

For the corn GA20 oxidase_5 gene (also referred to as Zm.GA20ox5), SEQ ID NO: 31 provides 3000 nucleotides upstream of the GA20 oxidase_5 start codon (nucleotides 1-3000); nucleotides 3001-3791 correspond to the first exon; nucleotides 3792-3906 correspond to the first intron; nucleotides 3907-4475 correspond to the second exon; nucleotides 4476-5197 correspond to the second intron; nucleotides 5198-5473 correspond to the third exon; and nucleotides 5474-5859 correspond to the 3'-UTR. SEQ ID NO: 31 also provides 3000 nucleotides downstream (3') of the end of the 3'-UTR (nucleotides 5860-8859).

**In** the corn genome, the Zm.GA20ox5 gene located next to the Zm.SAMT gene. These two genes are separated by an intergenic region of about 550 bp, with the Zm.SAMT gene positioned downstream and oriented in the opposite orientation relative to the Zm.GA20ox5 gene. A reference genomic sequence of the region encompassing the Zm.GA20ox5 and Zm.SAMT genes is provided in SEQ ID NOs. 35 and 36. SEQ ID NO. 35 represents the sequence of the sense strand of the Zm.GA20ox5 gene encompassing both Zm.GA20ox5 and Zm.SAMT genes (the "GA20ox5_SAMT genomic sequence" in Table 2). SEQ ID NO: 35 partially overlaps with SEQ ID NO: 31 and has a shorter Zm.GA20ox5 upstream sequence and a longer Zm.GA20ox5 downstream sequence compared to the SEQ ID NO: 31. SEQ ID NO. 36 represents the sequence of the sense strand of the Zm.SAMT gene *(i.e.,* the antisense strand of the Zm.GA20ox5 gene) encompassing both Zm.GA20ox5 and Zm.SAMT genes (the "SAMT_GA20ox5 genomic sequence" in Table 2). The elements or regions of the reference genomic Zm.GA20ox5 / Zm.SAMT sequence are annotated in Table 2 below by reference to the nucleotide coordinates of those elements or regions in SEQ ID NO. 35 or 36.

It was previously shown that suppression of GA20 oxidase gene(s) and/or targeting of a subset of one or more GA oxidase genes via transgenic suppression (e.g., an artificial microRNA-mediated suppression of both GA20 oxidase_3 and GA20 oxidase_5 genes) can be effective in achieving a short stature, semi-dwarf phenotype with increased resistance to lodging, but without reproductive off-types in the ear. *See* PCT Application No. PCT/US2017/047405 and US Application No. 15/679,699, both filed on August 17, 2017, and published as WO/2018/035354 and US20180051295, respectively. Furthermore, knocking out GA20 oxidase_3, GA20 oxidase_5, or both genes via genome editing also can cause reduced plant height and increased lodging resistance, and impacts GA hormonal levels. *See* PCT Application Nos. PCT/US2019/018128, PCT/US2019/018131, and PCT/US2019/018133, all filed on February 15, 2019.

**In** one aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert GA20 cDNA sequences).

**In** another aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert BR2 cDNA sequences).

**In** one aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert GA3 cDNA sequences).

**In** one aspect, a first gene region comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (insert Y1 cDNA sequences).

**In** an aspect, a deletion provided herein comprises all or part of a second gene region. **In** another aspect, a deletion provided herein comprises all of a second gene region. **In** still another aspect, a deletion provided herein comprises part of a second gene region.

**In** still another aspect, this disclosure provides a method of reducing expression of a gene in a cell comprising: a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; b) inducing a first double-stranded break and a second double-stranded break flanking a targeted region using a targeted editing technique, where the targeted region comprises the second coding region and the intervening region; and c) identifying one or more cells comprising a deletion of the targeted region of the chromosome, where the second promoter generates at least one antisense RNA of the first coding region, and where expression of the first coding region is reduced as compared to a control cell that does not comprise the deletion of the targeted region. **In** one aspect, a deletion leads to a portion of a first coding region being transcribed in reverse orientation.

In a further aspect, this disclosure provides a method comprising: a) identifying a chromosomal region comprising a first gene region comprising a first promoter and a first coding region, and a second gene region comprising a second promoter and a second coding region, where the first coding region and the second coding region are separated by an intervening region, and where the first promoter and the second promoter are positioned in opposite orientations; b) providing to one or more cells at least one RNA-guided nuclease or one or more vectors encoding at least one RNA-guided nuclease, where the at least one RNA-guided nuclease is capable of binding to at least 18, at least 19, at least 20, at least 21, at least 22, at least 23, at least 24, at least 25, or at least 26 consecutive nucleotides of a first target site and a second target site flanking a targeted region of a chromosome, where the targeted region comprises the second coding region and the intervening region, where the RNA-guided nuclease creates double-stranded breaks in the chromosome at the first target site and the second target site; b) identifying one or more cells comprising a deletion of the targeted region; and c) selecting one or more cells comprising the deletion of the targeted region.

In one aspect, this disclosure provides a modified plant, or part thereof, comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In one aspect, this disclosure provides a modified plant cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof. In another aspect, this disclosure provides a modified plant or modified plant tissue comprising a modified plant cell comprising a non-transposon mediated genome deletion or inversion of a gene or a portion thereof, at the endogenous locus of the gene, where the deletion or inversion results in the production of an RNA transcript comprising a sequence complementary to a native transcript sequence of the gene, or portion thereof.

It is known in the art that transposons, or transposable elements, are DNA sequences that can change their position within a genome. Transposons can create insertions, deletions, or inversions in a genome. In an aspect, methods, compositions, and cells provided herein do not comprise the use of a transposon *(e.g.,* "non-transposon mediated").

Methods provided herein are suitable for generating dominant alleles of protein-coding genes and non-coding RNAs. Without being limiting, examples of target genes of envisioned by the present disclosure in a plant genome would include genes for disease, insect, or pest tolerance; herbicide tolerance; genes for quality improvements such as yield, nutritional enhancements, environmental or stress tolerances; or any desirable changes in plant physiology, growth, development, morphology or plant product(s) including starch production (U.S. Pat. Nos. 6,538,181; 6,538,179; 6,538,178; 5,750,876; 6,476,295); modified oils production (U.S. Pat. Nos. 6,444,876; 6,426,447; 6,380,462); high oil production (U.S. Pat. Nos. 6,495,739; 5,608,149; 6,483,008; 6,476,295); modified fatty acid content (U.S. Pat. Nos. 6,828,475; 6,822,141; 6,770,465; 6,706,950; 6,660,849; 6,596,538; 6,589,767; 6,537,750; 6,489,461; 6,459,018); high protein production (U.S. Pat. No. 6,380,466); fruit ripening (U.S. Pat. No. 5,512,466); enhanced animal and human nutrition (U.S. Pat. Nos. 6,723,837; 6,653,530; 6,5412,59; 5,985,605; 6,171,640); or biopolymers (U.S. Pat. Nos. RE37,543; 6,228,623; 5,958,745 and U.S. Patent Publication No. US20030028917). Also environmental stress resistance (U.S. Pat. No. 6,072,103); pharmaceutical peptides and secretable peptides (U.S. Pat. Nos. 6,812,379; 6,774,283; 6,140,075; 6,080,560); improved processing traits (U.S. Pat. No. 6,476,295); improved digestibility (U.S. Pat. No. 6,531,648); low raffinose (U.S. Pat. No. 6,166,292); industrial enzyme production (U.S. Pat. No. 5,543,576); improved flavor (U.S. Pat. No. 6,011,199); nitrogen fixation (U.S. Pat. No. 5,229,114); hybrid seed production (U.S. Pat. No. 5,689,041); fiber production (U.S. Pat. Nos. 6,576,818; 6,271,443; 5,981,834; 5,869,720); and biofuel production (U.S. Pat. No. 5,998,700).

In one aspect, a gene edited by the methods provided herein is selected from the group consisting of a Y1 gene, a brachytic2 gene, a GA3 oxidase gene, and a GA20 oxidase gene. In another aspect, a gene edited by the methods provided herein encodes a non-coding RNA. In an aspect, a non-coding RNA edited by the methods provided herein is selected from the group consisting of a microRNA, a small interfering RNA, a transfer RNA, a ribosomal RNA, a *trans-acting* small interfering RNA, a naturally occurring antisense small interfering RNA, a heterochromatic small interfering RNA, and precursors thereof. In still another aspect, a gene edited by the methods provided herein encodes a miRNA. In a further aspect, a gene edited by the methods provided herein encodes a precursor miRNA (pre-miRNA).

In one aspect, a GA20 oxidase gene provided herein is encoded by an mRNA that encodes a protein having at least 80%, at least 85%, at least 90%, at least 91%, at least 92%, at least 93%, at least 94%, at least 95%, at least 96%, at least 97%, at least 98%, at least 99%, at least 99.5%, or 100% identical to a sequence selected from the group consisting of SEQ ID NOs: (insert protein sequences for GA20). In another aspect, a brachytic2 gene provided herein is encoded by an mRNA that encodes a protein having at least 80%, at least 85%, at least 90%, at least 91%, at least 92%, at least 93%, at least 94%, at least 95%, at least 96%, at least 97%, at least 98%, at least 99%, at least 99.5%, or 100% identical to a sequence selected from the group consisting of SEQ ID NOs: (insert protein sequences for BR2).

In one aspect, an unmodified allele provided herein comprises a polynucleotide sequence having at least 80%, at least 85%, at least 90%, at least 91%, at least 92%, at least 93%, at least 94%, at least 95%, at least 96%, at least 97%, at least 98%, at least 99%, at least 99.5%, or 100% identity or complementarity to a sequence selected from the group consisting of SEQ ID NOs: (list GA and BR2 sequences).

In another aspect, a non-coding RNA edited by the methods provided herein is selected from the group consisting of a microRNA, a small interfering RNA, a transfer RNA, a ribosomal RNA, a *trans-acting* small interfering RNA, a naturally occurring antisense small interfering RNA, a heterochromatic small interfering RNA, and precursors thereof.

### EXAMPLES

### Example 1. Generating a dominant allele of GA20 oxidase via targeted genomic inversion (For reference only)

Two functional guide RNAs (gRNAs) for a CRISPR/RNA-guided nuclease system are created to target flanking regions (a left target site and a right target site) of a GA20 oxidase_5 gene in the corn genome. *See* Figure 1, Panel A. Each of the two target sites are unique within the corn genome. The plant hormone gibberellin plays an important role in a number of plant developmental processes including germination, cell elongation, flowering, embryogenesis and seed development. Certain biosynthetic enzymes (e.g., GA20 oxidase and GA3 oxidase) and catabolic enzymes (e.g., GA2 oxidase) in the GA pathway are critical to affecting GA levels in plant tissues.

A transfer DNA (T-DNA) vector suitable for use in *Agrobacterium* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature com embryos are co-cultured with *Agrobacterium* containing the T-DNA vector for three days. The polynucleotides between the LB and RB sequences are integrated into the nuclear genome of the immature com embryos. Upon expression of the integrated polynucleotides, the gRNAs guide the nuclease to each of the two target sites in the GA20 oxidase_5 gene, where the nuclease creates a double-stranded break at each target site.

In the majority of events, the region between the target sites is deleted and non-homologous end-joining repair mechanisms joins the flanking regions. Less frequently, some events create insertion/deletion mutations at the left target site, the right target site, or both. In still other events the entire targeted region is inverted in what is referred to as a "complete inversion." *See* Figure 1, Panel B. Suitable methods known in the art *(e.g.,* PCR, DNA hybridization (Southern) blots, sequencing) are used to identify transformation events comprising a complete inversion. Transformed embryos comprising a targeted complete inversion in the 5' end of the GA20 oxidase_5 gene are selected and used to regenerate modified plants using techniques standard in the field.

Without being bound to any scientific theory, the presence of a complete inversion in one allele of the GA20 oxidase_5 creates a population of antisense mRNAs under the control of the native GA20 oxidase_5 promoter. The inverted region of the edited GA20 oxidase_5 allele mRNA and the corresponding region in the unedited GA20 oxidase_5 allele mRNA are complementary to each other and are capable of forming a dsRNA. Therefore, even though the modified com plant can be heterozygous for an edited GA20 oxidase_5 allele, the edited allele can reduce the expression of the GA20 oxidase_5 gene in the modified com plant.

RNA is extracted from modified com plants identified as comprising a complete inversion of the targeted region of the GA20 oxidase_5 gene. RNA is also extracted from control com plants that lack a complete inversion. Suitable methods known in the art *(e.g.,* quantitative reverse-transcriptase PCR, reverse-transcriptase PCR, RNA sequencing) are used to confirm that down-regulation of GA20 oxidase_5 occurs in modified com plants comprising a complete inversion of the targeted region.

### Example 2. Generating a dominant allele of BR2 via targeted genomic inversion (For reference only)

Two functional guide RNAs (gRNAs) for an RNA guided nuclease system are created to target flanking regions (a left target site and a right target site) of a BRACHYTIC2 (BR2) gene in the corn genome. Each of the two target sites are unique within the com genome.

A transfer DNA (T-DNA) vector suitable for use in *Agrobacterium tumefaciens* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature corn embryos are co-cultured with *Agrobacterium tumefaciens* containing the T-DNA vector for three days. The polynucleotides between the LB and RB sequences are integrated into the nuclear genome of the immature com embryos. Upon expression of the integrated polynucleotides, the gRNAs guide the CRISPR endonuclease to each of the two target sites in the BR2 gene, where the CRISPR endonuclease creates a double-stranded break at each target site.

In the majority of events, the region between the target sites is deleted and non-homologous end-joining repair mechanisms joins the flanking regions. Less frequently, some events create insertion/deletion mutations at the left target site, the right target site, or both. In still other events the entire targeted region is inverted in what is referred to as a "complete inversion." Suitable methods known in the art *(e.g.,* PCR, DNA hybridization (Southern) blots, sequencing) are used to identify transformation events comprising a complete inversion. Transformed embryos comprising a targeted complete inversion in the 5' end of the BR2 gene are selected and used to regenerate plants using techniques standard in the field.

Without being bound to any scientific theory, the presence of a complete inversion in one allele of the BR2 gene creates a population of anti-sense mRNAs under the control of the native BR2 promoter. The inverted region of the edited BR2 allele mRNA and the corresponding region in the unedited BR2 allele mRNA are complementary to each other and are capable of forming a dsRNA. Therefore, even though the com plant may be heterozygous for an edited BR2 allele, the edited allele can reduce the expression both alleles of the BR2 gene in the com plant, and thus results in a brachytic phenotype.

### Example 3. Generating a dominant allele of GA20 oxidase via a targeted genomic deletion to render a GA20 oxidase gene under the control of two reverse oriented promoters

A gene encoding GA20 oxidase_5 (also called GA20ox5) is located on com chromosome 8. It is adjacent to the corn gene GRMZM2G049269, which encodes an S-adenosyl-L-methionine-dependent methyltransferase superfamily protein ("SAMT" hereinafter). The SAMT is a member of a large, redundant gene family, and no phenotypes associated with mutations to this gene have been reported in com or *Arabidopsis.* The SAMT gene is positioned in the opposite orientation as compared to the GA20 oxidase_5 gene *(i.e.,* the SAMT gene is oriented to read 5' to 3', while the GA20 oxidase_5 gene is oriented to read 3' to 5' on the same DNA strand). *See* Figure 2, Panel A.

Two functional guide RNAs (gRNAs) for an RNA guidednuclease system are created to target the genomic DNA region between the GA20 oxidase_5 gene and the SAMT gene. The first gRNA targets an area near the transcriptional start site of the SAMT gene, and the second gRNA targets a region near the transcriptional stop site of the GA20 oxidase_5 gene. Each of the two target sites are unique within the corn genome. A transfer DNA (T-DNA) vector suitable for use in *Agrobacterium* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature com embryos are co-cultured with *Agrobacterium* containing the T-DNA vector for three days. The polynucleotides between the LB and RB sequences are integrated into the nuclear genome of the immature com embryos. Upon expression of the integrated polynucleotides, the gRNAs guide the nuclease to each of the two target sites in the genomic DNA region between the GA20 oxidase_5 gene and the SAMT gene, where the nuclease creates a double-stranded break at each target site.

In the majority of events, the region between the target sites is deleted and non-homologous end-joining repair mechanisms joins the flanking regions. Suitable methods known in the art *(e.g.,* PCR, DNA hybridization (Southern) blots, sequencing) are used to identify transformation events comprising a complete deletion. Transformed embryos comprising a targeted deletion between the GA20 oxidase_5 gene and SAMT gene are selected and used to regenerate modified plants using techniques standard in the field.

Without being bound to any scientific theory, by removing the genomic DNA between the GA20 oxidase_5 gene and the SAMT promoter, the native SAMT promoter can generate an antisense mRNA transcript of the GA20 oxidase_5 gene, while the native GA20 oxidase_5 promoter generates a sense mRNA transcript of the GA20 oxidase_5 gene. The complementary sense and antisense mRNA transcripts of the GA20 oxidase_5 gene are capable of forming a dsRNA that can be processed by RNA silencing mechanisms native to the corn cell. *See* Figure 2, Panel B. The processed dsRNA can then suppresses the expression of both GA20 oxidase_5 alleles. Additionally, because the mRNA encoding GA20 oxidase_3 is so similar to the sense GA20 oxidase_5 transcript, it is expected that the deletion between the SAMT promoter and the GA20 oxidase_5 gene will also down-regulate the expression of GA20 oxidase_3. It is also envisioned that suppression or silencing of the GA20 oxidase_5 gene may occur through other mechanisms as provided herein (e.g., nonsense-mediated decay), alternatively or in addition to any RNAi or PTGS forms of suppression.

RNA is extracted from modified com plants identified as comprising a targeted deletion between the SAMT promoter and the GA20 oxidase_5 gene. RNA is also extracted from control com plants that lack the deletion. Suitable methods known in the art *(e.g.,* quantitative reverse-transcriptase PCR, reverse-transcriptase PCR, RNA sequencing) are used to confirm that down-regulation of GA20 oxidase_5 and/or GA20 oxidase_3 occurs in corn modified plants comprising a deletion of the targeted region.

### Example 4. Generating a dominant allele of BR2 via a targeted genomic deletion to render a BR2 gene under the control of two reverse oriented promoters

A gene encoding BR2 is located on corn chromosome 1. It is adjacent to the corn gene GRMZM2G491632 which is expressed in the opposite orientation to BR2. Two functional guide RNAs (gRNAs) for an RNA guided nuclease system are created to target the genomic DNA region between the BR2 gene and the GRMZM2G491632 gene. The first gRNA targets an area near the end of the BR2 gene exon 1, and the second gRNA targets a region near the beginning of the coding sequence of the GRMZM2G491632 gene exon 1.

A transfer DNA (T-DNA) vector suitable for use in *Agrobacterium* transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above

Immature com embryos are co-cultured with *Agrobacterium* containing the T-DNA vector for three days. The polynucleotides between the LB and RB sequences are integrated into the nuclear genome of the immature com embryos. Upon expression of the integrated polynucleotides, the gRNAs guide the endonuclease to each of the two target sites in the genomic DNA region between the BR2 gene and the GRMZM2G491632 gene, where the endonuclease creates a double-stranded break at each target site.

In the majority of events, the region between the target sites is deleted and non-homologous end-joining repair mechanisms joins the flanking regions. Suitable methods known in the art *(e.g.,* PCR, DNA hybridization (Southern) blots, sequencing) are used to identify transformation events comprising a deletion. Transformed embryos comprising a targeted deletion between the BR2 gene and the GRMZM2G491632 gene are selected and used to regenerate plants using techniques standard in the field.

Without being bound to any scientific theory, by removing the genomic DNA between the BR2 gene and the GRMZM2G491632 promoter, the native GRMZM2G491632 promoter can generate an anti-sense mRNA transcript of the BR2 gene while the native BR2 promoter generates a sense mRNA transcript of the BR2 gene. The complementary sense and anti-sense mRNA transcripts of the BR2 gene can form a dsRNA that can be processed by RNAi machinery native to the com cell. The processed dsRNA could then suppress the expression of both BR2 alleles, resulting in a brachytic phenotype.

### Example 5. Insertion of designed elements into native promoters using genome editing techniques to create a dominant allele.(For reference only)

A gene comprising a root-specific promoter is identified in the *Arabidopsis thaliana* genome. *See* Figure 3, Panel A. This promoter is used to drive the expression of GUS in the roots of the plant. *See* Figure 3, Panel B. A functional gRNA is designed to target a region upstream of a TATA box of the root-specific promoter (the "target site"). A transfer DNA (T-DNA) vector suitable for use in *Agrobacterium* transformation is used to introduce the gRNA to *Arabidopsis.* The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above. A second T-DNA construct comprises a polynucleotide encoding a donor molecule comprising a designed element to be inserted at the target site, between a LB sequence and a RB sequence. In one embodiment, the donor molecule comprises the designed element flanked by homologous regions that are homologous to sequences present on either side of the target site. The designed element enables the formerly root-specific gene to be expressed constitutively in all tissues of the plant when it is inserted into the promoter region of a gene. In another embodiment, the donor molecule comprises the designed sequence flanked by target sites that are targeted by the gRNA in T-DNA vector 1.

The floral dip method is used to transform *Arabidopsis* using the vector described above. *See* Clough and Bent, 1998, Plant J, 16: 735-743. Upon expression of the integrated polynucleotides, the gRNA guides the nuclease to the target site and creates a double-stranded break at the target site. For donor molecues comprising designed sequence flanked by Homology arms, homologous repair mechanisms native to the *Arabidopsis* cells insert the designed element at the site of the double-stranded break. For donor molecules comprising the designed sequence flanked by gRNA target sites, the gRNAs guide the nuclease to create double standed breaks within the second T-DNA thereby releasing the designed sequence, which can then integrate within the genomic target site via NHEJ (Non Homologous End Joining) repair mechanisms. In a subset of the insertion events, the promoter inserts in the desired orientation. Without being bound by any particular theory, the presence of the designed element upstream of the TATA box induces constitutive expression of the gene throughout the modified plant, thereby creating a dominant allele of the gene.

Suitable methods known in the art (e.g., PCR, DNA hybridization (Southern) blots, sequencing) are used to identify transformation events comprising a targeted insertion of the designed element in the desired orientation. Transformed *Arabidopsis* plants comprising the designed element at the target site are selected and further examined.

Plants identified as comprising the designed element upstream of the TATA box *(see* Figure 3, Panel D) are examined for the expression of GUS using techniques standard in the art. Plants comprising the designed element exhibit GUS expression throughout the plant. *See* Figure 3, Panel C. Additionally, RNA is extracted from various tissues *(e.g.,* roots, stem, leaf, inflorescence) of the modified *Arabidopsis* plants identified as comprising the designed element. RNA is also extracted from control *Arabidopsis* plants that lack the designed element. Suitable methods known in the art (e.g., quantitative reverse-transcriptase PCR, reverse-transcriptase PCR, RNA sequencing) are used to confirm that GUS is more broadly expressed and/or more strongly expressed in the modified *Arabidopsis* plants.

### Example 6. Insertion of tissue-specific suppression elements using genome editing techniques to create a dominant allele. (For reference only)

An *Arabidopsis thaliana* plant comprising a GUS transgene under the control of a promoter that is functional in leaf, vascular, and root tissue is created using standard techniques in the art. *See* Figure 4 for a general overview of the concept provided in this Example; and Figure 5, Panels A and B. A functional gRNA is designed to target a region downstream of the GUS gene (the "target site"). A transfer DNA (T-DNA) vector suitable for use in *Agrobacterium* transformation is used to introduce the gRNA to *Arabidopsis.* The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above. A second T-DNA construct comprises a donor molecule comprising a leaf-specific promoter such as the COOL AIR promoter (see Chen and Penfield, Science, 2018, 360: 6392) between a LB sequence and a RB sequence. In one embodiment, the donor molecule comprises the promoter in an antisense orientation flanked by homologous regions that are homologous to sequences present on either side of the target site. The antisense oriented leaf-specific promoter enables the expression of a GUS antisense mRNA in tissues where the promoter is expressed (e.g., leaf tissue). Without being bound by any particular theory, the antisense RNA transcript of the GUS gene leads to the silencing of GUS in leaf tissue, but not root tissue. In another embodiment, the donor molecule comprises the promoter sequence flanked by target sites that are targeted by the gRNA in T-DNA vector 1.

The floral dip method is used to transform *Arabidopsis* using the vector described above. *See* Clough and Bent, 1998, Plant J, 16: 735-743. Upon expression of the polynucleotides, the gRNA guides the nuclease to the target site and creates a double-stranded break at the target site. For donor molecues comprising the antisense oriented promoter flanked by homology arms, homologous repair mechanisms native to the *Arabidopsis* cells insert the leaf-specific promoter at the site of the double-stranded break downstream of the GUS gene, thereby placing the promoter in an anti-sense orientation to the GUS gene. For donor molecues comprising the promoter flanked by gRNA target sites, the gRNAs guide the nuclease to create double standed breaks within the second T-DNA thereby releasing the promoter sequence that can then integrate within the genomic target site via NHEJ (Non Homologous End Joining) repair mechanisms. In a subset of the insertion events, the promoter inserts in an anti-sense orientation. Without being bound by a particular theory, the presence of the antisense leaf-specific promoter induces reduced expression of GUS throughout leaf tissue of the plant, thereby creating a dominant allele of the gene.

Suitable methods known in the art (e.g., PCR, DNA hybridization (Southern) blots, sequencing) are used to identify transformation events comprising a targeted insertion of the promoter in the desired orientation. Transformed *Arabidopsis* plants comprising the leaf-specific promoter at the target site are selected and further examined.

Plants identified as comprising the leaf-specific promoter downstream of the GUS gene in antisense orientation to the GUS gene *(see* Figure 5, Panel D) are examined for the expression of GUS using techniques standard in the art. Plants comprising the leaf-specific promoter in antisense orientation to the GUS gene exhibit GUS expression only in root tissues. *See* Figure 5, Panel C. Additionally, RNA is extracted from various tissues *(e.g.,* roots, stem, leaf, inflorescence) of the modified *Arabidopsis* plants identified as comprising the leaf-specific promoter. RNA is also extracted from control *Arabidopsis* plants that lack the leaf-specific promoter in antisense orientation to the GUS gene. Suitable methods known in the art (e.g., quantitative reverse-transcriptase PCR, reverse-transcriptase PCR, RNA sequencing) are used to confirm that expression of GUS is reduced in leaf tissues.

### Example 7. Insertion of tissue-specific suppression elements using genome editing techniques to create a dominant GA20 oxidase_5 allele. (For reference only)

A functional gRNA is designed to target a region downstream of the 3'-UTR of the GA20 oxidase_5 gene (the "target site"). A transfer DNA (T-DNA) vector suitable for use in *Agrobacterium* transformation is used to introduce the gRNA to a corn cell. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above. A second T-DNA construct comprises a donor molecule comprising an RTBV promoter between a LB sequence and a RB sequence. In one embodiment, the donor molecule comprises the RTBV promoter in an antisense orientation, flanked by homologous regions that are homologous to sequences present on either side of the target site. In another embodiment, the donor molecule comprises the promoter sequence flanked by target sites that are targeted by the gRNA in T-DNA vector 1.The antisense oriented RTBV promoter enables the expression of a GA20 oxidase_5 antisense mRNA in tissues where RTBV is expressed (e.g., stem and vascular tissue). Without being bound by any particular theory, the antisense RNA transcript of the GA20 oxidase_5 gene leads to the silencing of both GA20 oxidase_5 and GA20 oxidase_3 in stem and vascular tissue.

Immature com embryos are co-cultured with *Agrobacterium* containing the T-DNA vectors for three days. Upon expression of the polynucleotides, the gRNA guides the nuclease to the target site and creates a double-stranded break at the target site. For donor molecues comprising the antisense oriented RTBV promoter flanked by Homology arms, homologous repair mechanisms native to the com cell insert the the antisense RTBV promoter at the target site downstream of the 3' end of the GA20 oxidase_5 gene. For donor molecules comprising the promoter flanked by gRNA target sites, the gRNAs guide the nuclease to create double standed breaks within the second T-DNA there by releasing the promoter sequence that can then integrate within the genomic target site via NHEJ (Non Homologous End Joining) repair mechanisms. In a subset of the insertion events, the promoter inserts in an anti-sense orientation.

Suitable methods known in the art (e.g., PCR, DNA hybridization (Southern) blots, sequencing) are used to identify transformation events comprising a targeted insertion of the antisense RTBV promoter. Transformed embryos comprising the antisense RTBV promoter at the target site are selected and used to regenerate modified plants using techniques standard in the field.

RNA is extracted from various tissues (e.g., roots, stem, leaf, inflorescence) of the modified com plants identified as comprising the antisense RTBV promoter. RNA is also extracted from control com plants that lack the antisense RTBV promoter at the target site. Suitable methods known in the art (e.g., quantitative reverse-transcriptase PCR, reverse-transcriptase PCR, RNA sequencing) are used to confirm that expression of GA20 oxidase_5 and/or GA20 oxidase_3 are reduced in stem and vascular tissue in the modified com plants comprising the antisense RTBV promoter as compared to control com plants.

### Example 8. Engineering a truncated protein using genome editing techniques to create a dominant allele. (For reference only)

**A) Engineering GA20 oxidase truncated proteins:** Targeted editing of genes that result in truncated proteins or non-sense mutations to the proteins can create dominant alleles. *See* Figure 6. The corn GA20 Oxidase_5 gene and GA20 Oxidase_3 gene are highly similar in sequence and structure. Both genes comprise three exons. A gRNA is designed to introduce an edit in an exon of both genes.

A transfer DNA (T-DNA) vector suitable for use in *Agrobacterium* transformation is used to introduce the gRNA to a com cell. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above.

Immature com embryos are co-cultured with *Agrobacterium* containing the T-DNA vectors for three days. The polynucleotides between the LB and RB sequences are integrated into the nuclear genome of the immature com embryos. Upon expression of the integrated polynucleotides, the gRNA guides the nuclease to the target site and creates a double-stranded break at the target site. Without being bound by a particular theory, the non-homologous end-joining repair mechanisms native to the cell frequently repair such breaks imperfectly, which can lead to the insertion or deletion of one or more nucleotides. These insertions or deletions to an exon can produce premature stop codons (which would generate a truncated protein), or non-sense mutations. A premature stop codon has the capability of generating a dominant allele of GA20 Oxidase_5 and GA20 Oxidase_3.

Suitable methods known in the art (e.g., PCR, DNA hybridization (Southern) blots, sequencing) are used to identify transformation events comprising a targeted insertion or deletion in the GA20 Oxidase_5 and/or GA20 Oxidase_3 genes. Transformed embryos comprising an insertion or deletion capable of introducing a premature stop codon causing a truncated protein or a non-sense mutation at the target site are selected and used to regenerate modified plants using techniques standard in the field.

Protein is extracted from the modified com plants identified as comprising the identified insertion/deletion. Suitable methods known in the art (e.g., Western blot; HPLC; LC/MS; ELISA; immunoprecipitation) are used to confirm that a truncated protein or a non-sense mutation has been introduced to GA20 Oxidase_5 and/or GA20 Oxidase_3 genes. Additional experiments to confirm a reduction in gibberellin acid in stem and/or vascular tissue are performed as described in Bensen et al., Plant Physiol. 1990, 94: 77-84.

**B)Engineering Brachytic 2 (Br2) truncated protein:** A gRNA is designed to introduce an edit within an exon of the Brachytic 2 gene from corn.

A transfer DNA (T-DNA) vector suitable for use in *Agrobacterium* transformation is used to introduce the gRNA to a com cell. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the gRNA described above.

Immature com embryos are co-cultured with *Agrobacterium* containing the T-DNA vectors for three days. The polynucleotides between the LB and RB sequences are integrated into the nuclear genome of the immature com embryos. Upon expression of the integrated polynucleotides, the gRNA guides the nuclease to the target site where the nuclease creates a double-stranded break at the target site. Without being bound by a particular theory, the non-homologous end-joining repair mechanisms native to the cell frequently repair such breaks imperfectly, which can lead to the insertion or deletion of one or more nucleotides. These insertions or deletions to an exon can produce premature stop codons (which would generate a truncated protein), or non-sense mutations. A premature stop codon has the capability of generating a dominant allele of Brachytic 2 (Br2).

Suitable methods known in the art (e.g., PCR, DNA hybridization (Southern) blots, sequencing) are used to identify transformation events comprising a targeted insertion or deletion in the Br2 gene. Transformed embryos comprising an insertion or deletion capable of introducing a premature stop codon (causing a truncated protein) or a non-sense mutation at the target site are selected and used to regenerate modified plants using techniques standard in the field.

Protein is extracted from the modified com plants identified as comprising the identified insertion/deletion. Suitable methods known in the art (e.g., Western blot; HPLC; LC/MS; ELISA; immunoprecipitation) are used to confirm that a Br2 truncated protein is generated.

### Example 9. Generating an inverted repeat within a target gene (For reference only)

Targeted editing techniques can be used to convert a genomic locus into a locus that is capable of generating an RNAi-inducing hairpin when the edited locus is transcribed into RNA. *See* Figure 7. In a cell that is heterozygous at the locus of interest (*e.g.,* two polymorphic alleles are present), one or more nucleases are used to generate two double-stranded breaks (e.g., a first double-stranded break and a second double-stranded break) in the first allele, and one double-stranded break (e.g., a third double-stranded break) in the second allele. When the nucleases cut the first and second alleles, the portion of the first allele that is flanked by the first and second double-stranded breaks is released from the genomic DNA. In one outcome, the released portion of the first allele is inverted in orientation and integrated into the third double-stranded break in the second allele, thereby creating an edited locus that is capable of generating an RNAi-inducing hairpin when the edited locus is transcribed.

A first and a second functional guide RNA (gRNA) for a RNA guided nuclease system are created. The first and second gRNAs are complementary to a first target site and a second target site, respectively, flanking a portion of a first allele of a GA20 oxidase_5 gene in the corn genome. The first gRNA is also complementary to a second allele of the GA20 oxidase_5 gene at a third target site (which is homologous to the first target site), but the second gRNA is not complementary to the second allele due to a polymorphism between the first and second GA20 oxidase_5 alleles at the second target site.

A transfer DNA (T-DNA) vector suitable for use in *Agrobacterium* transformation is constructed. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises an expression cassette comprising a promoter operable in a plant cell operably linked to a polynucleotide encoding the first and second gRNAs described above.

Immature com embryos are co-cultured with *Agrobacterium* containing the T-DNA vector for three days. Upon expression of the polynucleotides, the gRNAs guide the nuclease to each of the three target sites in the GA20 oxidase_5 alleles, where the nuclease creates a double-stranded break at each target site.

In the majority of events, the region between the first and second target sites in the first GA20 oxidase_5 allele is deleted and non-homologous end-joining repair mechanisms joins the flanking regions. Less frequently, some events create insertion/deletion mutations at the first target site, the second target site, or both. In still other events the entire targeted region of the first allele integrates into the double-stranded break at the third target site in an inverted orientation. *See* Figure 7, Panel D. Suitable methods known in the art *(e.g.,* PCR, DNA hybridization (Southern) blots, sequencing) are used to identify transformation events comprising an inversion in the second GA20 oxidase_5 allele. Transformed embryos comprising an inversion in the second GA20 oxidase_5 allele are selected and used to regenerate modified plants using techniques standard in the field.

Without being bound to any scientific theory, the presence of an inversion in one allele of the GA20 oxidase_5 creates a population of RNA transcripts capable of forming a hairpin structure. Such a hairpin induces RNAi machinery in the cell, which leads to down-regulation of GA20 oxidase_5 RNA transcripts in a dominant manner (e.g., edited and unedited alleles are both down-regulated).

RNA is extracted from modified com plants identified as comprising an inversion in the second allele capable of generating a hairpin RNA transcript. RNA is also extracted from control corn plants that lack edited GA20 oxidase_5 alleles. Suitable methods known in the art (e.g., quantitative reverse-transcriptase PCR, reverse-transcriptase PCR, RNA sequencing) are used to confirm that down-regulation of GA20 oxidase_5 occurs in modified com plants comprising an inversion in the second allele capable of generating a hairpin RNA transcript. Additionally, due to the sequence similarities between GA20 oxidase_5 and GA20 oxidase_3, the inversion in the second allele of GA20 oxidase_5 also causes down-regulation of GA20 oxidase_3 RNA transcripts.

### Example 10. Inserting a miRNA target site into a desired genomic locus (For reference only)

Targeted editing techniques can be used to insert a donor molecule into a target site in a genomic locus. *See* Figure 8. If a donor molecule comprising a non-coding RNA target site is inserted into a 5'-UTR, an exon, an intron, or a 3'-UTR of a gene of interest, RNA transcription or protein translation of the gene of interest can be suppressed by a complementary non-coding RNA. When a gene of interest is a target of a non-coding RNA *(e.g.,* a miRNA or an siRNA), a cleaved mRNA from the gene of interest can generate secondary siRNAs, which can further suppress the transcription or translation of the gene of interest. Such secondary suppression can act in a dominant manner, as the secondary siRNAs are complementary to alleles with and without the insertion of the non-coding RNA target site.

A functional guide RNA (gRNA) is created that is complementary to a target site in the 3'-UTR of a GA20 oxidase_5 gene. A transfer DNA (T-DNA) vector suitable for use in *Agrobacterium* transformation is constructed. The T-DNA construct comprises a promoter that is operable in a plant cell operably linked to polynucleotides encoding a) an RNA guided/nuclease; b) CP4-EPSPS marker gene; c) the gRNA described above; and d) a donor molecule, between a left border (LB) sequence and a right border (RB) sequence. The donor molecule comprises a 21 nucleotide sequence homologous to miR166, as well as a first and a second homologous region that are homologous to the 3'-UTR of the GA20 oxidase_5 gene on either side of the target site.

Immature com embryos are co-cultured with *Agrobacterium* containing the T-DNA vector for three days. Upon expression of the polynucleotides, the gRNA guides the nuclease to the target site in the GA20 oxidase_5 3'-UTR, where the nuclease creates a double-stranded break. Homologous recombination repair mechanisms then insert the donor molecule into the target site, thereby incorporating a miR166 target site into the 3'-UTR of the GA20 oxidase_5 gene.

Suitable methods known in the art (e.g., PCR, DNA hybridization (Southern) blots, sequencing) are used to identify transformation events comprising the insertion of the miR166 target site into the 3'-UTR of the GA20 oxidase_5 gene. Transformed embryos comprising the insertion of the miR166 target site into the 3'-UTR of the GA20 oxidase_5 gene are selected and used to regenerate modified plants using techniques standard in the field.

Without being bound to any scientific theory, the presence of a miRNA binding site in GA20 oxidase_5 creates a population of secondary siRNA transcripts capable of suppressing GA20 oxidase_5 RNA transcription in a dominant negative manner.

RNA is extracted from modified com plants identified as comprising a miR166 target site insertion in the 3'-UTR of a GA20 oxidase_5 gene. RNA is also extracted from control com plants that lack an edited GA20 oxidase_5 gene. Suitable methods known in the art (e.g., quantitative reverse-transcriptase PCR, reverse-transcriptase PCR, RNA sequencing) are used to confirm that down-regulation of GA20 oxidase_5 occurs in modified com plants. Additionally, due to the sequence similarities between GA20 oxidase_5 and GA20 oxidase_3, the miRNA target site in the GA20 oxidase_5 gene is also capable of causing down-regulation of GA20 oxidase_3 RNA transcripts.

### Example 11. Generating dominant negative alleles by creating truncated proteins (For reference only)

Targeted editing of genes that result in truncated proteins (e.g., non-sense mutations) can create dominant negative alleles. In one embodiment, the targeted gene encodes a protein that has protein: protein interaction domains. *See* Figure 6. Examples of dominant truncated proteins are known in various plant species. For example, dominant mutant phenotypes caused by truncated proteins are known for *FAZ1* in rice and *AGAMOUS* and *SOC1* in *Arabidopsis thaliana.* The peptide CLAVATA3 (CLV3) is processed to a signaling peptide (CLE), which is bound by the receptor-like kinases CLV1 and CORYNE (CRN) and the receptor-like protein CLV2 to regulate the expression of *WUSCHEL (WUS)* in meristems. Com plants comprising mutant alleles of *CLV2* often exhibit ears with increased number of kernel rows. Without being limited by a particular theory, when *CLV2* is mutated, *WUS* expression can increase, which leads to an increased meristem size, which in turn gives rise to an increased number of kernel rows on the ear. CLV2 comprises an extracellular domain and a transmembrane domain, which forms a complex with CRN. A truncated CLV2 protein could function in a dominant manner to increase meristem size in corn.

A gRNA is designed to introduce a stop codon in the extracellular domain of CLV2 in corn. A transfer DNA (T-DNA) vector suitable for use in *Agrobacterium* transformation is used to introduce the gRNA to a com cell. The T-DNA construct comprises a promoter that is operable in a plant cell operably linked to polynucleotides encoding a) a RNA guidednuclease; b) CP4-EPSPS marker gene; and c) the gRNA described above, between a left border (LB) sequence and a right border (RB) sequence.

Immature com embryos are co-cultured with *Agrobacterium* containing the T-DNA vectors for three days. The polynucleotides between the LB and RB sequences are integrated into the nuclear genome of the immature com embryos. Upon expression of the integrated polynucleotides, the gRNA guides the nuclease to the target site and creates a double-stranded break at the target site. Without being bound by any theory, the non-homologous end-joining repair mechanisms native to the cell frequently repair such breaks imperfectly, which can lead to the insertion or deletion of one or more nucleotides. These insertions or deletions to an exon can produce premature stop codons (which would generate a truncated protein), or non-sense mutations. Such a mutation can generate a dominant negative allele of *CLV2.*

Suitable methods known in the art *(e.g.,* PCR, DNA hybridization (Southern) blots, sequencing) are used to identify transformation events comprising a targeted insertion or deletion in the *CLV2* gene. Transformed embryos comprising an insertion or deletion capable of causing a truncated protein at the target site are selected and used to regenerate modified plants using techniques standard in the field.

Protein is extracted from the modified corn plants identified as comprising the identified insertion/deletion. Suitable methods known in the art (e.g., Western blot; HPLC; LC/MS; ELISA; immunoprecipitation) are used to confirm that a non-sense mutation has been introduced to the *CLV2* gene. Additional phenotypic screening of ear kernel rows is done to an increase in meristem size. Light microscopy of sectioned meristems from modified and control plants is also done to quantify the increase in meristem size.

### Example 12. Preventing cleavage of a target gene using an engineered PPR protein (For reference only)

Members of the pentatricopeptide repeat (PPR) gene family are common in plant genomes. Many PPR proteins are capable of binding RNA molecules in a sequence-specific manner. PPR proteins comprise 2-30 PPR motifs, each of which aligns to a single nucleotide in an RNA molecule. Within the PPR motifs, the amino acids present at two or three specific positions confer nucleotide specificity. For example, without being limiting, a PPR motif binds an adenine nucleotide when a threonine is in position 6 and an asparagine is at the 1' position; a PPR motif binds a guanine nucleotide when a threonine is in position 6 and an aspartic acid is at the 1' position; a PPR motif binds a uracil (or thymine) nucleotide when an asparagine is at position 6 and an aspartic acid is at the 1' position; and a PPR motif binds a cytosine nucleotide when an asparagine is at position 6 and an asparagine or a serine is at the 1' position.

Without being limiting, an engineered PPR protein can be generated by at least two building strategies. In the first strategy, a PPR protein is constructed by treating each PPR motif as a separate block, such that a PPR protein is constructed by putting multiple desired motifs in order. The resulting engineered PPR protein is then capable of binding a target RNA molecule. However, such a strategy may not always work because each PPR motif comprises an internal scaffolding between the 1' and 6 positions, and that intra-motif scaffold is not shared across different PPR proteins. The second strategy makes use of the preexisting intra-motif scaffolding. In the second strategy, site-directed mutagenesis of the 1' and 6 positions is used to edit an existing PPR protein such that it will be specific to a new target RNA molecule.

An engineered PPR protein comprising PPR motifs is engineered such that the PPR protein can specifically bind to nucleotides of the miRNA target site of a suitable gene and the PPR protein is targeted to the cytoplasm. A nucleic acid sequence encoding the engineered PPR protein is inserted into a transfer DNA (T-DNA) vector suitable for use in *Agrobacterium* transformation. The nucleic acid sequence encoding the engineered PPR protein is operably linked to the promoter of the target gene or a constitutive promoter to ensure overlapping expression of the engineered PPR protein and the target gene mRNA. The nucleic acid molecule encoding the engineered PPR protein and operably linked promoter is positioned between a left border (LB) sequence and a right border (RB) sequence within the T-DNA construct.

Immature com embryos are co-cultured with *Agrobacterium* containing the T-DNA vector for three days. The polynucleotides between the LB and RB sequences are integrated into the nuclear genome of the immature com embryos. Upon expression of the integrated polynucleotides, the engineered PPR protein is expressed and binds to complementary target mRNAs.

Suitable methods known in the art (e.g., PCR, DNA hybridization (Southern) blots, sequencing) are used to identify transformation events comprising the insertion of the engineered PPR protein encoding nucleic acid molecule. Transformed embryos comprising the insertion of the engineered PPR protein encoding nucleic acid molecule are selected and used to regenerate modified plants using techniques standard in the field.

Without being bound to any scientific theory, the engineered PPR protein binds to the target gene mRNA and prevents the microRNA from suppressing the target gene.

RNA and protein is extracted from modified corn plants identified as comprising the engineered PPR protein. RNA and protein is also extracted from control com plants that lack the engineered PPR protein. Suitable methods known in the art (e.g., quantitative reverse-transcriptase PCR, reverse-transcriptase PCR, RNA sequencing, Western blot, HPLC, LC/MS, ELISA, immunoprecipitation) are used to confirm that expression of THE TARGET GENE is increased in modified com plants as compared to unmodified control com plants.

### Example 13. Cleaving a target gene transcript using an engineered PPR protein coupled to a nuclease (For reference only)

An engineered PPR protein comprising an NYN nuclease domain and PPR motifs is engineered as described in Example 12 such that the PPR protein can specifically bind to certain nucleotides of a target gene and the PPR protein is targeted to the nucleus. A nucleic acid sequence encoding the engineered PPR protein is inserted into a transfer DNA (T-DNA) vector suitable for use in *Agrobacterium* transformation. The nucleic acid sequence encoding the engineered PPR protein is operably linked to the promoter of the target gene or a constitutive promoter to ensure overlapping expression of the engineered PPR protein and the target gene mRNA. The nucleic acid molecule encoding the engineered PPR protein and operably linked promoter is positioned between a left border (LB) sequence and a right border (RB) sequence within the T-DNA construct.

Immature com embryos are co-cultured with *Agrobacterium* containing the T-DNA vector for three days. The polynucleotides between the LB and RB sequences are integrated into the nuclear genome of the immature com embryos. Upon expression of the integrated polynucleotides, the engineered PPR protein is expressed and binds to complementary target mRNAs.

Suitable methods known in the art (e.g., PCR, DNA hybridization (Southern) blots, sequencing) are used to identify transformation events comprising the insertion of the engineered PPR protein encoding nucleic acid molecule. Transformed embryos comprising the insertion of the engineered PPR protein encoding nucleic acid molecule are selected and used to regenerate modified plants using techniques standard in the field.

Without being bound to any scientific theory, the engineered PPR protein binds to the target gene mRNA, and the NYN nuclease domain cleaves the target gene mRNA. Therefore, expression of the target gene decreases.

RNA and protein are extracted from modified corn plants identified as comprising the engineered PPR protein. RNA and protein are also extracted from control corn plants that lack the engineered PPR protein. Suitable methods known in the art (e.g., quantitative reverse-transcriptase PCR, reverse-transcriptase PCR, RNA sequencing, Western blot, HPLC, LC/MS, ELISA, immunoprecipitation) are used to confirm that expression of the target gene is decreased in modified com plants as compared to unmodified control com plants.

### Example 14. Blocking translation of a target gene transcript using an engineered PPR protein (For reference only)

An engineered PPR protein comprising PPR motifs is engineered such that the PPR protein can specifically bind to specific nucleotides of an mRNA encoded by the target gene[GENE X], and the PPR protein is targeted to the cytoplasm. A nucleic acid sequence encoding the engineered PPR protein is inserted into a transfer DNA (T-DNA) vector suitable for use in *Agrobacterium* transformation. The nucleic acid sequence encoding the engineered PPR protein is operably linked to the promoter of the target gene or a constitutive promoter to ensure overlapping expression of the engineered PPR protein and the target gene mRNA. The nucleic acid molecule encoding the engineered PPR protein and operably linked promoter is positioned between a left border (LB) sequence and a right border (RB) sequence within the T-DNA construct.

Immature com embryos are co-cultured with *Agrobacterium* containing the T-DNA vector for three days. The polynucleotides between the LB and RB sequences are integrated into the nuclear genome of the immature corn embryos. Upon expression of the integrated polynucleotides, the engineered PPR protein is expressed and binds to complementary target mRNAs.

Suitable methods known in the art (*e.g*., PCR, DNA hybridization (Southern) blots, sequencing) are used to identify transformation events comprising the insertion of the engineered PPR protein encoding nucleic acid molecule. Transformed embryos comprising the insertion of the engineered PPR protein encoding nucleic acid molecule are selected and used to regenerate modified plants using techniques standard in the field.

Without being bound to any scientific theory, the engineered PPR protein binds to the target gene mRNA and prevents translation of the mRNA into protein. Therefore, protein translation of the target gene decreases.

Protein is extracted from modified corn plants identified as comprising the engineered PPR protein. Protein is also extracted from control corn plants that lack the engineered PPR protein. Suitable methods known in the art (e.g., Western blot, HPLC, LC/MS, ELISA, immunoprecipitation) are used to confirm that protein translation of the target gene is decreased in modified corn plants.

### Example 15. Design of a tether guide oligo (tgOligo). (For reference only)

A Cas9/sgRNA complex binds to a dsDNA molecule comprising target and non-target strands (Figure 9). Cas9-PAM interaction occurs on the non-target strand; gRNA-DNA annealing occurs on the target strand. RuvC (His840) and HNH (Asp10) nuclease domains cut the non-target and target strands, respectively (triangles). The blunt ends at the Cas9 cut site are held in place by Cas9 at the 5' end of the non-target strand (PAM location), and at both cut ends (3' and 5') of the target strand. The 3' cut end of the non-target strand is free and 'flaps' around. The 3' free 'flap' end of the non-target strand can be up to 35 nucleotides which can be sufficient for specific complementarity binding. A tgOligo (e.g., a ssDNA molecule commentary to the 3' free 'flap' end) is designed and can serve as a template for integration of desired nucleotide modifications. A tgOligo can be DNA, RNA, or a mix of nucleotides depending on the need and design of the edits. In the case of nucleases (e.g., Cpf1) that provide overhangs from a double-stranded break (DSB) cut, the overhangs can act in place of, or in conjunction with, tgOligos.

### Example 16. Engineering of Cas9-like nuclease. (For reference only)

Nucleases, such as Cas9, can be repurposed for structural and functional genomics in plants. Various dimerization domains can be conjugated to Cas9 to achieve dimerization (Figure 10). For example, inducible dimerization domains from Clonetech's homodimerization or heterodimerization iDimerize system can be used to achieve Cas9 dimerization.. Additional dimerization systems can also be used such as those described in Andersen et al., Scientific Reports 6, Article number: 27766 (2016); and Miyamoto, et al., Nature Chemical Biology 8(5):465-70(2012).

Nucleases, such as Cas9, can also be engineered to form a catalytically deactivated from, such catalytically deactivated Cas9 (dCas9). dCas9 binds to DNA at a target site specified by a gRNA and creates a loop structure accessible for template-based editing (Figure 1). dCas9 can be further modified to form a fusion with a ssDNA binding domain from Affymetrix or NEB for further facilitating template-based editing (Figure 1). The editing efficiency with this modified dCas9-ssDNA binding scheme is expected to be higher compared to a dCas9-alone approach, because a ssDNA template is bound to dCas9 complex and would be brought into proximity of the gRNA target.

### Example 17. Introduction of multiple tgOligo and gRNA molecules. (For reference only)

Multiple approaches can be used to incorporate tgOligos with editing components (e.g., nuclease, gRNA). Essentially, tgOligos can be incorporated in any manner available to deliver nucleases and gRNAs (transfection, transformation, etc.). The optimal approach depends on the editing component delivery system and the target organism to be edited. For example, in mammalian systems where RNPs (ribonucleoproteins - complexes of nuclease and gRNA) can be transfected across the cell membrane, tgOligos can be simultaneously transfected. Alternatively, a single transcription unit (STU) can be used to incorporate the nuclease (Cas9) and gRNAs in the same transgene construct. Similarly, tgOligos can be incorporated in a similar design (Figure 12). Multiple constructs could also be used, such as one for the nuclease, one for the gRNAs, and one for tgOligos - or any combination thereof from inclusive in constructs to combining constructs and transfection delivery. For tgOligos included in constructs (such as Figure 12), these would be RNA-based tgOligos. To utilize DNA-based or mixed nucleotide (DNA+RNA) tgOligos, a transfection or other delivery mechanism would likely be needed. Also if any tgOligo designs result in the tgOligo containing the same gRNA+PAM recognition site as the original gRNA target site, the tgOligo sequence can be modified to eliminate the PAM.

### Example 18. Genome editing based on a two-gRNA approach.

Two nuclease/gRNA complexes flanking a target genomic region are designed for achieving INDELs or complete inversion of the flanked genomic region. (Figure 1). With two nuclease/gRNA complexes, the flanked genomic region is most often deleted and NHEJ repair combining the two cut sites back together. There is also occurrence of INDEL (insertion/deletion) mutations at either nuclease+gRNA flanking site. It is also possible to recover with lower frequency complete inversions of the flanked genomic region.

### Example 19. Enhancement of the two-gRNA approach.

The two-gRNA approach from Example 18 is modified to improve genome editing efficiency. Using dimerization domains (see Figure 10), tgOligos (see Figure 9), or combination thereof can enhance recovery of complete knockout (deletion) of the genomic region flanked by the two gRNA target sites (Figure 13). Panel 1 of Figure 13 shows a dimerization-enhanced knock out (KO) event. Panel 2 of Figure 13 shows a tgOligo-enhanced KO event. Panel 3 of Figure 13 shows an enhanced KO event via a combination of dimerization and tgOligos. Panel 4 of Figure 13 shows a tgOligo-enhanced inversion event. Without being bound to any theory, tgOligos can facilitate recovery of inversion events by using complementarity of the tether portions to the opposite end of the flanked segment. The tgOligos can vary in length for the complementation to the 3' flap of the non-target strand as well as the template tether extension beyond the flap complementation.

Paired dimerization domains coupled with nuclease (eg:Cas9) or dead nuclease (eg:dCas9) (either alone or in conjunction with tgOligos) can also be used to facilitate inversion of flanked sequence target. Panel 5 of Figure 13 shows a dimerization-enhanced inversion event. Panel 6 of Figure 13 shows an inversion event assisted by a combination of nuclease(eg:Cas9) dimerization/deactivation and tgOligos.

### Example 20. Genome editing of corn BR2 gene by a tgOligo-assisted genomic inversion approach. (For reference only)

A tgOligo-assisted inversion approach (as illustrated in Panel 4 of Figure 13) is used to edit the corn BR2 gene to generate a dominant knockout (KO) mutant allele. The rationale for a genome inversion-based dominant KO mutation approach is depicted in Figure 14. In essence, two gRNAs are used. A first gRNA (shown on the left) targets the end of the first exon of BR2; a second gRNA (shown on the right) recognizes the start codon region of the adjacent GRMZM2G491632 gene. Inversion of the genomic segment flanked by these two gRNAs can lead to a BR2 antisense partial transcript. This BR2 antisense transcript is produced via the GRMZM2G491632 promoter activity. Adjusting the relative position of the two gRNAs can achieve a BR2 antisense complete transcript (e.g., moving the first gRNA on the left to target the start codon region of the BR2 gene) or a BR2 antisense transcript under the control of the native BR2 promoter (e.g., moving the second gRNA on the right to target the stop codon region of the BR2 gene).

Reference sequences are listed in SEQ ID NO: 1 for BR2 (NCBI accession AY366085) and SEQ ID NO: 2 for GRMZM2G491632 (from MaizeGDB). GRMZM2G491632 is a gene annotated immediately adjacent to BR2; and these two genes are in reverse orientation of each other. SEQ ID NO: 3 is the gRNA to the sense strand at the proximal end of BR2. SEQ ID NO: 4 is the gRNA to the antisense strand at the proximal end of GRMZM2G491632.

A first RNA tgOligo corresponding to the BR2 gRNA (SEQ ID NO: 3) is designed to complement the sense strand flank gRNA target site, generally about 20 nt long. Optionally, a 20 nt segment upstream of the target site is added. An example of a BR2 RNA tgOligo comprises a DNA-complementary section as set forth in SEQ ID NO: 5 (serving as a DSB 3' flap complement region), which is complementary to SEQ ID NO: 3 with 10 nt included from upstream. Next, a sequence having at least 20 nt starting with the first base of the PAM of the antisense strand gRNA (SEQ ID NO: 4) is selected to give rise to a 50 nt sequence including the PAM (SEQ ID NO: 6, serving as a tether region). Subsequently, the 3' flap complement (SEQ ID NO: 5) is reversed and attached to the end of the tether (SEQ ID NO: 6) to form a complete tgOligo which complements both the sense gRNA and template from antisense gRNA segment for inversion (SEQ ID NO: 7).

A second RNA tgOligo corresponding to the GRMZM2G491632 gRNA (SEQ ID NO: 4) is designed as follows: a) from the reference sequence (SEQ ID NO: 2) reverse complement the antisense strand flank gRNA target site; b) select at least 20 nt starting with the first base of the PAM of the sense strand gRNA (SEQ ID NO: 3) and reverse complement. This example is 50 nt including the PAM (SEQ ID NO: 9); c) attach the 3' flap complement (SEQ ID NO: 8) to the end of the tether (SEQ ID NO: 9) to complete the tgOligo design complementing the sense gRNA and template from antisense gRNA segment for inversion (SEQ ID NO: 10).

A combination of two gRNAs and the first and second tgOligos are used to edit the com BR2 locus to achieve a genomic inversion. The resulting inversion of BR2 and GRMZM2G491632 is expected to form a sequence with high similarity (95%+) to SEQ ID NO: 11.

### Example 21. Enhancement of template-based genome editing or site directed integration (SDI).(For reference only)

Nuclease dimerization or deactivation, tgOligos, or their combination can be used to enhance targeting of template-based editing or site directed integration (SDI) at a single location or multiple locations. Various representative schemes are depicted in Figure 15. In these schemes, a template molecule (regardless of its homology to a target site in the genome) is brought into proximity of the target site by nuclease (Cas9) complexes with dimerization domains (Panels 1 to 3 of Figure 15), tgOligos (not illustrated alone), or a combination thereof (Panel 4 of Figure 15). dCas9 can be used on the template (Panel 1 of Figure 15) or active Cas9 can help facilitate integration of the template (Panels 2 to 4 of Figure 15).

### Example 22. Genome editing of corn Y1 gene to generate dominant alleles. (For reference only)

The enhanced genome editing schemes depicted in Figure 15 are tested in creating a dominant allele for a traditionally recessive trait. The following is a summary of the molecular designs for the com Y1 gene. (SEQ ID NO: 12).

For Y1, the first exon from SEQ ID NO: 12 is shown in SEQ ID NO: 13. To make an antisense template, SEQ ID NO: 13 is reverse complemented into SEQ ID NO: 14 which is used as a template sequence for editing (corresponding to the template sequences between the dCas9 complexes and Cas9 complexes depicted in Figure 15's Panels 1 and 2). The sense strand gRNA for Y1 (SEQ ID NO: 12) is in the 5-UTR (SEQ ID NO: 15). The antisense strand gRNA for Y1 (SEQ ID NO: 12) is in the 3-UTR (SEQ ID NO: 16). The region between these two gRNAs corresponds to the to-be-replaced genomic sequences between the Cas9 complexes depicted in Figure 15's Panels 1, 2, and 4.

To provide a template for integration (as depicted in Figure 15's Panels 1 and 2), SEQ ID NO: 14 is added between the gRNA target sites targeted by (SEQ ID NOs: 15 and 16). The resulting SEQ ID NO: 17 comprises the sense strand gRNA site with 10 nt upstream, SEQ ID NO: 14, and the antisense strand gRNA site with 10 nt downstream.

This template molecule (SEQ ID NO: 17) is then paired with gRNAs (SEQ ID NOs: 15 and 16) and used in editing following the schemes depicted in Figure 15's Panels 1 and 2. To utilize tgOligos to further help facilitate integration of the template (SEQ ID NO: 17) (see Panel 4 of Figure 15), two tgOligos are incorporated (SEQ ID NOs: 18 and 19).

### Example 23. Genome editing of corn BR2 gene to generate dominant alleles. (For reference only)

The enhanced genome editing schemes depicted in Figure 15 are also tested in creating a dominant allele for com BR2 gene (SEQ ID NO: 1). The following is a summary of the molecular designs for BR2.

New gRNAs are designed to be able to replace the BR2 gene with an antisense template similar to the Y1 concept described in Example 20. A sense strand gRNA is provided in SEQ ID NO: 20 and an antisense strand gRNA is provided in SEQ ID NO: 21. The region between these two gRNAs corresponds to the to-be-replaced genomic sequences between the Cas9 complexes depicted in Figure 15's Panels 1, 2, and 4.

The first 250 nt coding sequence of the BR2 gene (SEQ ID NO: 22) is made into an antisense template. SEQ ID NO: 22 is reverse-complemented to create a BR2 Exon 1 antisense sequence template (SEQ ID NO: 23).

To provide a template for integration (as depicted in Figure 15's Panels 1 and 2), SEQ ID NO: 23 is added between the gRNA target sites targeted by gRNAs SEQ ID NOs: 20 and 21. SEQ ID NO: 24 comprises the sense strand gRNA site with 3 nt upstream, SEQ ID NO: 23, and the antisense strand gRNA site with 10 nt downstream.

This template molecule (SEQ ID NO: 23) is then paired with gRNAs (SEQ ID NOs: 20 and 21) and used in editing following the schemes depicted in Figure 15's Panels 1 and 2. To utilize tgOligos to further help facilitate integration of the template (SEQ ID NO: 24) (see Panel 4 of Figure 15), two tgOligos are incorporated (SEQ ID NOs: 25 and 26).

The examples shown above for editing Y1 and BR2 corn genes can be followed to design neighboring template edits or integrations as illustrated in Panel 3 of Figure 15. It should also be obvious that while the examples provided for Y1 and BR2 use antisense templates of the first exon of these genes, the template integrations could be more subtle, such as changing nucleotides to alter amino acids in the native proteins, or more complex such as integrating a non-native sequence or gene. This is further illustrated in Figure 16.

A potential advantage to creating antisense templates in the native genomic region of Y1 and BR2 as described above is that the native promoter and gene expression elements are used to regulate the antisense transcript to appropriately achieve gene silencing of a native allele in a heterozygous organism (e.g., in a dominant manner).

### Example 24. Genome editing-based dominant mutant allele via stacking of an inverted Y1 gene head-to-tail. (For reference only)

The tgOligo and nuclease dimerization concepts described in the above examples can also be used to stack an inverted gene head-to-tail next to the native copy. This would result in an antisense transcript to silence the gene expression, and therefore create a dominant mutant allele for a normally recessive trait (e.g., the com Y1 gene, the com BR2 gene, the com GA20 oxidase gene (see Figure 17).

### Example 25. Genome editing to trigger microprotein interference and dominant suppression.(For reference only)

Microproteins are short, single-domain proteins that possess the ability to interfere with larger multi-domain proteins. *See* Figure 6 and Figure 18. Targets of microproteins are often transcriptional regulators that bind to DNA as active homodimers. Microproteins interfere with their targets by forming non-functional heterodimeric complexes that cannot bind to DNA.

Expression of *an Arabidopsis thaliana* microprotein, ATHB17, in maize exhibits a dominant phenotype *(e.g.,* increased ear weight at silking). *See* Rice et al., 2014, PLoS ONE, 9(4):e94238. Without being bound to any theory, an ATHB17 microprotein forms non-functional heterodimers with maize transcriptional repressor proteins. The heterodimers are non-functional thus increase the transcription of maize genes that would be normally be repressed in the absence of the ATHB17 microprotein.

The maize homolog of *ATHB17, Zmhdz18,* is selected as a target for genome editing to create a maize microprotein. *Zmhdz18* is an HD-Zip II member comprising a homeodomain immediately adjacent to a leucine zipper domain, a hypothetical N-terminal repression domain, and a redox sensing motif. Without being bound by a particular theory, a Zmhdz18 microprotein will form non-functional heterodimers with transcriptional repressor proteins to increase expression of typically repressed genes and exhibit a dominant phenotype.

A first and a second functional guide RNA (gRNA) for a CRISPR/ nuclease system are created. The first and second gRNAs are complementary to a first target site and a second target site, respectively, flanking a portion of the *Zmhdz18* gene in the com genome. The flanked portion encodes the amino terminus of the normal Zmhdz18 protein.

A transfer DNA (T-DNA) vector suitable for use in *Agrobacterium* transformation is constructed. The T-DNA construct comprises comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising a promoter operable in a plant cell operably linked to polynucleotides encoding the two gRNAs described above.

Immature com embryos are co-cultured with *Agrobacterium* containing the T-DNA vector for three days. The polynucleotides between the LB and RB sequences are integrated into the nuclear genome of the immature com embryos. Upon expression of the integrated polynucleotides, the gRNAs guide the nuclease to the target sites in the *Zmhdz18* gene, where the nuclease creates a double-stranded break at each target site.

In the majority of events, the region between the first and second target sites in the *Zmhdz18* gene is deleted and non-homologous end-joining repair mechanisms joins the flanking regions. Suitable methods known in the art (e.g., PCR, DNA hybridization (Southern) blots, sequencing) are used to identify transformation events comprising a deletion between the first and second target sites in the *Zmhdz18* gene. Transformed embryos comprising the deletion are selected and used to regenerate modified plants using techniques standard in the field.

Without being bound to any scientific theory, the deletion creates a dominant *Zmhdz18* allele that encodes a Zmhdz18 microprotein. This microprotein can form homodimers and heterodimers with other proteins to interfere with the transcription of maize genes that results in a dominant phenotype.

RNA and protein are extracted from modified corn plants identified as comprising a deletion in a *Zmhdz18* allele capable of encoding a microprotein. RNA and protein are also extracted from control com plants that lack a deletion in *Zmhdz18.* Suitable methods known in the art (*e.g*., quantitative reverse-transcriptase PCR, reverse-transcriptase PCR, RNA sequencing) are used to confirm that expression of maize genes in pathways downstream of *Zmhdz18* are altered. Additional suitable methods *(e.g.,* Western blot, HPLC, LC/MS, ELISA, immunoprecipitation) are used to confirm that the deletion in *Zmhdz18* generates a microprotein.

### Example 26: Generating a dominant positive allele of MIR1 via targeted genomic deletion to render MIR1 gene under the control of an upstream promoter. (For reference only)

The GRMZM2G150276 gene (SEQ ID NO: 99) encoding the MIR1 protein (SEQ ID NO: 100) is located on com chromosome 6. MIR1 is predicted to be involved in insect resistance and encodes a cysteine protease that accumulates in the whorls in response to larval feeding in maize genotypes resistant to some lepidopteran pests *(see* Pechan et al., Plant Cell., 2000 (7) :1031-40). The MIR1 gene is adjacent to the gene GRMZM2G150302 and expressed in the same orientation. See Figure 19, Panel A. The GRMZM2G150302 gene is a member of a large, redundant gene family of xylan synthases. The GRMZM2G150302 promoter (SEQ ID NO: 101) is hypothesized to have a broader/expanded expression profile when compared to the native MIR1 gene expression, which is restricted to the whorls.

Two functional guide RNAs (gRNAs) for an RNA guided nuclease system are created to target the genomic DNA region between the MIR1 gene and the GRMZM2G150302 gene. The first gRNA targets an area near the transcriptional start site of the MIR1 gene and the second gRNA targets a region near the transcriptional start site of the GRMZM2G150302 gene. Each of the two target sites are unique within the corn genome. A transfer DNA (T-DNA) vector suitable for use in Agrobacterium transformation is used. The T-DNA construct comprises several expression cassettes between a left border (LB) sequence and a right border (RB) sequence. The first expression cassette comprises a promoter that is operable in a plant cell operably linked to a polynucleotide encoding an RNA guided nuclease. A second expression cassette comprises a promoter that is operable in a plant cell operably linked to the CP4-EPSPS marker gene. The construct also comprises expression cassettes comprising promoters operable in a plant cell operably linked to polynucleotides encoding the two gRNAs described above.

Immature com embryos are co-cultured with Agrobacterium containing the T-DNA vector for three days. The polynucleotides between the LB and RB sequences are integrated into the nuclear genome of the immature com embryos. Upon expression of the integrated polynucleotides, the gRNAs guide the nuclease to each of the two target sites in the genomic DNA region between the MIR1 gene and the GRMZM2G150302 gene, where the nuclease creates a double-stranded break at each target site.

In the majority of events, the region between the target sites is deleted and non-homologous end-joining repair mechanisms joins the flanking regions. Suitable methods known in the art *(e.g.,* PCR, DNA hybridization (Southern) blots, sequencing) are used to identify transformation events comprising a complete deletion. Transformed embryos comprising a targeted deletion between the MIR1 gene and GRMZM2G150302 gene are selected and used to regenerate modified plants using techniques standard in the field.

Without being bound by a particular theory, by removing the genomic DNA between the MIR1 gene and the GRMZM2G150302 gene, the native GRMZM2G150302 promoter (SEQ ID NO: 101) can drive the transcription of the MIR1 gene (SEQ ID NO: 99) thereby broadening/expanding the expression profile of the MIR1 gene. See Fig 19, panel B.

RNA is extracted from various tissue types (e.g., roots, stem, leaf, inflorescence) from modified corn plants identified as comprising a targeted deletion between the MIR1 gene and the GRMZM2G150302 gene. RNA is also extracted from control corn plants that lack the deletion. Suitable methods known in the art (e.g., quantitative reverse-transcriptase PCR, reverse-transcriptase PCR, RNA sequencing) are used to confirm that the MIR1 gene is more broadly expressed and/or more strongly expressed in com modified plants comprising a deletion of the targeted region.

### Example 27. Constructs for creation of dominant negative deletion mutant alleles

The endogenous Zm.GA20ox5 gene is separated from an endogenous Zm.SAMT gene in the maize genome by an intergenic region of about 550 bp, or by 1170 bp if measured between stop codons, with the Zm.SAMT gene positioned downstream and oriented in the opposite orientation relative to the Zm.GA20ox5 gene. The sequence of the genomic locus or region encompassing the Zm.GA20ox5 and Zm.SAMT genes is provided in SEQ ID NOs. 35 and 36. SEQ ID NO. 35 represents a sequence of the GA20ox5-SAMT genomic locus corresponding to the sense strand of the Zm.GA20ox5 gene and encompassing both Zm.GA20ox5 and Zm.SAMT genes (the "GA20ox5_SAMT genomic sequence" in Table 2). SEQ ID NO. 36 represents a sequence of the GA20ox5-SAMT genomic locus corresponding to the sense strand of the Zm.SAMT gene (the antisense strand of the Zm.GA20ox5 gene) and encompassing both Zm.GA20ox5 and Zm.SAMT genes (the "SAMT_GA20ox5 genomic sequence" in Table 2). The elements or regions of the genomic sequences encompassing both Zm.GA20ox5 and Zm.SAMT genes are annotated in Table 2 below by reference to the nucleotide coordinates of those elements or regions in each of SEQ ID NOs. 35 and 36. As proposed herein, if a genomic region between the neighboring Zm.GA20ox5 and Zm.SAMT genes (including possibly all or part of those genes) were deleted, then the endogenous Zm.SAMT gene promoter may drive expression of an antisense RNA transcript through all or part of the Zm.GA20ox5 gene that can hybridize to a separate RNA transcript expressed from one or both of the copies or alleles of the Zm.GA20ox5 and/or Zm.GA20ox3 gene(s). Since the Zm.GA20ox3 and Zm.GA20ox5 genes share a high level of nucleotide sequence similarity in their respective exon coding regions, the antisense RNA transcript expressed from the oppositely oriented Zm.SAMT gene promoter may hybridize to transcripts of both GA20 oxidase genes and cause the suppression or silencing of one or both of the Zm.GA20ox3 and/or Zm.GA20ox5 gene(s). Thus, a mutant allele having a deletion between the Zm.GA20ox5 and Zm.SAMT genes may behave as a dominant or semi-dominant negative mutation or allele by causing suppression or silencing of one or both (wild-type and/or mutant) copies or alleles of the endogenous Zm.GA20ox5 gene, in addition to possible further suppression or silencing of one or both copies or alleles of the endogenous Zm.GA20ox3 gene.

**Table 2. Annotation of genomic sequence elements of Zm.GA20ox5 and Zm.SAMT genomic region**

| **Gene Name or Region** | **Element / Feature** | **Location in the GA20ox5_SAMT genomic sequence (SEQ ID NO: 35)** | **Location in the SAMT _GA20ox5 genomic sequence (SEQ ID NO: 36)** |
|---|---|---|---|
| GA20ox5 | Promoter and 5' UTR | 1..398 | 8670..9067 |
| GA20ox5 | Exon 1 | 399..1189 | 7879..8669 |
| GA20ox5 | Intron 1 | 1190..1304 | 7764..7878 |
| GA20ox5 | Exon 2 | 1305..1629 | 7439..7763 |
| GA20ox5 | Intron 2 | 1630..2595 | 6473..7438 |
| GA20ox5 | Exon 3 | 2596..2871 | 6197..6472 |
| GA20ox5 | 3' UTR | 2872..3180 | 5888..6196 |
| ***Intergenic Region*** | | **3181..3736** | **5332..5887** |
| SAMT | 3' UTR | 3737..4141 | 4927..5331 |
| SAMT | Exon 8 | 4042..4258 | 4810..5026 |
| SAMT | Intron 8 | 4259..4512 | 4556..4809 |
| SAMT | Exon 7 | 4513..4707 | 4361..4555 |
| SAMT | Intron 7 | 4708..4989 | 4079..4360 |
| SAMT | Exon 6 | 4990..5262 | 3806..4078 |
| SAMT | Intron 6 | 5263..5348 | 3720..3805 |
| SAMT | Exon 5 | 5349..5523 | 3545..3719 |
| SAMT | Intron 5 | 5524..6037 | 3031..3544 |
| SAMT | Exon 4 | 6038..6148 | 2920..3030 |
| SAMT | Intron 4 | 6129..6239 | 2829..2939 |
| SAMT | Exon 3 | 6240..6510 | 2558..2828 |
| SAMT | Intron 3 | 6511..6894 | 2174..2557 |
| SAMT | Exon 2 | 6895..7044 | 2024..2173 |
| SAMT | Intron 2 | 7045..7139 | 1929..2023 |
| SAMT | Exon 1 | 7140..8126 | 942..1928 |
| SAMT | 5' UTR 2 | 8127..8268 | 800..941 |
| SAMT | Intron 1 | 8269..8771 | 297..799 |
| SAMT | 5' UTR 1 | 8772..8942 | 126..296 |
| SAMT | Promoter | 8943..9067 | 1..125 |

FIG. 20 illustrates the concept for creating an antisense RNA molecule that targets the Zm.GA20ox5 gene by deleting a genomic region between the Zm.GA20ox5 and its neighboring Zm.SAMT gene oriented in the opposite direction, through genome editing. The deletion can be generated using two or more guide RNAs that create double stranded breaks in the genome at the two ends of the intended deletion. The antisense RNA molecule generated from the oppositely oriented Zm.SAMT gene promoter can then hybridize to a sense Zm.GA20ox5 RNA transcript and trigger suppression or silencing of one or both copies or alleles (wild-type or mutant) of the endogenous Zm.GA20ox5 gene. FIG. 20 provides an embodiment where small RNAs may be generated through RNA interference. However, it is envisioned that suppression or silencing of the Zm.GA20ox5 gene may occur through other mechanisms as provided herein, alternatively or in addition to any RNAi or PTGS forms of suppression. Given that the Zm.GA20ox3 and Zm.GA20ox5 genes share a high level of nucleotide sequence similarity in their respective coding regions, the antisense RNA transcript may also hybridize to RNA transcripts of the Zm.GA20ox3 gene and cause the suppression or silencing of one or both of the Zm.GA20ox3 and/or Zm.GA20ox5 gene(s). Thus, a deletion between the Zm.GA20ox5 and Zm.SAMT genes may act as a dominant or semi-dominant negative mutation or allele for one or both of the Zm.GA20ox3 and/or Zm.GA20ox5 gene(s).

In the illustrative example provided in FIG. 20, a pair of guide RNAs are used including one guide RNA having a targeting or spacer sequence designed to target a site in the Zm.GA20ox5 gene, and another guide RNA having a targeting or spacer sequence designed to target a site in the Zm.SAMT gene. The size of the deletion and the location of the two breakpoints at the ends of the deletions may be determined by selecting which guide RNAs are used with a RNA-guided endonuclease to create the genome breaks. By creating a double strand break at both target sites, a deletion of the intervening region can be generated that will condense the genomic locus and bring the oppositely oriented Zm.SAMT gene promoter into closer proximity to the GA20ox5 gene, such that the Zm.SAMT gene promoter can drive the expression of an antisense RNA transcript that reads through at least a portion of the GA20ox5 gene. Even though a 3' portion of the GA20ox5 gene may be deleted, the remaining 5' portion of the GA20ox5 gene can be sufficient for an antisense RNA transcript or molecule to be generated under the control of the Zm.SAMT gene promoter that causes suppression or silencing of the Zm.GA20ox3 and/or GA20ox5 gene(s). Thus, the presence of a single copy or allele of the deletion mutant may act in a dominant or semi-dominant negative manner to cause a corn plant to have a short stature, lodging resistant phenotype.

Deletions in the Zm.GA20ox5 / Zm.SAMT genomic region were generated using three different plasmid vector constructs for transformation. Each vector construct comprises a functional cassette for the expression of Cpfl (or Cas12a), and further comprises one or two functional cassettes for the expression of guide RNAs, in addition to a selectable marker gene and plasmid maintenance elements. For the Vector-1 and Vector-2 constructs, the Cpfl (or Cas12a) expression cassette comprises a maize ubiquitin promoter (SEQ ID NO: 37) operably linked to a sequence encoding a wild-type Lachnospiraceae bacterium Cpfl RNA-guided endonuclease enzyme (SEQ ID NO: 38) fused to two nuclear localization signals (SEQ ID NOs: 40 and 41). The wild-type Cpfl expression cassette further contains a synthetic sequence (atggcg) which provides a start codon. For the Vector-3 construct, the Cpfl (or Cas12a) expression cassette comprises a maize ubiquitin promoter (SEQ ID NO: 37) operably linked to a sequence encoding a *Lachnospiraceae bacterium* G532R/K595R mutant Cpfl RNA-guided endonuclease enzyme (SEQ ID NO: 39) fused to two nuclear localization signals (SEQ ID NOs: 42 and 43). *See, e.g.,* Gao, L. et al., Nature Biotechnol. 35(8): 789-792 (2017).

Table 3 below provides the target site, spacer and targeting/spacer sequence for each guide RNA encoded by the guide RNA cassette(s) in each vector construct. Each guide RNA unit within the guide RNA cassettes comprises a guide RNA scaffold sequence compatible with the LbCpf1 enzyme along with the unique spacer or targeting sequence complementary to its intended target site. For the Vector-2 construct, the guide RNA expression cassette comprises a maize RNA polymerase III (Pol3) promoter (SEQ ID NO: 44) operably linked to a sequence encoding two guide RNAs having targeting/spacer sequences encoded by the SP1b and SP1f DNA sequences in Table 3 below, with one guide RNA (SP1b) targeting a site in the first exon of the Zm.SAMT gene, and the other guide RNA (SP1f) targeting a site in the first intron of the Zm.GA20ox5 gene (see also FIG. 21 (top panel) showing the placement of the two guide RNA target sites for SP1b and SP1f (SAMT_408 and GA20ox5_6531) relative to the genomic region encompassing the endogenous Zm.GA20ox5 and Zm.SAMT genes).

The Vector-1 construct has two guide RNA expression cassettes. One guide RNA expression cassette of the Vector-1 construct comprises a maize Pol3 promoter (SEQ ID NO: 44) operably linked to a sequence encoding two guide RNAs having targeting/spacer sequences encoded by the SP2f1 and SP2f2 DNA sequences in Table 3 below, with one guide RNA (SP2fl) targeting a site in the first exon of the Zm.GA20ox5 gene, and the other guide RNA (SP2f2) targeting a site in the second exon of the Zm.GA20ox5 gene. The other guide RNA expression cassette of the Vector-1 construct comprises a synthetic promoter operably linked to a sequence encoding two guide RNAs having targeting/spacer sequences encoded by the SP2b1 and SP2b2 DNA sequences in Table 3 below, with each guide RNA (SP2b1 and SP2b2) targeting different sites in the first exon of the Zm.SAMT gene. For the Vector-1 construct, see also the middle panel of FIG. 21 showing the placement of the four guide RNA target sites for SP2f1, SP2f1, SP2b1 and SP2b2 (GA20ox5_7090, GA20ox5_1654, SAMT_304 and SAMT_161) relative to the genomic region encompassing the endogenous Zm.GA20ox5 and Zm.SAMT genes.

The Vector-3 construct has two guide RNA expression cassettes. One guide RNA expression cassette of the Vector-3 construct comprises a maize Pol3 promoter (SEQ ID NO: 74) operably linked to a sequence encoding two guide RNAs having targeting/spacer sequences encoded by the SP3f1 and SP3f2 DNA sequences in Table 3 below, with each guide RNA (SP3f1 and SP3f2) targeting different sites in the second intron of the Zm.GA20ox5 gene. The other guide RNA expression cassette of the Vector-1 construct comprises a synthetic promoter operably linked to a sequence encoding two guide RNAs having targeting/spacer sequences encoded by the SP3b1 and SP3b2 DNA sequences in Table 3 below, with one guide RNA (SP3b1) targeting a site in the first exon of the Zm.SAMT gene, and another guide RNA (SP3b2) targeting a site in the 5' UTR of the Zm.SAMT gene. For the Vector-3 construct, see also the lower panel of FIG. 21 showing the placement of the four guide RNA target sites for SP3f1, SP3f1, SP3b1 and SP3b2 (GA20ox5_1695_TYC, GA20ox5_1732_TYC, SAMT_8110_TYC and SAMT_8165_TYC) relative to the genomic region encompassing the endogenous Zm.GA20ox5 and Zm.SAMT genes.

**Table 3. Transgenic constructs and their respective target sites and guide RNA spacers**

| **Vector Construct** | **guide RNA Spacer ID** | **Target Site** | **Spacer Sequence** | **SEQ ID NO** |
|---|---|---|---|---|
| **Vector-2** | SP1b | SAMT_408 | AGGACACCGACAACAATGATGCC | 45 |
| | SP1f | GA20ox5_6531 | GGTCCACTAGGATTCGGGAAATA | 46 |
| | | | | |
| **Vector-1** | SP2f1 | GA20ox5 7090 | GAGCCAATGGGGTAAGTAAGGTA | 47 |
| | SP2f2 | GA20ox5_1654 | GTTACCATGAAGGTGTCGCCGAT | 48 |
| | SP2b1 | SAMT_304 | GTCCAATAAGAAGCCGGTGGTGA | 49 |
| | SP2b2 | SAMT_161 | CACCTCGGCCAAATGCCATCAGT | 50 |
| | | | | |
| **Vector-3** | SP3f2 | GA20ox5 1695 TYC | GTTGAGCTCTCTCTGTGCTGTTA | 51 |
| | SP3f1 | GA20ox5 1732 TYC | CTAGGATTCGGGAAATAACAGCA | 52 |
| | SP3b1 | SAMT_8110 TYC | CCTCGGCCAAATGCCATCAGTGC | 53 |
| | SP3b2 | SAMT 8165 TYC | CGTGGTTTATCTCCACCAACAAC | 54 |

### Example 28. Characterization of Deletion Mutant Alleles of GA20Ox5 gene

An inbred com plant line was transformed via *Agrobacterium-mediated* transformation with a transformation vector having one of the constructs as described above in Example 27. The transformed plant tissue was grown to mature R0 plants. R0 plants having one or more unique genome edit(s) were selfed to produce R1 plants. To characterize the edits and recover plants with a deletion between the GA20Ox5 and SAMT genes, a PCR-based assay was performed using a pair of PCR primers flanking the intended deletion region. The same pair of primers (SEQ ID NOs: 55 and 56) were used for all three vectors in Table 3. If a deletion is present between the GA20Ox5 and SAMT genes, the PCR assay would result in an amplicon that could be sequenced. However, due to the large size of the intended deletion, the PCR assay would not produce a PCR product in the absence of a larger deletion. For each PCR assay, a 15 µL PCR reaction volume was used containing the Phusion PCR master mix from Thermo Fisher Scientific, 3 µL of genomic DNA template, and two PCR primers. After PCR amplification, a 3 µL PCR mixture was added to 21 µL of Tris-EDTA buffer and then analyzed on a ZAG instrument for the presence or absence of PCR products that indicate a GA20Ox5-SAMT deletion. The PCR products were sequenced to determine the junction sequence generated in each deletion around the GA20ox5-SAMT genomic locus (see Table 4).

R0 plants with a deletion between the GA20ox5 and SAMT genes were selected and selfed to produce R1 plants. The R1 plants were subject to a quantitative PCR assay to determine the zygosity of the GA20ox5-SAMT genomic locus (see Table 5). Each R1 plant was sequenced to determine all of the deletion edits around the GA20Ox5-SAMT genomic locus. Due to multiple gRNAs with a given construct, multiple deletions may occur on the same chromosome of a R0 plant and thus be present in a R1 plant, which may be homozygous or heterozygous for a mutant allele comprising the genomic deletion(s) (see Table 5). In Table 5, "homo" means homozygous for the mutant allele, and "hetero" means heterozygous for the mutant allele.

**Table 4. Individual deletion junction sequences for edits made using the vectors in Table 3.**

| **SEQ ID NO.** | **Deletion Junction Number** | **Junction Sequence (with deletion size shown in the parentheses)** | **Junction Sequence Description** |
|---|---|---|---|
| 57 | 1001 | GCGGCCGTCCATCTTTCCACCTCGGCCAAA- (-8)- GTGCCTGGCGAACATGTACCAGAGCACCAG | 8 nt deletion at SAMT_161 |
| 58 | 1002 | GGCCGTCCATCTTTCCACCTCGGCCAAATG- (-3)- TCAGTGCCTGGCGAACATGTACCAGAGCAC | 3 nt deletion at SAMT_161 |
| 59 | 1003 | GGCCGTCCATCTTTCCACCTCGGCCAAATG- (-6)- GTGCCTGGCGAACATGTACCAGAGCACCAG | 6 nt deletion at SAMT_161 |
| 60 | 1004 | GAGTGGCGCCCCGTCCGGCCCGTCCCGGGC- (-6357)- TTCTTATTGGACGAAATCTCCAGCGGGAAG | 6357 nt deletion between GA20ox5_1654 and SAMT_304 |
| 61 | 1005 | CCGGCCCGTCCCGGGCGCCATGGTCATCAA- (-6518)- GTGCCTGGCGAACATGTACCAGAGCACCAG | 6518 nt deletion between GA20ox5_1654 and SAMT_161 |
| 62 | 1006 | GTCCGGCCCGTCCCGGGCGCCATGGTCATC- (-6342)- GGCTTCTTATTGGACGAAATCTCCAGCGGG | 6342 nt deletion between GA20ox5_1654 and SAMT_304 |
| 63 | 1007 | GTCCGGCCCGTCCCGGGCGCCATGGTCATC- (-6348)- TTATTGGACGAAATCTCCAGCGGGAAGACA | 6348 nt deletion between GA20ox5_1654 and SAMT_304 |
| 64 | 1008 | CGTCCGGCCCGTCCCGGGCGCCATGGTCAT- | 6344 nt deletion between |
| | | (-6344)- GCTTCTTATTGGACGAAATCTCCAGCGGGA | GA20ox5_1654 and SAMT_304 |
| 65 | 1009 | CTGTGTGTATATTCAGTTGAGCTCTCTCTG-(- 6478)- CACGGCTGGACCAACAGCCCCCCCAAAATC | 6478 nt deletion between GA20ox5_1695 and SAMT 8165 |
| 66 | 1010 | CTTGGCCGCTCTTGTCCTGTGTGTATATTC-(- 6160)- GGTGTCCTCAAATTTCTCGGACCCTTCACC | 6160 nt deletion between GA20ox5_6531 and SAMT_408 |
| 67 | 1011 | TGTATATTCAGTTGAGCTCTCTCTGTGCTG-(- 6133)- GTTGTCGGTGTCCTCAAATTTCTCGGACCC | 6133 nt deletion between GA20ox5_6531 and SAMT_408 |
| 68 | 1012 | TATATTCAGTTGAGCTCTCTGTGCTGTT-(- 6130)- TGTTGTCGGTGTCCTCAAATTTCTCGGACC | 6130 nt deletion between GA20ox5_6531 and SAMT_408 |
| 69 | 1013 | ATATTCAGTTGAGCTCTCTGTGCTGTTA-(- 6130)- GTTGTCGGTGTCCTCAAATTTCTCGGACCC | 6130 nt deletion between GA20ox5_6531 and SAMT_408 |
| 70 | 1014 | ATTCAGTTGAGCTCTCTCTGTGCTGTTATT-(- 6131)- GTCGGTGTCCTCAAATTTCTCGGACCCTTC | 6131 nt deletion between GA20ox5_6531 and SAMT_408 |
| 71 | 1015 | CTCGGCCAGGATTTCGAGCCAATGGGGTAA- (-6759)- CTTCTTATTGGACGAAATCTCCAGCGGGAA | 6759 nt deletion between GA20ox5_7090 and SAMT_304 |
| 72 | 1016 | CGGCCAGGATTTCGAGCCAATGGGGTAAGT- (-6753)- CCGGCTTCTTATTGGACGAAATCTCCAGCG | 6753 nt deletion between GA20ox5_7090 and SAMT_304 |
| 73 | 1017 | TCGGCCAGGATTTCGAGCCAATGGGGTAAG- (-12)- AAGGAGCGCCGGTTTACATTTACCGCACGT | 12 nt deletion at GA20ox5_7090 |
| 74 | 1018 | TCGGCCAGGATTTCGAGCCAATGGGGTAAG- (-4)- GTAGTAAGAAGGAGCGCCGGTTTACATTTA | 4 nt deletion at GA20ox5_7090 |
| 75 | 1019 | GGACTACTTCGTCGGCACCCTCGGCCAGGA- (-39)- GCCGGTTTACATTTACCGCACGTCGGCGTG | 39 nt deletion at GA20ox5_7090 |

**Table 5. Deletion edits and genotype of R0 ad R1 plants.**

| **R0 Edit ID** | **R1 Plant ID** | **Editing Deletion Type** | **R1 zygosity call for deletion mutant** | **Deletion Junction Number(s) (Table 4)** |
|---|---|---|---|---|
| E221089 | P43596818 | 6759 nt deletion between GA20ox5_7090 and SAMT_304; 6 nt deletion at T161 | Homozygous | 1015; 1003 |
| E221089 | P43596820 | 6759 nt deletion between GA20ox5_7090 and SAMT_304; 6 nt deletion at T161 | Homozygous | 1015; 1003 |
| E221089 | P43596823 | 6759 nt deletion between GA20ox5_7090 and SAMT_304; 6 nt deletion at T161 | Homozygous | 1015; 1003 |
| E221089 | P43596801 | 6759 nt deletion between GA20ox5_7090 and SAMT_304; 6 nt deletion at T161 | Homozygous | 1015; 1003 |
| E221089 | P43596831 | 6759 nt deletion between GA20ox5_7090 and SAMT_304; 6 nt deletion at T161 | Homozygous | 1015; 1003 |
| E220938 | P43596469 | 6753 nt deletion between GA20ox5_7090 and SAMT_304; 8 nt deletion at T161 | Homozygous | 1016; 1001 |
| E220938 | P43596438 | 6753 nt deletion between GA20ox5_7090 and SAMT_304; 8 nt deletion at T161 | Homozygous | 1016; 1001 |
| E220938 | P43596489 | 6753 nt deletion between GA20ox5_7090 and SAMT_304; 8 nt deletion at T161 | Homozygous | 1016; 1001 |
| E220242 | P95046375 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046377 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046392 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046378 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046370 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046369 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046368 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046395 | 6344 nt deletion between GA20ox5_1654 and SAMT_304; 12 nt deletion at GA20ox5_7090; 3 nt deletion at SAMT_161 | Homozygous | 1008; 1017; 1002 |
| E220242 | P95046396 | 6344 nt deletion between GA20ox5_1654 and SAMT_304; 12 nt deletion at GA20ox5_7090; 3 nt deletion at SAMT_161 | Homozygous | 1008; 1017; 1002 |
| E220698 | P43596662 | 6518 nt deletion between GA20ox5_1654 and SAMT_161; 4 nt deletion at T7090 | Homozygous | 1005; 1018 |
| E220698 | P43596671 | 6518 nt deletion between GA20ox5_1654 and SAMT_161; 4 nt deletion at T7090 | Heterozygous | 1005; 1018 |
| E220698 | P43596694 | 6518 nt deletion between GA20ox5_1654 and SAMT_161; 4 nt deletion at T7090 | Homozygous | 1005; 1018 |
| E220698 | P43596679 | 6518 nt deletion between GA20ox5_1654 and SAMT_161; 4 nt deletion at T7090 | Homozygous | 1005; 1018 |
| E220698 | P43596701 | 6518 nt deletion between GA20ox5_1654 and SAMT_161; 4 nt deletion at T7090 | Heterozygous | 1005; 1018 |
| E220698 | P43596654 | 6518 nt deletion between GA20ox5_1654 and SAMT_161; 4 nt deletion at T7090 | Homozygous | 1005; 1018 |
| E220698 | P43596690 | 6518 nt deletion between GA20ox5_1654 and SAMT_161; 4 nt deletion at T7090 | Heterozygous | 1005; 1018 |
| E220698 | P43596703 | 6518 nt deletion between GA20ox5_1654 and SAMT_161; 4 nt deletion at T7090 | Homozygous | 1005; 1018 |
| E220698 | P43596711 | 6518 nt deletion between GA20ox5_1654 and SAMT_161; 4 nt deletion at T7090 | Homozygous | 1005; 1018 |
| E220055 | P95046321 | 6348 nt deletion between GA20ox5_1654 and SAMT_304; 39 nt deletion at T7090 | Homozygous | 1007; 1019 |
| E220055 | P95046342 | 6348 nt deletion between GA20ox5_1654 and SAMT_304; 39 nt deletion at T7090 | Homozygous | 1007; 1019 |
| E220055 | P95046314 | 6348 nt deletion between GA20ox5_1654 and SAMT_304; 39 nt deletion at T7090 | Homozygous | 1007; 1019 |
| E220055 | P95046297 | 6348 nt deletion between GA20ox5_1654 and SAMT_304; 39 nt deletion at T7090 | Homozygous | 1007; 1019 |
| E220228 | P43596770 | 6357 nt deletion between GA20ox5_1654 and SAMT_304 | Homozygous | 1004 |
| E220141 | P43596991 | 6342 nt deletion between GA20ox5_1654 and SAMT_304 | Homozygous | 1006 |
| E220141 | P43597019 | 6342 nt deletion between GA20ox5_1654 and SAMT_304 | Homozygous | 1006 |
| E220141 | P43596954 | 6342 nt deletion between GA20ox5_1654 and SAMT_304 | Homozygous | 1006 |
| E220141 | P43596970 | 6342 nt deletion between GA20ox5_1654 and SAMT_304 | Homozygous | 1006 |
| E220141 | P43596980 | 6342 nt deletion between GA20ox5_1654 and SAMT_304 | Homozygous | 1006 |
| E187994 | P43597077 | 6160 nt deletion between GA20ox5_6531 and SAMT_408 | Homozygous | 1010 |
| E187994 | P43597052 | 6160 nt deletion between GA20ox5_6531 and SAMT_408 | Heterozygous | 1010 |
| E187994 | P43597049 | 6160 nt deletion between GA20ox5_6531 and SAMT_408 | Heterozygous | 1010 |
| E187994 | P43597037 | 6160 nt deletion between GA20ox5_6531 and SAMT_408 | Homozygous | 1010 |
| E188579 | P43596586 | 6133 nt deletion between GA20ox5_6531 and SAMT_408 | Heterozygous | 1011 |
| E188579 | P43596582 | 6133 nt deletion between GA20ox5_6531 and SAMT_408 | Heterozygous | 1011 |
| E188579 | P43596603 | 6133 nt deletion between GA20ox5_6531 and SAMT_408 | Heterozygous | 1011 |
| E188579 | P43596594 | 6133 nt deletion between GA20ox5_6531 and SAMT_408 | Homozygous | 1011 |
| E188790 | P09617231 | 6130 nt deletion between GA20ox5_6531 and SAMT_408 | Homozygous | 1012 |
| E188790 | P09617182 | 6130 nt deletion between GA20ox5_6531 and SAMT_408 | Heterozygous | 1012 |
| E188790 | P09617144 | 6130 nt deletion between GA20ox5_6531 and SAMT_408 | Heterozygous | 1012 |
| E188790 | P09617191 | 6130 nt deletion between GA20ox5_6531 and SAMT_408 | Heterozygous | 1012 |
| E188790 | P09617225 | 6130 nt deletion between GA20ox5_6531 and SAMT_408 | Homozygous | 1012 |
| E188790 | P09617216 | 6130 nt deletion between GA20ox5_6531 and SAMT_408 | Homozygous | 1012 |
| E188790 | P09617192 | 6130 nt deletion between GA20ox5_6531 and SAMT_408 | Homozygous | 1012 |
| E188790 | P09617208 | 6130 nt deletion between GA20ox5_6531 and SAMT_408 | Homozygous | 1012 |
| E188569 | P43596926 | 6130 nt deletion between GA20ox5_6531 and SAMT_408 | Homozygous | 1013 |
| E188569 | P43596908 | 6130 nt deletion between GA20ox5_6531 and SAMT_408 | Homozygous | 1013 |
| E188569 | P43596931 | 6130 nt deletion between GA20ox5_6531 and SAMT_408 | Homozygous | 1013 |
| E188569 | P43596895 | 6130 nt deletion between GA20ox5_6531 and SAMT_408 | Heterozygous | 1013 |
| E188569 | P43596896 | 6130 nt deletion between GA20ox5_6531 and SAMT_408 | Heterozygous | 1013 |
| E188569 | P43596911 | 6130 nt deletion between GA20ox5_6531 and SAMT_408 | Heterozygous | 1013 |
| E189115 | P43596944 | 6131 nt deletion between GA20ox5_6531 and SAMT_408 | Homozygous | 1014 |
| E180294 | P43596566 | 6478 nt deletion between GA20ox5_1695 and SAMT_8165 | Homozygous | 1009 |
| E180294 | P43596550 | 6478 nt deletion between GA20ox5_1695 and SAMT_8165 | Homozygous | 1009 |
| E180294 | P43596542 | 6478 nt deletion between GA20ox5_1695 and SAMT_8165 | Homozygous | 1009 |
| E180294 | P43596530 | 6478 nt deletion between GA20ox5_1695 and SAMT_8165 | Homozygous | 1009 |
| E180294 | P43596524 | 6478 nt deletion between GA20ox5_1695 and SAMT_8165 | Homozygous | 1009 |
| E180294 | P43596534 | 6478 nt deletion between GA20ox5_1695 and SAMT_8165 | Homozygous | 1009 |
| E180294 | P43596558 | 6478 nt deletion between GA20ox5_1695 and SAMT_8165 | Homozygous | 1009 |
| E180294 | P43596538 | 6478 nt deletion between GA20ox5_1695 and SAMT_8165 | Homozygous | 1009 |

### Example 29. Reduced plant height of corn plants with edited allele.

R1 corn plants homozygous or heterozygous for an edited allele of the GA20 oxidase 5 gene (as identified in Example 28) were grown to maturity to measure their plant heights along with wild type control plants. R1 seeds were planted in soil and grown to maturity in the greenhouse under day/night temperatures of 85°/70° and 16/8 hours of photoperiod using standard nutrient and light conditions for com plant growth and development. Plant heights (PHT) of R1 plants were measured at R2 growth stage from the soil level to the base of the uppermost fully expanded leaf.

Table 6 provides the plant heights of individual R1 plants homozygous for deletion edits between the GA20ox5 and SAMT genes made using the Vector-2 or Vector-1 construct described in Example 27, along with wild type (WT) control plants. Average plant heights for WT and each homozygous deletion edit are also provided in Table 6 (see also FIG. 22 showing the average plant heights with error bars). These plant heights demonstrate that plants homozygous for an edited GA20 oxidase 5 allele comprising a deletion between the GA20ox5 and SAMT genes have significantly reduced plant heights averaging between 57.3 inches and 70.1 inches for plants having the edited alleles, versus an average plant height of 78.5 inches for the WT control.

Table 7 provides the plant heights of individual R1 plants homozygous or heterozygous for deletion edits between the GA20ox5 and SAMT genes made using the Vector-2 construct described in Example 27, along with wild type (WT) control plants (see also FIG. 23 showing average plant heights with error bars). The data in Table 7 overlaps with Table 6 since R1 plants homozygous for the deletion edits made using the Vector-2 construct and the wild type control plants are the same as in Table 6. These plant heights demonstrate that plants heterozygous or homozygous for the edited GA20 oxidase 5 alleles comprising a deletion between the GA20ox5 and SAMT genes and made using the Vector-2 construct have significantly reduced plant heights averaging between 57.3 inches and 64 inches for plants homozygous these edited alleles, and between 60.5 inches and 67 inches for plants heterozygous for these edited alleles, relative to an average plant height of 78.5 inches for the WT control plants. The reductions in plant height are similar between plants homozygous and heterozygous for the deletion edit alleles, but plant heights overall for plants comprising the deletion edit alleles regardless of zygosity are significantly lower than those of wild type control plants.

The plant height data described in this example demonstrate that deletion of the region between GA20ox5 and SAMT genes leads to reduced plant heights as compared to wild type control plants, for plants homozygous or heterozygous for the edited deletion alleles, suggesting that these deletion alleles of the GA20 oxidase 5 gene act in a dominant or semi-dominant manner to produce a reduced plant height phenotype (semi-dwarf or short stature corn plants), especially since edited loss-of-function alleles of the GA20 oxidase 3 or GA20 oxidase 5 genes alone without an antisense or inversion sequence have been shown to not produce short stature corn plants. See, e.g., Published PCT Application Nos. WO/2019/161149, WO/2019/161147 and WO/2019/161144. Further plant height measurements will be made in subsequent generations to confirm the shorter plant height phenotype.

**Table 6. Plant Heights of homozygous R1 plants using Vector-2 and Vector-1.**

| **Editing Construct ID** | **Edit ID** | **R1 Plant ID** | **Plant height (inches)** |
|---|---|---|---|
| Vector-2 | E187994 | P43597037 | 65 |
| Vector-2 | E187994 | P43597077 | 63 |
| | **E187994 Average** | | 64.0 |
| Vector-2 | E188569 | P43596931 | 65 |
| Vector-2 | E188569 | P43596908 | 55.75 |
| Vector-2 | E188569 | P43596926 | 51 |
| | **E188569 Average** | | 57.3 |
| Vector-2 | E188579 | P43596594 | 61 |
| Vector-2 | E188790 | P09617225 | 70 |
| Vector-2 | E188790 | P09617231 | 60.75 |
| Vector-2 | E188790 | P09617208 | 60.25 |
| Vector-2 | E188790 | P09617216 | 59.5 |
| Vector-2 | E188790 | P09617192 | 55 |
| | **E188790 Average** | | 61.1 |
| Vector-2 | E189115 | P43596944 | 58.5 |
| Vector-1 | E220055 | P95046314 | 69.5 |
| Vector-1 | E220055 | P95046342 | 69 |
| Vector-1 | E220055 | P95046321 | 68 |
| Vector-1 | E220055 | P95046297 | 66.25 |
| | **E220055 Average** | | 68.2 |
| Vector-1 | E220141 | P43596991 | 72 |
| Vector-1 | E220141 | P43596954 | 72 |
| Vector-1 | E220141 | P43596970 | 71.5 |
| Vector-1 | E220141 | P43596980 | 68 |
| Vector-1 | E220141 | P43597019 | 67 |
| | **E220141 Average** | | 70.1 |
| Vector-1 | E220228 | P43596770 | 52 |
| Vector-1 | E220242 | P95046370 | 74 |
| Vector-1 | E220242 | P95046369 | 71.5 |
| Vector-1 | E220242 | P95046392 | 69.5 |
| Vector-1 | E220242 | P95046395 | 69 |
| Vector-1 | E220242 | P95046378 | 69 |
| Vector-1 | E220242 | P95046368 | 66 |
| Vector-1 | E220242 | P95046396 | 65 |
| Vector-1 | E220242 | P95046377 | 64.75 |
| Vector-1 | E220242 | P95046399 | 64 |
| Vector-1 | E220242 | P95046375 | 63.5 |
| | **E220242 Average** | | 67.6 |
| Vector-1 | E220698 | P43596694 | 70 |
| Vector-1 | E220698 | P43596662 | 68 |
| | **E220698 Average** | | 69.0 |
| Vector-1 | E220938 | P43596438 | 68.5 |
| Vector-1 | E220938 | P43596469 | 60.5 |
| Vector-1 | E220938 | P43596489 | 58 |
| | **E220938 Average** | | 62.3 |
| Vector-1 | E221089 | P43596831 | 69 |
| Vector-1 | E221089 | P43596820 | 67 |
| Vector-1 | E221089 | P43596823 | 65 |
| | **E221089 Average** | | 67 |
| Wild type | WT1 | | 80 |
| Wild type | WT2 | | 79.5 |
| Wild type | WT3 | | 79 |
| Wild type | WT4 | | 79 |
| Wild type | WT5 | | 75 |
| | **Wild type Average** | | 78.5 |

**Table 7. Plant Heights of homozygous and heterozygous R1 plants using Vector-2.**

| **Event ID** | **R1 Plant ID** | **R1 zygosity for deletion mutant** | **Plant height (inches)** |
|---|---|---|---|
| E187994 | P43597052 | Heterozygous | 60.5 |
| E187994 | P43597049 | Heterozygous | 73.5 |
| | **E187994 Heterozygous Average** | | 67.0 |
| E187994 | P43597037 | Homozygous | 65 |
| E187994 | P43597077 | Homozygous | 63 |
| | **E187994 Homozygous Average** | | 64.0 |
| E188569 | P43596895 | Heterozygous | 63.5 |
| E188569 | P43596911 | Heterozygous | 59.5 |
| E188569 | P43596896 | Heterozygous | 69 |
| | **E188569** | | 64.0 |
| | **Heterozygous Average** | | |
| E188569 | P43596908 | Homozygous | 55.75 |
| E188569 | P43596926 | Homozygous | 51 |
| E188569 | P43596931 | Homozygous | 65 |
| | **E188569 Homozygous Average** | | 57.3 |
| E188579 | P43596582 | Heterozygous | 62 |
| E188579 | P43596603 | Heterozygous | 59 |
| E188579 | P43596586 | Heterozygous | 65 |
| | **E188579 Heterozygous Average** | | 62.0 |
| E188579 | P43596594 | Homozygous | 61 |
| E188790 | P09617182 | Heterozygous | 65.75 |
| E188790 | P09617238 | Heterozygous | 65 |
| E188790 | P09617144 | Heterozygous | 61 |
| E188790 | P09617191 | Heterozygous | 50.25 |
| | **E188790 Heterozygous Average** | | 60.5 |
| E188790 | P09617192 | Homozygous | 55 |
| E188790 | P09617208 | Homozygous | 60.25 |
| E188790 | P09617225 | Homozygous | 70 |
| E188790 | P09617231 | Homozygous | 60.75 |
| E188790 | P09617216 | Homozygous | 59.5 |
| | **E188790 Homozygous Average** | | 61.1 |
| Wild type | WT1 | | 80 |
| Wild type | WT2 | | 79.5 |
| Wild type | WT3 | | 79 |
| Wild type | WT4 | | 79 |
| Wild type | WT5 | | 75 |
| | **Wild type Average** | | 78.5 |

### Example 30. Collection of samples from R2 plants for molecular assays.

For the E220141 and E221089 deletion edits from the Vector-1 construct, R1 plants homozygous for those deletion edits (P43596991 and P43596831, respectively) were selfed to produce homozygous inbred R2 plants. The R2 inbred plants containing one of the E220141 and E221089 edits, and wild type control plants of the same inbred line, were grown under standard conditions in the greenhouse and sampled at V2 growth stage for the molecular assays described below. The plants were cut just above the soil level and the entire above-ground portion of the plants were placed in 50ml conical tubes and immediately frozen in liquid nitrogen. Each sample contained one or two sibling plants of the same genotype. The number of samples for each assay and genotype are provided in Table 8. The frozen samples were milled and used for the small RNA and GA hormone assays described in Examples 31 and 32 below.

**Table 8. Description of samples for small RNA and GA hormones assays.**

| **Editing Construct ID** | **Edit ID (R2 Inbreds)** | **Number of samples for small RNA assay** | **Number of samples for GA hormone assay** |
|---|---|---|---|
| Inbred Wild type | | 2 | |
| Vector-1 | E220141 | 2 | 7 |
| Vector-1 | E221089 | 1 | 10 |

### Example 31. Detection of small RNAs in plants having an edited deletion allele.

To generate small RNA libraries for sequencing, Illumina's TruSeq small RNA Library Preparation Kit was used according to the manufacturer's protocol (Document # 15004197v02) with a modification at the library purification step. Samples of each genotype for this small RNA assay experiment are identified in Example 30 above. After amplification of cDNA, individual libraries were gel purified using a 6% Novex TBE PAGE Gel for size separation. The gel was stained with 1xSYBR Gold for 20 minutes. The final library product was sequenced on Illumina's NextSeq platform with a minimum depth of 3 million reads per sample. After sequencing, reads were processed through the following steps: the sequencing adapters were trimmed; reads matching housekeeping noncoding RNAs were removed and libraries normalized to reads per million. Between 1 and 9 samples per genotype were assayed.

The mutated GA20 oxidase 5 (GA20ox5) gene containing the E220141 and E221089 deletion edits were predicted to produce antisense RNA transcripts spanning all or part of the coding sequence of the GA20ox5 gene under the control of the downstream native SAMT promoter in the reverse orientation that could hybridize to mRNA transcripts expressed from the wild type and/or mutant GA20 oxidase 5 alleles and/or the GA20 oxidase 3 gene or allele(s). Since antisense RNA sequences can trigger RNA interference (RNAi) and suppression of genes encoding identical or homologous RNA sequences, plants containing the deletion edits were assayed for the presence of small RNAs. Processing of the double stranded RNA would be expected to produce small RNAs of about 21, 22 or 24 nucleotides in length corresponding to the coding sequence of the GA20ox5 gene. In this experiment, the edited R2 plants, as well as wild type control plants, did not show a noticeable accumulation of small RNAs corresponding to the GA20ox5 gene in the 21, 22 or 24-nucleotide small RNA range, which was measured to be 0 or 1 read per million total sequencing reads (data not shown). These data indicate that the edited plants either do not produce small RNAs at the V2 growth stage sampled in this example or act through a different dominant negative mechanism. However, the pattern of expression of antisense RNA transcripts complementary to all or part of the coding sequence of the GA20 oxidase 5 gene is also dependent on the SAMT gene promoter, which may not drive expression (or expression at a sufficiently high level) at the V2 growth stage to produce a measurable effect on the levels of small RNAs. Without being bound by theory, it is possible that expression of antisense transcripts from an edited deletion allele of the endogenous GA20ox5 gene may be more robust at later stages of development and thus have a greater or more measurable effect on the level of small RNAs and RNAi suppression at those later stages.

Future experiments will also seek to determine whether the levels of GA20ox3 and/or GA20ox5 mRNA transcripts are reduced in plants homozygous or heterozygous for an edited GA20ox5 allele having a deletion between the GA20ox5 and SAMT genes, relative to controls.

### Example 32. Detection of GA hormones in plants having an edited deletion allele

Reduced expression of GA20 oxidase genes can alter the levels of GA hormones in com plants, which can in turn affect plant height with lower levels of active GAs potentially reducing plant height. The levels of bioactive GA hormones and their precursors were measured in plants containing the edited GA20ox5 alleles. GA20 oxidase is active in the GA biosynthetic pathway and catalyzes the sequential oxidation of metabolic intermediates GA12 and GA53 into GA9 and GA20, respectively (the "early 13-hydroxylation pathway" and "non 13-hydroxylation pathway"). The primary bioactive forms of GA include GA1, GA3 and GA4, which are further downstream (3') of GA20 oxidase activity and the GA9 and GA20 intermediates in the biosynthetic pathway. A reduction or suppression of the expression level and/or enzymatic function of GA20 oxidase genes, as may be expected with the GA20ox5 deletion edits, may result in reduction of downstream metabolites (GA20 and GA9) and accumulation of upstream precursors (GA53 and GA12).

For this experiment, samples were collected as provided in Example 30 above. Freshly frozen plant sample tissues were extracted and cleaned using Waters solid phase extraction MAX cartridge plate. GA hormones and 2 internal standards were analyzed using UPLC coupled with an ABSciex 5500 Mass Spectrometry with MRM method. The final GA hormone values were calculated based on the calibration curve with ABSciex software Multi-Quan. Each GA hormone calibration curve was in good linear fit, the R2 linear regression >0.99. The 8 technical controls per 96-well plate for each hormone were also included and evaluated in analytical process for meeting the standard criterion. GA levels were measured in terms of pmol/gram of sample tissue.

As shown in FIG. 24, the levels of GA12 were increased in inbred plants homozygous for the edited E221089 allele but were statistically neutral or unchanged in inbred plants homozygous for the edited E220141 allele, relative to wild type control plants. As further shown in FIG. 24, the levels of GA9 were decreased in inbred plants homozygous for the edited E220141 allele but neutral in inbred plants homozygous for the edited E221089 allele, relative to wild type control plants.

As shown in FIG. 25, the levels of GA20 were decreased in inbred plants homozygous for either of the edited alleles (E221089 or E220141), relative to wild type control plants. As further shown in FIG. 25, the levels of GA53 were increased in inbred plants homozygous for either of the edited alleles (E221089 or E220141), relative to wild type control plants.

FIG. 26 provides the results for levels of active GAs (GA1, GA3 and GA4) measured in samples collected at V2 growth stage of the edited inbred plants relative to wild type controls. As shown in FIG. 26, the levels of these active GAs were generally not statistically changed in the inbred plants homozygous for the edited alleles (E221089 or E220141), except for an increase in GA4 in inbred plants homozygous for either of the edited alleles (E221089 or E220141).

These data support the theory that an antisense transcript may be expressed from the edited GA20 oxidase 5 gene, allele or locus having a deletion between the neighboring GA20 oxidase 5 and SAMT genes, that may reduce the expression level(s) of the GA20 oxidase 5 and/or GA20 oxidase 3 gene(s) and thus affect the levels of GA hormones in plants containing the edited alleles. The data in this experiment show increased accumulation of the GA12 and GA53 precursors upstream (5') of GA20 oxidase activity and decreased levels of GA9 and GA20 products of GA20 oxidase activity in plants containing the edited GA20 oxidase 5 allele, although the levels of GA12 and GA9 were unchanged in the edited E220141 and E221089 inbred plants, respectively.

Although the levels of bioactive GAs were not shown to be reduced in this example, this may be due to the early V2 growth stage when the plant tissue samples were collected for this experiment. Indeed, the pattern of expression of an antisense RNA transcript complementary to all or part of the coding sequence of the GA20 oxidase 5 gene is dependent on the SAMT gene promoter, which may not drive expression (or expression at a sufficiently high level) at the early V2 growth stage to produce a measurable effect on the levels of active GAs. Without being bound by theory, it is possible that expression of antisense transcripts from the edited deletion alleles of the endogenous GA20ox5 gene under the control of the endogenous SAMT gene promoter may be more robust at later stages of development and thus have a greater or more measurable effect on the level(s) of active GAs at those later stages. The active GAs are also further downstream and not a direct product of GA20 oxidase enzyme activity. Future experiments will determine if lower active GA levels are observed at later stages of development in plants heterozygous or homozygous for an edited GA20 oxidase 5 locus comprising a deletion between the GA20ox5 and SAMT genes, which is supported by the altered levels of GA precursors observed in this example at the early V2 growth stage.

### Example 33. Creating dominant alleles by genome editing to produce a hairpin-containing transcript (For reference only)

FIG. 27 provides illustrative examples for the production, through targeted gene editing, of a genomic modification of the Zm.GA20ox3 locus to encode a RNA transcript with an inverted sequence that can hybridize to a corresponding sequence of the RNA transcript to produce a stem-loop structure, to cause the suppression of one or both of the copies or alleles at the endogenous Zm.GA20ox3 and Zm.GA20ox5 loci. The Zm.GA20ox3 and Zm.GA20ox5 genes share a high level of nucleotide sequence similarity of their respective exon regions. Thus, a fragment of the Zm.GA20ox5 gene, sharing sufficient sequence identity or homology with a corresponding sequence of the Zm.GA20ox3 gene, inserted into a preselected site in the Zm.GA20ox3 gene in the reverse orientation near the corresponding Zm.GA20ox3 sequence, can hybridize to the corresponding sequence to form a stem-loop structure and trigger suppression of the Zm.GA20ox3 and Zm.GA20ox5 genes. The insertion site is determined by the design of the guide RNA directing a double-stranded genomic DNA cleavage. Without being bound to any particular theory, the edited Zm.GA20ox3 gene produces a transcript able to form a stem-loop structure which can induce suppression or gene silencing of the wild-type or other alleles of the Zm.GA20ox3 and Zm.GA20ox5 genes. In this example, the inserted Zm.GA20ox5 fragment can be excised from either copy or allele of the endogenous Zm.GA20ox5 gene. This type of editing is called trans-fragment targeting (TFT) editing since the fragment originates from the endogenous Zm.GA20ox5 gene. The boundaries of the excision fragment are defined by a pair of properly designed guide RNAs. According to this example, the inserted Zm.GA20ox5 fragment can also be excised from a donor template which is a double-stranded DNA molecule comprising the desired Zm.GA20ox5 fragment and flanked by target sites for guide RNA(s). The flanking gRNA target sites can be identical, utilizing the same gRNA, or different and according to some embodiments oriented to limit the amount of gRNA target site included in the DNA insertion fragment post cleavage. These target sites can match to the endogenous genome, be artificially-derived, or match to a non-endogenous genome, relative to the endogenous genome of the plant to be edited. According to some embodiments, the target sites flanking the insertion fragment of the donor template to be excised for insertion into the endogenous locus may be heterologous with respect to the native or endogenous genome, such that any off-target DNA cleavage within the endogenous genome can be minimized or eliminated. This method is referred to as "template assist" when performed in combination with guide RNAs targeted for excising a fragment from the endogenous Zm.GA20ox5 locus. Conversely, a Zm.GA20ox3 fragment may be excised via TFT from either copy or allele of the Zm.GA20ox3 gene and/or from a donor template (e.g., template assist method) and inserted into a pre-selected site of the endogenous Zm.GA20ox5 gene to produce an inverted-repeat sequence which can be transcribed into a RNA transcript comprising a stem-loop structure that triggers RNA-mediated suppression or silencing of the wild-type or other alleles of the Zm.GA20ox3 and/or Zm.GA20ox5 genes.

With either of these approaches, however, it is possible for other types of edits or mutations to be formed, such as deletion(s) and/or inversion(s) depending on which DNA cut(s) or break(s) are created at the gRNA or editing target site(s) and the fragment(s) inserted into or between those cut site(s). An inserted DNA fragment may originate from either copy or allele of the Zm.GA20ox3 or Zm.GA20ox5 gene, or from a DNA template molecule. Therefore, a deletion can be generated from cutting one or more target sites, and/or an inversion sequence can be generated by a DNA fragment being inserted in an opposite, reverse or antisense orientation relative to the coding sequence of the edited Zm.GA20 oxidase gene. The inversion may be present in the edited gene with or without a corresponding sequence that when expressed could hybridize to form a RNA hairpin or stem-loop structure with the encoded inversion sequence of the mRNA transcribed from the edited gene. The presence of an antisense inversion sequence without the corresponding sequence and resulting hairpin or stem-loop structure may be sufficient to trigger suppression of one or both of the Zm.GA20ox3 and Zm.GA20ox5 genes through canonical or non-canonical RNA mechanisms.

A plant transformation construct (Vector-4) was designed to create a double stranded break (DSB) in the Zm.GA20ox3 gene to allow for insertion of an antisense DNA fragment of the Zm.GA20ox5 gene either from the endogenous Zm.GA20ox5 locus or an exogenously provided donor template. In this example, the construct generally contains 4 functional regions or cassettes relevant to gene editing and creation of the insertion (e.g., inversion) in the edited gene: expression of a Cpfl or Cas12a variant protein, expression of three guide RNAs for the Zm.GA20ox3 gene locus, expression of an additional three guide RNAs for the Zm.GA20ox5 gene locus, and a donor template region comprising a Zm.GA20ox5 gene fragment for the template assist method of inserting the Zm.GA20ox5 gene fragment (approximately 400 nucleotides in length) from the donor template. Each guide RNA unit contains a common scaffold compatible with the Cpfl mutant, and a unique spacer/targeting sequence complementary to its intended target site.

The Cpfl expression cassette comprises a maize ubiquitin promoter (SEQ ID NO: 37) operably linked to a sequence encoding a *Lachnospiraceae bacterium* G532R/K595R mutant Cpfl RNA-guided endonuclease enzyme (SEQ ID NO: 39) fused to two nuclear localization signals (SEQ ID NOs: 42 and 43). *See, e.g.,* Gao, L. et al., Nature Biotechnol. 35(8): 789-792 (2017).

One expression cassette comprises a sequence encoding three guide RNAs (two guide RNAs having targeting/spacer sequences encoded by the SP1 and SP2 DNA sequences in Table 9 below (see also FIG. 27) that target two closely spaced-apart sites in exon 1 of the Zm.GA20ox3 gene; and another guide RNA having a targeting/spacer sequence encoded by the SP3 DNA sequence in Table 9 below (see also FIG. 27) targeting a site in exon 1 of the Zm.GA20ox5 gene), operably linked to a maize RNA polymerase III (Pol3) promoter (SEQ ID NO: 44). Spacer sequences SP1 and SP2 target two alternative breakage sites in exon-1 of Zm.GA20ox3 that are spaced apart by about 68 nucleotides, and either breakage site is able to receive a reverse complement insertion fragment, or the insertion fragment could replace the sequence between SP1 and SP2. It is also possible that the insertion fragment could integrate at both SP1 and SP2 target sites.

Another expression cassette comprises a sequence encoding an additional three guide RNAs (two guide RNAs having targeting/spacer sequences encoded by the SP4 and SP5 DNA sequences in Table 9 (see also FIG. 27) that target two closely spaced-apart target sites in exon 1 of the Zm.GA20ox5 gene; and another guide RNA having targeting/spacer sequences encoded by the SP6 DNA sequence in Table 9 (see also FIG. 27) targeting two identical engineered sites flanking the Zm.GA20ox5 gene fragment in the donor template region), operably linked to a synthetic promoter. Spacer sequences SP3 or SP4 target two alternative breakage sites in exon-1 of the endogenous Zm.GA20ox5 gene that are spaced apart by about 83 nucleotides, whereas the spacer sequence SP5 targets another breakage point in exon-1 of the endogenous Zm.GA20ox5 gene spaced apart from the cleavage site for the SP4 spacer by a greater distance of about 577 nucleotides. The targeting/spacer sequence SP6 is derived from a soybean PDS gene that has been separately demonstrated to work in combination with Cpfl to direct cleavage of a target site in an endogenous PDS gene of a soybean plant.

Another nearly identical plant transformation construct (Vector-5) was designed to create a double stranded break (DSB) in the Zm.GA20ox3 gene to allow for insertion of an antisense DNA fragment of the Zm.GA20ox5 gene, but this second construct did not encode the guide RNA having the targeting/spacer sequence encoded by the SP6 DNA sequence for the template assist method, such that the fragment would originate from an endogenous copy of the Zm.GA20ox5 gene.

With the constructs described in this example, guide RNAs with spacers SP3 and SP4 may work in combination with a guide RNA with spacer SP5 would produce a fragment between about 500 and 700 bp from exon-1 of the endogenous Zm.GA20ox5 gene that could be inserted into a site within exon-1 of the endogenous Zm.GA20ox3 gene in the reverse complementary orientation, such that the RNA molecule transcribed from the endogenous Zm.GA20ox3 gene forms a stem-loop structure in the RNA transcript that can trigger suppression or silencing of the other copy/copies or allele(s) of the endogenous Zm.GA20ox3 and/or Zm.GA20ox5 gene(s). The resulting fragment could be referred to as a SP3-SP5 fragment (SEQ ID NO: 76) or a SP4-SP5 fragment (SEQ ID NO: 77), depending on whether spacer SP3 or SP4 was involved. In addition, a donor template containing a Zm.GA20ox5 gene fragment flanked by two SP6 spacer sequences can produce a Zm.GA20ox5 gene fragment (referred to as a SP6-SP6 fragment (e.g., SEQ ID NO: 88), if used in combination with the first transformation construct described above containing the SP6 sequence, for insertion into a site within the endogenous Zm.GA20ox3 gene in the reverse complementary orientation.

The DNA sequences encoding the guide RNA spacers and their intended target sites are listed in Table 9.

**TABLE 9. Example guide RNAs used for editing the Zm.GA20ox3 locus.**

| **Guide RNA Spacer** | **Target Site** | **Spacer Sequence** | **SEQ ID** |
|---|---|---|---|
| SP1 | GA20ox3_2587 | CTCCAACCACACCCTCTCCTTCC | 78 |
| SP2 | GA20ox3_2655 | CCGGCACCCTCGGCCAAGATTTC | 79 |
| SP3 | GA20ox5_428 | CTCCCTGCCTTCGTCTTTGTCGT | 80 |
| SP4 | GA20ox5_511 | CTGCATACTTGCAGCTCGCACAT | 81 |
| SP5 | GA20ox5_1088 | CTCCAACCACACCCTCTCCTTCC | 82 |
| SP6 | Template Gm.PDS site | GTAAGAAGCTCTTCACCGTTCCA | 83 |

### Example 34. Confirmation of inversion edits in plants using the constructs in Example 33 (For reference only)

An inbred com plant line was transformed via *Agrobacterium-mediated* transformation with one of the transformation vectors described above in Example 33. The transformed plant tissues were grown to produce mature R0 plants. R0 plants having one or more unique genome edit(s) were self-crossed to produce R1 plants. To determine the edits and insertions in the endogenous Zm.GA20ox3 gene of the R0 and R1 plants, one or two PCR assay approaches were performed, with primers designed to identify the size or junctions of the intended insertions. One approach used a PCR primer pair including one primer (SEQ ID NO: 84) hybridizing to a sequence in the inserted Zm.GA20ox5 gene fragment and another primer (SEQ ID NO: 85) hybridizing to a sequence in the endogenous GA20ox3 gene, where the primers are oriented such that a PCR product is generated when the Zm.GA20ox5 gene fragment is inserted in the antisense orientation in the endogenous GA20ox3 gene (i.e., the PCR product was only generated across the 3' end of the inserted fragment when oriented in the inverted antisense direction). If a PCR fragment was generated, then a Zm.GA20ox5 gene fragment was inserted at the target site in the antisense orientation. In addition, whether the inserted Zm.GA20ox5 fragment originated from the endogenous Zm.GA20ox5 locus or the donor template region could also be determined and distinguished by PCR product size and/or sequencing the PCR products. This first PCR approach was used to determine which type of inverted insertion occurred in the endogenous Zm.GA20ox3 gene (see Tables 10 and 11).

According to a second PCR approach, a PCR primer pair including one primer (SEQ ID NO: 86) hybridizing to a sequence upstream (on the 5' side) of the two guide RNA target sites (SP1 and SP2) in the Zm.GA20ox3 gene and the other primer (SEQ ID NO: 87) hybridizing to a sequence downstream of the two SP1 and SP2 guide RNA target sites, such that a PCR product is generated spanning the possible insertion sites in the Zm.GA20ox3 gene. Thus, the presence and size of the PCR fragment using this approach would show whether an insertion occurred at the target sites, but independent of orientation. The PCR product can also be sequenced to determine the type and orientation of the insertion. According to this second approach, the size/sequence of the PCR product could also be used to determine whether the inserted GA20ox5 fragment originated from the endogenous GA20ox5 locus or the donor template region, and the zygosity of the plant could be determined by whether the wild-type PCR fragment size/sequence was present.

Individual R1 plants produced by selfing R0 plants having one or more of the edits were assayed for the type of insertion and the zygosity of the insertion mutant or allele (see Tables 10 and 11). As used herein, "homo" means homozygous for the mutant allele, and "hetero" means heterozygous for the mutant allele. Tables 10 and 11 further provide the genomic DNA sequence of the coding sequence or region of the edited GA20 oxidase 3 gene from the start to stop codon and the sequence of the inversion or antisense sequence within such coding sequence or region of the edited GA20 oxidase 3 gene (each by SEQ ID NO). To avoid repetition, the inversion type and coding and inversion sequences are only provided in the first row of Tables 10 and 11 for each Edit ID. Edit IDs E270933 and E271059 in Table 10 were generated with the Vector-4 construct, and Edit IDs E376333 and E376314 in Table 11 (and Edit ID E376274) were created using the Vector-5 construct. Additional edits were generated with these constructs, but either were not recovered in R1 plants/seeds, had other T-DNA insertions and/or did not produce small RNAs, and were therefore discarded and not advanced for further testing. Edit ID E376274 created with the Vector-5 construct comprising an inverted GA20ox5 SP4-SP5 fragment inserted into the GA20ox3 SP1 target site was not recovered in R1 plants/seeds and was therefore not advanced. Edit ID E376274 for the GA20 oxidase 3 gene has a genomic coding sequence of SEQ ID NO: 97 and an inversion sequence of SEQ ID NO: 98.

Tables 10 and 11 also provide information about possible simple/small or larger edit(s) or deletion(s) that may be present in the GA20 oxidase 5 gene. Simple or small deletion(s) may also be present in the endogenous GA20 oxidase 5 (GA20ox5) gene at one or more of the individual SP3, SP4 and SP5 target sites, and large deletion(s) may be present in the endogenous GA20ox5 gene spanning between the SP3/SP5 or SP4/SP5 target sites. For the Vector-5 construct, Table 11 provides pooled information and numbers for the R1 plants grouped by Edit ID. As can be seen in Tables 10 and 11, R0 and R1 plants in many cases did contain one or more edits or deletions in the GA20ox5 locus, although some R1 plants (designated as "unknown" in the tables) were not determined to contain an edit or deletion in the GA20ox5 gene. In other cases, the zygosity of an edited GA20ox5 allele was not determined and is therefore designated as "homozygous or heterozygous". However, the edited GA20ox5 alleles present in R0 and R1 plants were removed and segregated away from the edited GA20ox3 alleles in subsequent generations.

**TABLE 10. Editing Inversion and Zygosity in R0 and R1 plants for Vector-4**

| **Edit ID** | **R1 Plant ID** | **Editing inversion type** | **R1 zygosity call for GA20ox3 mutant** | **GA20ox5 Edit** | **Genomic Coding Sequence (SEQ ID NO)** | **Inversion Sequence (SEQ ID NO)** |
|---|---|---|---|---|---|---|
| E270933 | P757870 | GA20ox5 SP4-SP5 fragment into GA20ox3 SP1 target site; 6 nucleotide deletion at SP2 | hetero | unknown | 89 | 90 |
| E270933 | P757885 | | hetero | unknown | | |
| E270933 | P758012 | | hetero | unknown | | |
| E270933 | P758049 | | hetero | unknown | | |
| E270933 | P758040 | | hetero | Heterozygous large deletion | | |
| E270933 | P758007 | | hetero | unknown | | |
| E270933 | P757888 | | hetero | Heterozygous large deletion | | |
| E270933 | P757932 | | hetero | Heterozygous large deletion | | |
| E270933 | P757965 | | hetero | unknown | | |
| E270933 | P758046 | | hetero | unknown | | |
| E270933 | P757857 | | hetero | unknown | | |
| E270933 | P757881 | | hetero | unknown | | |
| E270933 | P757925 | | hetero | unknown | | |
| E270933 | P757982 | | hetero | unknown | | |
| E270933 | P757985 | | hetero | Heterozygous large deletion | | |
| E270933 | P758051 | | hetero | unknown | | |
| E270933 | P757886 | | hetero | unknown | | |
| E270933 | P757853 | | hetero | unknown | | |
| E270933 | P757904 | | hetero | unknown | | |
| E270933 | P757956 | | hetero | unknown | | |
| E270933 | P757970 | | hetero | unknown | | |
| E270933 | P757987 | | hetero | unknown | | |
| E270933 | P757962 | | hetero | unknown | | |
| E270933 | P757949 | | hetero | unknown | | |
| E270933 | P758001 | | hetero | unknown | | |
| E270933 | P758004 | | hetero | unknown | | |
| E271059 | P758336 | donor template fragment into GA20ox3 SP1 target site; 9 nucleotide deletion at SP2 | hetero | Homozygous or heterozygous small deletion | 91 | 92 |
| E271059 | P758342 | | hetero | Homozygous or heterozygous small deletion | | |
| E271059 | P758343 | | hetero | Biallelic for small deletions; T-DNA insert | | |
| E271059 | P758349 | | hetero | Homozygous or heterozygous small deletion | | |
| E271059 | P758352 | | hetero | Homozygous small deletion | | |
| E271059 | P758354 | | hetero | Homozygous or heterozygous small deletion | | |
| E271059 | P758355 | | hetero | Homozygous small deletion | | |
| E271059 | P758330 | | hetero | Homozygous or heterozygous small deletion | | |

**TABLE 11. Editing Inversion and Zygosity in R0 and R1 plants for Vector-5**

| **Edit ID** | **Number of R1 Plants** | **Editing inversion type** | **R1 zygosity call for GA20ox3 mutant** | **GA20ox5 Deletion** | **Genomic Coding Sequence (SEQ ID NO)** | **Inversion Sequence (SEQ ID NO)** |
|---|---|---|---|---|---|---|
| E376333 | 8 | GA20ox5 SP4-SP5 fragment to GA20ox3 SP2; 7 nucleotide deletion at SP1 | hetero | 4 with large GA20ox5 deletion; 1 with small GA20ox5 deletion; and 3 with both. | 93 | 94 |
| E376333 | 17 | | homo | 2 with large GA20ox5 deletion; 1 with small GA20ox5 deletion; 6 with both; and 8 unknown. | | |
| E376333 | 10 | | homo or hetero | unknown | | |
| E376314 | 8 | GA20ox5 SP4-SP5 fragment to GA20ox3 SP2; 6 nucleotide at SP1 | hetero | unknown | 95 | 96 |
| E376314 | 1 | | homo | unknown | | |
| E376314 | 1 | | homo or hetero | unknown | | |

### Example 35. Reduced plant height of corn plants with edited allele (For reference only)

**R1** com plants heterozygous for an edited allele of the GA20 oxidase 3 gene with the corresponding inversion identified in Example 34 were grown to maturity to measure their plant heights along with wild type control plants. R1 seeds were planted in soil and grown to maturity in the greenhouse under day/night temperatures of 85°F / 70°F (29.4°C / 21.1°C) and a photoperiod of 16 hours light/8 hours dark using standard nutrient and light conditions for com plant growth and development. Plant heights (PHT) of these R1 plants were measured at R2 growth stage from the soil level to the base of the uppermost fully expanded leaf. Table 12 provides the plant heights of individual R1 plants heterozygous for one of two hairpin inversion edits, along with wild type control plants. Average plant heights for WT and each edit are also provided (see also FIG. 28 showing the average plant heights with error bars).

These plant heights demonstrate that plants heterozygous for an edited GA20 oxidase 3 allele comprising an inversion sequence have reduced plant heights averaging 54.0 inches or 57.3 inches for the two edited alleles, versus an average plant height of 64.2 inches for the WT control.

The plant height data shown in this example demonstrate that plants heterozygous for an edited allele of the GA20 oxidase 3 gene comprising an antisense inversion sequence have significantly reduced plant heights in comparison to wild type control plants, suggesting that these edited hairpin inversion alleles of the GA20 oxidase 3 gene act in a dominant or semi-dominant manner to produce a reduced plant height phenotype (i.e., semi-dwarf or short stature corn plants), especially since edited loss-of-function alleles of the GA20 oxidase 3 or GA20 oxidase 5 genes alone without an antisense or inversion sequence have been shown to not produce short stature com plants. See, e.g., Published PCT Application Nos. WO/2019/161149, WO/2019/161147 and WO/2019/161144. However, many of these R1 plants may also be homozygous or heterozygous for edited GA20ox5 allele(s) (see Table 10). The presence and zygosity of edited GA20ox5 alleles is unknown for many of the R1 plants, but R1 Plant IDs P758040, P757888, P757932 and P757985 for the E270933 Edit ID were heterozygous for a large deletion in the GA20ox5 gene, R1 Plant ID P758352 was homozygous for a small deletion in the GA20ox5 gene, R1 Plant ID P758343 contained small deletion(s) and a T-DNA insert in the GA20ox5 gene, and P758336 was homozygous or heterozygous for a small deletion in the GA20ox5 gene. Therefore, it is possible that additional mutation(s) in the GA20ox5 gene could also have an effect on R1 plant height. Further plant height measurements will be made in subsequent generations having the edited GA20ox5 alleles removed to confirm the shorter plant height phenotype.

**Table 12. Plant Heights of R1 plants heterozygous for edited inversion alleles of Zm.GA20ox3**

| **Edit ID** | **R1 Plant ID** | **Plant height (inches)** |
|---|---|---|
| E270933 | P757870 | 56.25 |
| E270933 | P757885 | 49.75 |
| E270933 | P758012 | 55 |
| E270933 | P758049 | 48.5 |
| E270933 | P758040 | 54.5 |
| E270933 | P758007 | 62 |
| E270933 | P757888 | 58 |
| E270933 | P757932 | 58 |
| E270933 | P757965 | 52.75 |
| E270933 | P758046 | 56.5 |
| E270933 | P757857 | 61.5 |
| E270933 | P757881 | 63.5 |
| E270933 | P757925 | 53.25 |
| E270933 | P757982 | 56.75 |
| E270933 | P757985 | 50.25 |
| E270933 | P758051 | 50.5 |
| | **Edit ID E270933 Average** | 55.4 |
| E271059 | P758352 | 54 |
| E271059 | P758343 | 54.5 |
| E271059 | P758336 | 63.5 |
| | **Edit ID E271059 Average** | 57.3 |
| Wild type | WT 1 | 63.5 |
| Wild type | WT 2 | 63.75 |
| Wild type | WT 3 | 68.5 |
| Wild type | WT 4 | 61 |
| | **Wild type Average** | 64.2 |

### Example 36. Collection of samples from R2 or R3 plants for molecular assays. (For reference only)

For the E270933 inversion edit from the Vector-4 construct, a R1 plant heterozygous for the E270933 edit (P757982) was selfed (self-pollinated) to obtain selected homozygous R2 plants, which were themselves either (i) self-pollinated to produce homozygous inbred R3 plants or (ii) crossed to another elite parental line to produce heterozygous hybrid R3 plants. For the E376333 inversion edit from the Vector-5 construct, a R1 plant homozygous for the E376333 edit (P127584) containing a large deletion in the GA20ox5 gene was either (i) self-pollinated to produce homozygous inbred R2 plants or (ii) crossed to another elite parental line to produce heterozygous hybrid R2 plants. Edited GA20ox5 alleles present in R1 plants were removed in R2 and R3 plants by segregation and selection. The R3 plants containing the E270933 edit, the R2 plants containing the E376333 edit, and wild type control plants of the same parental lines, were grown under standard conditions in the greenhouse and sampled at V2 growth stage for the molecular assays described below. The plants were cut just above the soil level and the entire above-ground portion of the plants were placed in 50 mL conical tubes and immediately frozen in liquid nitrogen. Each sample contained one or two sibling plants of the same genotype. The number of samples for each assay and genotype are provided in Table 13. The frozen samples were milled and used for the small RNA and GA hormone assays described in Examples 37 and 38 below.

**Table 13. Description of samples for small RNA and GA hormones assays.**

| **Editing Construct ID** | **Edit ID** | **Parental elite line(s)** | **Number of samples for small RNA assay** | **Number of samples for GA hormone assay** |
|---|---|---|---|---|
| Vector-4 | E270933 | Hybrid | 8 | 10 |
| Vector-5 | E376333 | Hybrid | 5 | 5 |
| Vector-4 | E270933 | Inbred | 9 | 10 |
| Vector-5 | E376333 | Inbred | 9 | 10 |
| Hybrid Wild type | | | 1 | |
| Inbred Wild type | | | 2 | |

### Example 37. Detection of small RNAs in plants having an edited inversion allele. (For reference only)

To generate small RNA libraries for sequencing, Illumina's TruSeq small RNA Library Preparation Kit was used according to the manufacturer's protocol (Document # 15004197v02) with a modification at the library purification step. Samples of each genotype for this small RNA assay experiment are identified in Example 36 above. After amplification of cDNA, individual libraries were gel purified using a 6% Novex TBE PAGE gel for size separation. The gel was stained with 1x SYBR Gold for 20 minutes. The final library product was sequenced on Illumina's NextSeq platform with a minimum depth of 3 million reads per sample. After sequencing, reads were processed through the following steps: the sequencing adapters were trimmed; reads matching housekeeping noncoding RNAs were removed; and libraries were normalized to reads per million. Between 1 and 9 samples per genotype were assayed.

mRNAs expressed from the edited GA20 oxidase 3 genes containing the E270933 and E376333 inversion edits were predicted to produce a hairpin or stem-loop RNA structure comprising the inversion sequence and the native sequence in the GA20 oxidase 3 gene that is complementary and could hybridize to the inversion sequence. Since double stranded RNA hairpins or stem-loop structures can trigger RNA interference (RNAi) and suppression of genes encoding identical or homologous RNA sequences, plants containing the inversion edits were assayed for the presence of small RNAs. RNAi would be expected to produce small RNAs of about 21 nucleotides in length (21-mers) from the stem of the stem-loop structure consisting in this example of the GA20ox5 inversion sequence and the GA20ox3 native sequence.

As shown in FIG. 29, small 21-mer RNAs corresponding to the regions in the stem of the edited stem-loop comprising the inversion sequence from GA20ox5 and a corresponding sequence of the edited GA20ox3 gene were detected in samples from plants containing the edited GA20ox3 alleles indicating the presence of small RNAs in tissues from these edited plants, which were not present in wild type control plants. The abundance of these small RNAs was measured as the number of reads per million total sequencing reads. Small RNAs were present in inbred plants homozygous for the edited allele and hybrid plants heterozygous for the edited allele, with these small RNAs ranging between 19 and 84 reads per million. The abundance of small RNAs appeared consistent with the number of copies of the edited GA20ox3 allele given that heterozygous hybrid plants produced fewer small RNAs than the homozygous inbred plants.

The presence in these samples of small RNAs corresponding to the edited complementary stem region of the edited GA20ox3 gene is consistent with the edited GA20ox3 inversion allele triggering RNAi suppression of the GA20ox3 gene and possibly also the GA20ox5 gene. Additional experiments will determine whether the levels of GA20ox3 and/or GA20ox5 mRNA transcripts are reduced in plants homozygous or heterozygous for edited GA20ox3 or GA20ox5 alleles containing an inversion sequence, relative to controls.

### Example 38. Detection of GA hormones in plants having an edited inversion allele. (For reference only)

Reduced expression of GA20 oxidase genes can alter the levels of GA hormones in corn plants, which can in turn affect plant height with lower levels of active GAs potentially reducing plant height. The levels of bioactive GA hormones and their precursors were measured in plants containing the edited GA20ox3 alleles. GA20 oxidase is active in the GA biosynthetic pathway and catalyzes the sequential oxidation of metabolic intermediates GA12 and GA53 into GA9 and GA20, respectively (the "early 13-hydroxylation pathway" and "non 13-hydroxylation pathway"). The primary bioactive forms of GA include GA1, GA3 and GA4, which are further downstream of GA20 oxidase activity and the GA9 and GA20 intermediates in the biosynthetic pathway. A reduction or suppression of the expression level and/or enzymatic function of GA20 oxidase genes, as may be expected with the GA20ox3 inversion edits, may result in reduction of downstream metabolites (GA20 and GA9) and accumulation of upstream precursors (GA53 and GA12).

For this experiment, samples were collected as provided in Example 36 above. Freshly frozen plant sample tissues were extracted and cleaned using Waters solid phase extraction MAX cartridge plate. GA hormones and two internal standards were analyzed using UPLC coupled with an ABSciex 5500 Mass Spectrometry with MRM method. The final GA hormone values were calculated based on the calibration curve with ABSciex software Multi-Quan. Each GA hormone calibration curve was in good linear fit, the R2 linear regression was >0.99. The eight technical controls per 96-well plate for each hormone were also included and evaluated in analytical process for meeting the standard criterion. GA levels were measured in terms of pmol/gram of sample tissue.

As shown in FIG. 30, the levels of GA12 were increased in inbred plants homozygous for the edited allele (E270933 and E376333) but were statistically neutral or unchanged in hybrid plants heterozygous for the edited E270933 and E376333 alleles, relative to wild type control plants. As further shown in FIG. 30, the levels of GA9 were increased in inbred plants homozygous for one of the edited alleles (E270933) but neutral in inbred plants homozygous for the other edited allele (E376333), and the levels of GA9 were neutral (E270933) or decreased (E376333) in hybrid plants heterozygous for the edited allele, each relative to wild type control plants.

As shown in FIG. 31, the levels of GA20 were decreased in inbred plants homozygous for one of the edited alleles (E376333) but were increased in inbred plants homozygous for the other edited allele (E270933), and the levels of GA20 were neutral (E270933) or decreased (E376333) in hybrid plants heterozygous for these edited alleles, each relative to wild type control plants. As further shown in FIG. 31, the levels of GA53 were increased in inbred plants homozygous for each edited allele (E270933 and E376333) and in hybrid plants heterozygous for each edited allele (E270933 and E376333), relative to wild type control plants.

FIG. 32 provides the results for levels of active GAs (GA1, GA3 and GA4) measured in samples collected at V2 growth stage of the edited inbred and hybrid plants relative to wild type controls. As shown in FIG. 32, the levels of these active GAs were generally not statistically changed in the inbred and hybrid plants containing each of the edited alleles (E270933 and E376333), except for a small increase in GA4 in inbred plants homozygous for one of the edited alleles (E270933).

These data support the theory that the edited GA20 oxidase 3 gene containing an inversion sequence and encoding a transcript that may form a RNA stem-loop structure is able to affect the levels of GA hormones in inbred and hybrid plants containing the edited alleles. While the data in this experiment are mixed, there is support for increased accumulation of the GA12 and GA53 precursors upstream of GA20 oxidase activity and decreased levels of GA9 and GA20 products of GA20 oxidase activity in plants containing the edited GA20 oxidase 3 allele, although some samples had increased levels of the downstream GA9 and GA20 products. Greater support for decreased GA20 oxidase expression and/or activity with the edited GA20 oxidase 3 alleles is provided in this example by the accumulated levels of upstream GA12 and GA53 precursors. GA12 was neutral to increased in samples from plants with the edited GA20 oxidase 3 allele, and GA53 was increased across all samples from plants having the edited GA20 oxidase 3 allele.

Although the levels of bioactive GAs were not shown to be reduced in this example, this may be due to the early V2 growth stage when the plant tissue samples were collected. The pattern of expression from the endogenous GA20 oxidase 3 locus of transcripts containing the inversion, antisense or stem-loop sequence is also dependent on the endogenous GA20 oxidase 3 gene promoter, which may not drive expression (or expression at a sufficiently high level) at the V2 growth stage to produce a measurable effect on the levels of GA hormones. Without being bound by theory, it is possible that expression of an inversion/hairpin-containing transcript from an edited allele of an endogenous GA20ox3 or GA20ox5 gene under the control of the respective GA20ox3 or GA20ox5 endogenous promoter may be greater at later stages of development and thus have a greater effect on the level(s) of GA hormones at those later stages. The active GAs are also further downstream and not a direct product of GA20 oxidase activity. Future experiments will determine if lower active GA levels are observed at later stages of development in plants heterozygous or homozygous for an edited GA20 oxidase 3 or GA20 oxidase 5 allele. This is supported by the altered levels of GA precursors observed in this example at the early V2 growth stage.

## Claims

1. A method of generating a dominant negative allele of a gene in a cell or of reducing the expression of a gene in a cell comprising::
a) identifying a chromosomal region comprising a first gene region comprising a first promoter and a first coding region, and a second gene region comprising a second promoter and a second coding region, wherein the first coding region and the second coding region are separated by an intervening region, and wherein the first promoter and the second promoter are positioned in opposite orientations;
b) inducing a first double-stranded break and a second double-stranded break flanking a targeted region using a targeted editing technique, wherein the targeted region comprises the second coding region and the intervening region; and
c) identifying one or more cells comprising a deletion of the targeted region, wherein the second promoter generates at least one antisense RNA of the first coding region, and wherein expression of the first coding region is reduced as compared to a control cell that does not comprise the deletion of the targeted region.

2. The method according to claim 1, comprising:
a) identifying a chromosomal region comprising a first gene region comprising a first promoter and a first coding region, and a second gene region comprising a second promoter and a second coding region, wherein the first coding region and the second coding region are separated by an intervening region, and wherein the first promoter and the second promoter are positioned in opposite orientations;
b) providing to one or more cells at least one RNA-guided nuclease or one or more vectors encoding at least one RNA-guided nuclease,
wherein the at least one RNA-guided nuclease is capable of binding to at least 18, at least 19, at least 20, at least 21, at least 22, at least 23, at least 24, at least 25, or at least 26 consecutive nucleotides of a first target site and a second target site flanking a targeted region of a chromosome, wherein the targeted region comprises the second encoding region and the intervening region,
wherein the RNA-guided nuclease creates double-stranded breaks in the chromosome at the first target site and the second target site;
c) identifying one or more cells comprising a deletion of the targeted region; and
d) selecting one or more cells comprising the deletion of the targeted region.

## Patentansprüche

1. Verfahren zur Erzeugung eines dominant negativen Allels eines Gens in einer Zelle oder zur Verringerung der Expression eines Gens in einer Zelle, umfassend:
a) Identifizieren einer chromosomalen Region, umfassend eine erste Genregion, die einen ersten Promotor und eine erste kodierende Region umfasst, und eine zweite Genregion, die einen zweiten Promotor und eine zweite kodierende Region umfasst, wobei die erste kodierende Region und die zweite kodierende Region durch eine dazwischenliegende Region getrennt sind, und wobei der erste Promotor und der zweite Promotor in entgegengesetzten Orientierungen positioniert sind;
b) Induzieren eines ersten doppelsträngigen Bruchs und eines zweiten doppelsträngigen Bruchs, die eine Zielregion flankieren, unter Verwendung einer gezielten Editierungstechnik, wobei die Zielregion die zweite kodierende Region und die dazwischenliegende Region umfasst; und
c) Identifizieren einer oder mehrerer Zellen, die eine Deletion der Zielregion umfassen, wobei der zweite Promotor mindestens eine Antisense-RNA der ersten kodierenden Region erzeugt und wobei die Expression der ersten kodierenden Region im Vergleich zu einer Kontrollzelle, die die Deletion der Zielregion nicht umfasst, reduziert ist.

2. Das Verfahren nach Anspruch 1, umfassend:
a) Identifizieren einer chromosomalen Region, umfassend eine erste Genregion, die einen ersten Promotor und eine erste kodierende Region umfasst, und eine zweite Genregion, die einen zweiten Promotor und eine zweite kodierende Region umfasst, wobei die erste kodierende Region und die zweite kodierende Region durch eine dazwischenliegende Region getrennt sind und wobei der erste Promotor und der zweite Promotor in entgegengesetzten Orientierungen positioniert sind;
b) Bereitstellen mindestens einer RNA-geführten Nuklease oder eines oder mehrerer Vektoren, die für mindestens eine RNA-geführte Nuklease kodieren, für eine oder mehrere Zellen,
wobei die mindestens eine RNA-geführte Nuklease in der Lage ist, an mindestens 18, mindestens 19, mindestens 20, mindestens 21, mindestens 22, mindestens 23, mindestens 24, mindestens 25 oder mindestens 26 aufeinanderfolgende Nukleotide einer ersten Zielstelle und einer zweiten Zielstelle, die eine Zielregion eines Chromosoms flankieren, zu binden, wobei die Zielregion die zweite kodierende Region und die dazwischenliegende Region umfasst,
wobei die RNA-geführte Nuklease doppelsträngige Brüche in dem Chromosom an der ersten Zielstelle und der zweiten Zielstelle erzeugt;
c) Identifizieren einer oder mehrerer Zellen, die eine Deletion der Zielregion umfassen; und
d) Auswählen einer oder mehrerer Zellen, die die Deletion der Zielregion umfassen.

## Revendications

1. Méthode de génération d'un allèle dominant négatif d'un gène dans une cellule ou de réduction de l'expression d'un gène dans une cellule comprenant :
a) identifiant une région chromosomique comprenant une première région génique comprenant un premier promoteur et une première région codante, et une deuxième région génique comprenant un deuxième promoteur et une deuxième région codante, dans laquelle la première région codante et la deuxième région codante sont séparées par une région intervenante, et dans laquelle le premier promoteur et le deuxième promoteur sont positionnés dans des orientations opposées ;
b) induisant une première cassure double brin et une deuxième cassure double brin flanquant une région ciblée à l'aide d'une technique d'édition ciblée, dans laquelle la région ciblée comprend la deuxième région codante et la région intervenante ; et
c) identifiant une ou plusieurs cellules comprenant une délétion de la région ciblée, dans laquelle le deuxième promoteur génère au moins un ARN antisens de la première région codante, et dans laquelle l'expression de la première région codante est réduite par rapport à une cellule de contrôle qui ne comprend pas la déletion de la région ciblée.

2. Méthode selon la revendication 1, comprenant :
a) identifiant une région chromosomique comprenant une première région génique comprenant un premier promoteur et une première région codante, et une deuxième région génique comprenant un deuxième promoteur et une deuxième région codante, la première région codante et la deuxième région codante étant séparées par une région intervenante, et le premier promoteur et le deuxième promoteur étant positionnés dans des orientations opposées ;
b) fournissant à une ou plusieurs cellules au moins une nucléase guidée par ARN ou un ou plusieurs vecteurs codant pour au moins une nucléase guidée par ARN,
dans laquelle la nucléase guidée par ARN est capable de se lier à au moins 18, au moins 19, au moins 20, au moins 21, au moins 22, au moins 23, au moins 24, au moins 25 ou au moins 26 nucléotides consécutifs d'un premier site cible et d'un deuxième site cible encadrant une région ciblée d'un chromosome, dans laquelle la région ciblée comprend la deuxième région codante et la région intervenante,
dans laquelle la nucléase guidée par ARN crée des cassures double brin dans le chromosome au niveau du premier site cible et du deuxième site cible ;
c) identifiant une ou plusieurs cellules comprenant une délétion de la région ciblée ; et
d) sélectionnant une ou plusieurs cellules comprenant la délétion de la région ciblée.
